## Europäisches Patentamt
## European Patent Office
## Office européen des brevets

(11) Publication number: **0 100 561**
**B1**

(12) # EUROPEAN PATENT SPECIFICATION

(45) Date of publication of patent specification: **31.05.89**

(21) Application number: **83107804.3**

(22) Date of filing: **08.08.83**

(51) Int. Cl.⁴: **C 12 N 15/00, C 12 P 21/00, C 12 P 21/02, C 12 P 21/04, C 12 N 9/00, A 61 K 39/00, A 61 K 39/29, A 61 K 39/395, A 61 K 45/02 // C12R1/865, C12R1/88**

(54) Yeast hybrid vectors and their use for the production of polypeptides.

(30) Priority: **09.08.82 GB 8222883**
**31.12.82 GB 8237026**
**02.06.83 GB 8315145**

(43) Date of publication of application:
**15.02.84 Bulletin 84/07**

(45) Publication of the grant of the patent:
**31.05.89 Bulletin 89/22**

(84) Designated Contracting States:
**AT BE CH DE FR IT LI LU NL SE**

(56) References cited:
**EP-A-0 045 573**
**EP-A-0 048 081**
**EP-A-0 060 057**
**EP-A-0 073 635**
**EP-A-0 073 657**
**WO-A-81/02425**
**FR-A-2 458 585**

(73) Proprietor: **CIBA-GEIGY AG**
**Klybeckstrasse 141**
**CH-4002 Basel (CH)**

(72) Inventor: **Hinnen, Albert, Dr.**
**Landstrasse 66**
**CH-5264 Gipf-Oberfrick (CH)**
Inventor: **Meyhack, Bernd, Dr.**
**Säckingerstrasse 6**
**CH-4310 Rheinfelden (CH)**
Inventor: **Meyer, François, Dr.**
**Pfaffenrainstrasse 46**
**CH-4103 Bottmingen (CH)**

(74) Representative: **Zumstein, Fritz, Dr. et al**
**Bräuhausstrasse 4**
**D-8000 München 2 (DE)**

Courier Press, Leamington Spa, England.

**Description**

Field of the invention

The invention relates to DNA fragments containing the promoters of the yeast acid phosphates genes, hybrid vectors containing said promoters capable of transforming yeast cells and yeast cells transformed with said hybrid vectors. This invention also provides processes for the preparation of said DNA fragments, said hybrid vectors and said yeast cells, wherein recombinant DNA technology is applied. Furthermore, the invention concerns a process for the manufacture of polypeptides which are encoded by gene inserts in said hybrid vectors, and which are useful in the treatment of human and animal diseases, and of derivatives thereof.

Background of the invention

With the development of recombinant DNA technology, the controlled microbial production of useful polypeptides, especially such of medical interest, has become possible. Most of the recent work with recombinant DNA technology concerns prokaryotic organisms. Methods have been elaborated and are now well established to introduce into these organisms DNA which codes for eukaryotic proteins. Several bacterial species, especially strains of *Escherichia coli*, which have been modified by this now technology, are now available and permit the commercial production of polypeptides of utmost importance, such as insulin, human leukocyte and fibroblast interferon and human growth hormone.

However, for many purposes it will be desirable or necessary in future to use eukaryotic systems in the commercial preparation of proteins, especially of pharmacologically important proteins. Since yeasts are eukaryotes they share many biological pathways with other eukaryotes, most importantly with mammalian cells. As many pharmacologically important proteins are synthesized by mammalian cells, the relatedness of the two systems can be advantageous. For example, the secretory pathway of yeast resembles that of higher animal cells and it is known that yeast cells have the machinery for the cleavage of signal sequences (uncharged N-terminal part of a protein, usually split off during the secretory transport) (47). Associated with the sectretory pathway is a glycosylation system. The basic steps leading to glycosylated proteins are similar in all eukaryotes and it is expected that yeast cells, contrary to prokaryotic cells, can produce proteins which are faithfully glycosylated (although some final steps in the pathway will have to be modified).

Moreover, yeast cells are free of endotoxins. Contaminating endotoxins are often found in protein preparations from *E. coli* and have to be removed through expensive purification steps.

Since yeast is a microorganism, yeast cells are easy to cultivate. The cell mass obtainable per volume of culture fluid is considerably higher for yeast than for *E. coli.* In addition, the fermentational behaviour of yeast is well understood and conditions for large scale fermentations are already established.

In the last few years, baker's yeast, *Saccharomyces cerevisiae*, has received increasing attention among molecular biologists from basic and applied research areas. To a large extent, this development is due to the establishment of a transformation system (Hinnen et al. (1); Beggs (2)) which allows this microorganism to be used for genetic manipulations, such as introduction and cloning of heterologous DNA. Similar to the prokaryotic systems, plasmids are the preferred vectors used to transform yeast cells, i.e. to introduce recombinant DNA into yeast cells.

There are various patent applications and other publications which relate to vectors, suitable for transforming yeast cells, yeasts transformed with said plasmids, polypeptides produced by said transformed yeasts and processes for the production thereof;

The general yeast transformation protocol is disclosed by Hinnen et al. (1), Beggs (2), Hicks et al. (3) and Struhl et al. (4).

Expression of a human interferon gene linked to DNA fragments of the 5′-flanking sequences of the *Saccharomyces cerevisiae* alcohol dehydrogenase 1 (*ADH1*) gene in a plasmid and transformed into yeast cells is described by Hitzeman et al. (5).

The transformation of *Saccharomyces cerevisiae* with a plasmid containing the chromosomal rabbit β-globin gene is reported by Beggs et al. (6). As set forth in the publication, the gene is incorrectly transcribed and no splicing of the primary β-globin transcripts could be detected.

Eukaryotic cells, such as yeast and especially mammalian cells, co-transformed with foreign DNA coding for a polypeptide and linked with an inducible promoter, and with unlinked DNA which permits the identification of the transformed cells, and a process for the production thereof is described in PCT patent application 81/02425 (7).

A DNA sequence coding for a eukaryotic replication site, eukaryotic vectors conferring mitotic stability at low copy number and containing a eukaryotic replication site, and yeast cells transformed with said vectors are disclosed in European patent application 48081 (8).

Hybrid DNAs comprising, inter alia, a eukaryotic host autonomously replicating segment, method for the production thereof and a method for high-frequency transforming eukaryotic cells, e.g. yeast, with said hybrid DNAs is disclosed in European patent application 45573 (9).

Plasmids comprising the ovalbumin gene controlled by the promoter of the *Escherichia coli* β-lac Z gene and capable of being transformed into yeast cells are described in German Offenlegungsschrift 2923297 (10) and French patent application 2458585 (11).

Hybrid plasmids comprising DNA of a bacterial plasmid, whole or part of the DNA of the yeast 2 μ plasmid and the yeast *URA3* gene and yeasts transformed with said hybrid plasmids are disclosed in European patent application 11562 (12).

Object of the invention

During the last years, there was great progress in the field of genetic engineering, and the first systems using genetically manipulated microorganisms, especially strains of the enterobacteria *Escherichia coli*, are now working. However, there exists a need for additional and improved systems, especially eukaryotic systems, such as yeasts, which are suitable for the economic and large-scale production of proteins in industry. At present, various yeast vectors are available for gene cloning. For the efficient expression of foreign genes in yeast structural coding sequences will have to be combined with strong yeast promoters which, advantageously, should show regulatory features which would allow exogenous control of gene expression. It is an object of the present invention to provide yeast promoters meeting these requirements. It is also an object of the present invention to provide hybrid vectors containing said promoters and foreign structural genes controlled by said promoters.

Detailed description of the invention

1. DNA fragments containing yeast acid phosphatase promoters and their preparation

The present invention provides newly isolated yeast promoters having improved expression properties and a process for the production thereof.

The yeast promoters according to the present invention are derived from the genomic DNA of yeast, especially of *Saccharomyces cerevisiae*. At least two structural genes (*PHO3* and *PHO5*) and several regulatory genes (*PHO2, PHO4, PHO80*, PHO81, PHO85) are involved in the expression of acid phosphatase in yeast (for reference, see, for example, (13)). *PHO5* and *PHO3* code for a repressible (regulated) and a constitituve yeast acid phosphatase, respectively. The *PHO5* gene is repressed at high concentrations of inorganic phosphate and turned on (derepressed) under inorganic phosphate starvation (usually to a high extent under appropriate physiological conditions), whereas the *PHO3* gene is expressed constitutively at low levels. The repressible enzyme is glycosylated and has a molecular weight of about 490 Kilodaltons (14).

The promoters controlling the acid phosphatase genes have not been isolated or used in prior art recombinant DNA technology and hence their nucleotide sequences have not been elucidated. In contrast to other yeast promoters used in recent recombinant DNA technology (e.g. *ADH1*), the DNA sequences directly following the yeast acid phosphatase promoters code for signal peptides which are thought to be involved in the secretion process. It would be advantageous to link a foreign protein coding region to a yeast signal sequence ensuring *in vivo* transport of the protein across the yeast cell membrane. This would result in a reduction of product degradation and contamination of the product by host cell material and would facilitate product recovery.

It is a disadvantage of the promoters hitherto used in recombinant DNA technology that the respective genes are transcribed constitutively. The expressed polypeptide may be either toxic to the yeast cell (fungicidal activity) or may at least inhibit cell proliferation (fungistatic activity), or the polypeptide may be enzymatically digested within the cell, especially if it is exposed to yeast proteases for a long time. In all cases mentioned, the yield of the desired polypeptide would be low. These disadvantages can be avoided by using the *PHO5* promoter and vectors containing said promoter. The *PHO5* promoter can be repressed or turned on (derepressed) at the will of the experimentator, solely by increasing or decreasing the concentration of inorganic phosphate in the medium. Thus, the promoter can be repressed during the exponential growth phase of the yeast and may be turned on only during early stationary phase at maximal cell density allowing expression of the gene controlled by the *PHO5* promoter. This property combined with a high level of transcription makes the *PHO5* promoter the preferred one in the present invention.

The present invention relates especially to a DNA restriction fragment consisting essentially of the repressible yeast acid phosphatase (*PHO5*) promoter comprising the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

and subfragments and mutants thereof which retain the promoter function.

Optionally, the yeast acid phosphatase promoter is followed by all or part of the signal sequence of the yeast acid phosphatase coding region naturally linked to said promoter. In addition, the DNA fragment may contain sequences which are required for efficient translation of mRNA. Also enclosed are those mutants of said DNA fragment which retain the promoter function.

Preferred DNA restriction fragments are those having the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

and subfragments and mutants thereof which retain the promoter function,

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTACTATGG

CTTCATCTCTCATGAGAATAAGAACAACAACAAATAGAGCAAGCAAATTCGGG

and mutants thereof which retain the promoter function, and

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

and mutants thereof which retain the promoter function.

A DNA fragment according to the invention may be prepared, for example, by

(A) preparing the acid phosphatase *PHO5* gene by complementing acid phosphatase *PHO5* deficient yeast strains by transformation with plasmid DNA from a yeast gene library containing the wild-type copy of said gene and isolating said gene,

(B) preparing subclones of the obtained gene, and

(C) identifying the location of the promoter region of the above subclones and isolating by restriction digest DNA fragments essentially consisting of the acid phosphatase *PHO5* promoter, and for the preparation of subfragments, shortening the DNA restriction fragments obtained by digestion with a restriction endonuclease or with a suitable exonuclease.

More especially, the following steps are involved in the preparation of said DNA fragment:

(1) a yeast gene library is constructed using wild-type yeast DNA cloned into a hybrid bacterial (especially *Escherichia coli*)-yeast plasmid carrying appropriate markers capable of expression in both the bacterial and yeast cell (for suitable markers, see below).

(2) Clones containing the yeast acid phosphatase *PHO5* gene are selected by transformation of an acid phosphatase *PHO5* deficient yeast strain using plasmid pools of the above library.

(3) Plasmids containing the acid phosphatase *PHO5* gene are isolated from the transformed yeast and amplified by transforming back into *E. coli* selecting for the phenotypic property of the bacterial marker (e.g. ampicillin resistance).

(2') In an alternative approach the gene library is divided into subpools which are used to transform acid phosphatase *PHO5* deficient yeast strains, and (3') positive sub-pools are again sub-divided and transformed as above until a single clone is identified.

(4) The plasmid DNA of the identified clone is isolated, digested with suitable restriction endonucleases and the fragments are recloned into an appropriate yeast vector.

**EP 0 100 561 B1**

(5) DNA fragments containing the yeast acid phosphatase *PHO5* gene can be identified by transforming yeast acid phosphatse *PHO5* deficient yeast strains with said vectors. By means of this procedure the boundaries of the acid phosphatase *PHO5* gene can be determined with a precision of approximately 300 base pairs.

(6) DNA sequencing of the identified fragments serves to locate the *PHO5* promoter region, the acid phosphatase *PHO5* protein coding regions and, additionally, the restriction site(s) which may be useful in further processing, for example, for cutting off DNA sequences which are not necessary for promoter function, with restriction endonucleases.

Depending on the choice of the restriction endonucleases, the DNA fragments containing the acid phosphatase promoter may also include at the 3' and 5' termini orgininal flanking DNA sequences which do not affect the promoter function and may be used as connecting sequences in the subsequent cloning procedures. If desired, these additional sequences can be shortened by digestion with a restriction endonuclease (if possible) or with a suitable exonuclease, for example Ba131. In addition, the fragments can be ligated to chemically synthesized DNA linkers which preferably include the recognition sequence of an appropriate restriction endonuclease. This allows a convenient connection of the acid phosphatase promoter with foreign polypeptide coding regions. It is also possible to isolate and/or construct a DNA fragment which contains the yeast acid phosphatase promoter and part or all of the adjacent signal sequence from the acid phosphatase protein coding region. When ligated to an appropriately cut foreign polypeptide coding region, the resulting hybrid DNA will be expressed in yeast to yield polypeptides with acid phosphotase signal sequences or fused signal sequences.

The yeast acid phosphatase promoters according to the present invention may be used to control the expression of a yeast or a non-yeast polypeptide coding region in a yeast hybrid vector.


2. Hybrid vectors containing yeast acid phosphatase promoters and their preparation

The present invention also relates to hybrid vectors consisting essentially of the repressible yeast acid phosphatase (*PHO5*) promoter as specified above and a yeast or a non-yeast polypeptide coding region which is controlled by said promoter.

The terms "vector", "hybrid vector", "DNA sequences" etc. used in the present application relate in particular to double stranded DNAs.

However, single stranded DNAs are also comprised. Vectors and hybrid vectors may be present in linear or, preferably, circular form.

The yeast or non-yeast polypeptide coding region (gene) controlled by one of the above promoters may be derived from genomic DNA or from cDNA prepared via the mRNA route or may be synthesized chemically. The non-yeast polypeptide coding regions (genes) orginate from viruses, prokaryotic cells or eukaryotic cells, including from higher eukaryotic cells, especially from human cells. When expressed in the host yeast cell, these genes can provide for the production of a wide variety of polypeptides including glycosylated polypeptides, such as enzymes which can be used, for example, for the production of nutrients and for performing enzymatic reactions in chemistry, or non-enzymatic polypeptides, for example hormones, polypeptides with immunomodulatory, anti-viral and anti-cancer properties, antibodies, viral antigens, vaccines, clotting factors, foodstuffs and the like. For example, such genes code for amylases, proteases, lysozyme, viral thymidine kinase, rennin, β-lactamase, glucose isomerase; secretin, thymosin, relaxin, calcitonin, somatostatin, human or bovine growth hormone, insulin, luteinizing hormone, parathyroid hormone, adrenocorticotropin, β-endorphin, melanocyte-stimulating hormone, β-lipotropin, urogastrone; interferon, such as human interferon, e.g. a human interferon-α or -β polypeptide derived from human leukocyte, lymphoblastoid or fibroblast cells, or human interferon-γ; lymphokines, tumour necrose factor; anti-rennin antibody, hepatitis A virus antigen, hepatitis B virus (HBV) surface or core antigens, hepatitis non-A non-B virus antigen, human histocompatibility antigens, food and mouth disease virus antigen, influenza haemagglutinin, fowl pest virus haemaglutinin; serum albumin, ovalbumin, thaumatin, eglins or plasminogen activators.

A chosen polypeptide coding region may optionally include a signal sequence or a part thereof. As indicated above, this can given rise to a fused protein containing the *PHO5* signal sequence or a hybrid signal sequence containing part of the *PHO5* signal sequence and part of the signal sequence of the foreign polypeptide together with the foreign mature polypeptide. In both instances, those combinations are favoured which lead to the cleavage of the signal sequence upon maturation of the foreign polypeptide.

Apart from an acid phosphatase promoter and a yeast or a non-yeast polypeptide coding region, the hybrid vectors according to the invention may contain additional DNA sequence(s) which are inessential or less important for the function of the promoter, i.e. for the expression of the polypeptide coding region, but which may perform important functions, for example, in the propagation of the yeast cells transformed with said hybrid vectors. The additional DNA sequence(s) may be derived from prokaryotic and/or eukaryotic cells and may include chromosomal and/or extra-chromosomal DNA sequences. For example, the additional DNA sequences may stem from (or consist of) plasmid DNA, such as bacterial or eukaryotic plasmid DNA, viral DNA and/or chromosomal DNA, such as bacterial, yeast or higher eukaryotic chromosomal DNA. Preferred hybrid vectors contain additional DNA sequences derived from bacterial plasmids, especially *Escherichia coli* plasmid pBR322 or related plasmids, bacteriophage λ, yeast 2μ plasmid, and/or yeast chromosomal DNA.

6

Preferably, the additional DNA sequences carry a yeast replication origin and a selective genetic marker for yeast. Hybrid vectors containing a yeast replication origin, e.g. the chromosomal autonomously replicating segment(ars), are extrachromosomally maintained within the yeast cells after transformation and are autonomously replicated upon mitosis. Hybrid vectors containing sequences homologous to yeast 2μ plasmid DNA can be used as well. These hybrid vectors will get integrated by recombination into 2μ plasmids already present within the cell or will replicate autonomously. 2μ sequences are especially suitable for high-frequency transformation plasmids and can give rise to high copy numbers.

In addition, the hybrid vectors according to the invention may include a DNA sequence of a gene present in the host yeast chromosome (e.g. PHO5), the promoter of which may be linked to the yeast or non-yeast polypeptide coding region. By virtue of the homologous sequence the whole vector can be stably introduced into the host chromosome by recombination. Thus, during propagation the progeny cells will retain the introduced genetic material even without selective pressure.

As to the selective gene marker for yeast, any marker gene can be used which facilitates the selection for transformants due to the phenotypic expression of the marker. Suitable markers for yeast are particularly whose expressing antibiotic resistance or, in the case of auxotrophic yeast mutants, genes which complement host lesions. Corresponding genes confer, for example, resistance to the antibiotic cycloheximide or provide for prototrophy in an auxotrophic yeast mutant, for example the URA3, LEU2, HIS3 or TRP1 gene. It is also possible to employ as markers structural genes which are associated with an autonomously replicating segment providing that the host to be transformed is auxotrophic for the product expressed by the marker.

Advantageously, the additional DNA sequences which are present in the hybrid vectors according to the invention may also include a replication origin and a selective genetic marker for a bacterial host, especially Escherichia coli. There are useful features which are associated with the presence of an E. coli replication origin and an E. coli marker in a yeast hybrid vector: Firstly, large amounts of hybrid vector DNA can be obtained by growth and amplification in E. coli and, secondly, the construction of hybrid vector is conveniently done in E. coli making use of the whole repertoire of clining technology based on E. coli. E. coli plasmids, such as pBR322 and the like, contain both E. coli replication origin and E. coli genetic markers conferring resistance to antibiotics, for example tetracycline and ampicillin, and are advantageously employed as part of the yeast hybrid vectors.

The additional DNA sequences which contain, for example, replication origin and genetic markers for yeast and a bacterial host (see above) are hereinafter referred to as "vector DNA" which together with the acid phosphatase promoter and the yeast or non-yeast polypeptide coding region is forming a hybrid vector according to the invention.

The hybrid vectors can be prepared by methods known in the art, for example by introducing into a vector DNA

a DNA fragment consisting essentially of the repressible yeast acid phosphatase (PHO5) promoter as specified above and a yeast or non-yeast polypeptide coding region such that it is controlled by said promoter.

Conveniently mapped linear or, preferably, circular vector DNA, for example bacterial plasmid DNA or the like (see above), having at least one restriction site, preferably two or more restriction sites, can be employed. Advantageously, the vector DNA already contains replication origins and gene markers for yeast and/or a bacterial host. The vector DNA is cleaved using an appropriate restriction endonuclease. The restricted DNA is ligated to the DNA fragment containing the acid phosphatase promoter and to the DNA segment coding for a yeast or non-yeast polypeptide. Prior to or after linking of the promoter and the polypeptide coding region (or simultaneously as well), it is also possible to introduce replication origins and/or markers for yeast or a bacterial host. At all events, the restriction and annealing conditions are to be chosen in such a manner that there is not interference with the essential functions of the vector DNA and of the promoter. The hybrid vector may be built up sequentially or by ligating two DNA segments comprising all sequences of interest.

Various techniques may be used to join DNA segments in vitro. Blunt ends (fully base-paired DNA duplexes) produced by certain restriction endonucleases may be directly ligated with T4 DNA ligase. More usually, DNA segments are linked through their single-stranded cohesive ends and covalently closed by a DNA ligase, e.g. T4 DNA ligase. Such single-stranded "cohesive termini" may be formed by cleaving DNA with another class of endonucleases which produce staggered ends (the two strands of the DNA duplex are cleaved at different points at a distance of a few nucleotides). Single strands can also be formed by the addition of nucleotides to blunt ends or staggered ends using terminal transferase ("homopolymeric tailing") or by simply chewing back one strand of a blunt-ended DNA segment with a suitable exonuclease, such as λ-exonuclease. A further approach to the production of staggered ends consists in ligating to the blunt-ended DNA segment a chemically synthesized linker DNA which contains a recognition site for a staggered-end forming endonuclease and digesting the resulting DNA with the respective endonuclease.

In order to be efficiently expressed, the gene coding for a yeast or a non-yeast protein must be properly located with respect to sequences containing transcriptional (acid phosphatase promoter) and translational functions (ribosome binding sites). Firstly, the ligation of the DNA segment comprising the promoter with the polypeptide coding region has to be achieved in the proper orientation. If two orientations are possible the correct one can be determined by conventional restriction analysis. Hybrid vectors containing an

7

incorrectly oriented gene insert can be re-oriented by excising the gene insert with a suitable restriction endonuclease and re-ligating the gene with the hybrid vector fragment. In any case improper orientation can be avoided by ligating two DNA segments each with different restriction sites at their ends. Furthermore, the construction of the hybrid vector should be done in such a way that it allows correct transcription initiation and termination. As to the latter point, the transcript should preferably end in a DNA sequence derived from yeast chromosomal DNA or yeast 2µ plasmid. Advantageously, the transcript ends in a DNA sequence containing transcription termination signals of a yeast gene, e.g. of PHO5 or PHO3. Secondly, a proper reading frame must be established. Ordinarily, the nucleotide sequence of both promoter region and polypeptide coding region is known prior to ligation or can easily be determined (e.g. (15)) so that there are no problems in establishing the correct reading frame. In addition, specific secondary DNA structures might be needed for even more efficient expression of the gene.

A preferred region for joining the acid phosphatase promoter to a foreign coding sequence is between the major acid phosphatase mRNA start and the ATG of the acid phosphatase coding region, for example, when using the PHO5 promoter, within a stretch of about 40 bp between the major PHO5 mRNA start and the ATG of the PHO5 acid phosphatase coding region. For a junction in this region the foreign coding sequence should have its own ATG for translation initiation, or else it has to be provided by an additional synthetic oligonucleotide.

Since many polypeptides of higher organisms are primarily expressed as pre-polypeptides consisting of signal peptides attached to the N-termini of the mature polypeptides, it may be useful to include a signal sequence in the gene insert. Suitable signal sequences are those naturally linked to the polypeptide gene to be expressed or to the acid phosphatase promoter. Alternatively, fused signal sequences may be constructed by ligating part of the acid phosphatase signal sequence with part of the polypeptide signal sequence. If the direct expression of a mature polypeptide is desired, signal sequences or parts thereof optionally following the promoter region or optionwlly preceding the mature polypeptide coding region have to be eliminated, for example by digestion with an exonuclease, e.g. with Bal31.

Intermediate products, such as vectors still lacking one or more essential functions, as well as the final hybrid vectors according to hhe invention may be transformed into a bacterial host, especially E. coli, for the above reasons (e.g. production of large amounts of intermediate products and hybrid plasmids, respectively). Bacterial vectors, such as the E. coli plasmid pBR322 and those fragments thereof which contain a bacterial replication origin and gene marker(s) are the most preferred vectors for that reason. When using such a bacterial vector, the final steps for the preparation of the yeast hybrid vectors preferably also include the introduction of a genetic marker and a replication origin for yeast.

DNA segments, which may be inserted into the bacterial vector in order to produce the hybrid vectors according to the invention, such as an autonomously replicating segment (ars, cf. (4)), sequences of yeast 2µ plasmid (2) or yeast marker DNA (cf. 16), can be isolated from yeast chromosomal DNA and yeast 2µ plasmid DNA, respectively, in a conventional manner. The gene coding for a yeast or a non-yeast polypeptide may be isolated from chromosomal or extrachromosomal DNA, derived from cDNA prepared via the mRNA route (see above) using conventional techniques (e.g. 17, 18) or may be synthesized chemically.

In a preferred embodiment of the invention, the method for the preparation of the hybrid vectors comprises the steps of

(1) constructing a yeast gene library using wild-type yeast DNA,

(2) isolating the acid phosphatase gene PHO5 gene, and cloning it into a bacterial plasmid, such as pBR322, or a biologically functional, in particular an intact replication origin and selection marker containing fragment thereof,

(3) inserting into said plasmid a genetic marker for yeast, such as the TRP1 gene, and a yeast replication origin, such as a chromosomal autonomously replicating segment or alternatively yeast 2µ plasmid sequences into an appropriate restriction site,

(4) inserting a DNA segment coding for a yeast or a non-yeast polypeptide, such as human interferon or HBV surface antigen, in such a manner that the acid phosphatase promoter controls said polypeptide coding segment, and

(5) optionally inserting a DNA sequence containing transcription termination signals of a yeast gene, e.g. of PHO5, downstream from the polypeptide coding region.

It is likewise possible to alter the order of steps, such as steps 3 to 5, for example, by first introducing the polypeptide coding segment and subsequently inserting the genetic marker and the replication origin for yeast into the recombinant plasmid obtained as a product of step 2.

Prior to inserting a gene marker for yeast, a yeast replication origin and a polypeptide coding segment, inessential functions, such as the acid phosphatase structural gene, may optionally be excised from the recombinant plasmid obtained in step 2.

Especially, the DNA segment coding for a yeast or non-yeast polypeptide is joined to the acid phosphatase promoter (step 4) in the region between the major acid phosphatase mRNA start and the ATG of the acid phosphatase coding region. Optionally, a synthetic linker containing an appropriate restriction site is introduced to allow a junction between said DNA segment and the acid phosphatase promoter.

Intermediate hybrid vectors comprising the yeast acid phosphatase promoter and still lacking the yeast or non-yeast polypeptide coding sequence are also an object of the present invention and can be prepared by the above successive steps (1), (2), (3) and optionally (5), wherein the acid phosphatase promoter is preferably terminated in the region between the major acid phosphatase mRNA start and the ATG of the acid phosphatase gene and/or, optionally, a synthetic linker containing an appropriate restriction site is introduced to allow the insertion of a DNA segment coding for a yeast or non-yeast polypeptide.

## 3. Transformation of yeast with hybrid vectors containing yeast acid phosphatase promoters

Another aspect of the present invention involves a process for the production of transformed yeast cells capable of producing yeast or non-yeast polypeptides, which process comprises transforming yeast with any of the hybrid vectors described in chapter 2.

Useful yeasts include species of the genera *Saccharomyces, Schizosaccharomyces, Torulopsis* and related genera (cf. (19)), especially strains of *Saccharomyces cerevisiae*.

The transformation of yeast with the hybrid vectors may be accomplished by procedures known from the literature, e.g. according to the method described by Hinnen et al (1). This method can be divided into three steps:

(1) Removal of the yeast cell wall.

(2) Treatment of the "naked" yeast cells (spheroplasts) with the transforming DNA in the presence of PEG (polyethyleneglycol) and Ca$^{2+}$ ions.

(3) Regeneration of the cell wall and selection of the transformed cells in a solid layer of agar.

Preferred methods:

ad (1): The yeast cell wall is removed enzymatically using various preparations of glucosidases, such as snail gut juices (e.g. Glusulase® or Helicase®) or enzyme mixtures obtained from microorganisms (e.g. Zymolyase®) in osmotically stabilized solutions (e.g. 1 M sorbitol).

ad (2): The yeast spheroplasts aggregate in the presence of PEG and local fusions of the cytoplasmic membranes are induced. The generation of "fusion-like" conditions is crucial and many transformed yeast cells will become diploid or even triploid during the process of transformation. Procedures which allow selection of fused spheroplasts can be used to enrich for transformants, i.e. transformed cells can easily be screened for among preselected fusion products.

ad (3): Since yeast cells without cell wall do not divide the cell wall has to be regenerated. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplasts and thereby facilitates regeneration of the cell wall. However, cell wall regeneration may also be obtained (although at lower efficiencies) by plating the spheroplasts onto the surface of preformed agar layers.

Preferably, the regeneration agar is prepared in a way to allow regeneration and selection of transformed cells at the same time. Since yeast genes coding for enzymes of amino acid biosynthetic pathways are generally used as selective markers (cf. chapter 2), the regeneration is preferably performed in yeast minimal medium agar. However, if very high efficiencies of regeneration are required a two step procedure might be advantageous: (1) regeneration of the cell wall in a rich complex medium, and (2) selection of the transformed cells by replica plating the cell layer onto selective agar plates.

If the hybrid vector does not contain any marker gene the transformed cells can also be identified by means of alternative methods. Such methods include, for example, *in situ* hybridization with a labeled DNA fragment homologous to sequences of the hybrid vector (e.g. according to Hinnen et al. (1)), *in situ* immunoassays provided that the antibody of the production of the introduced gene is available, or other screening methods which measure gene products encoded by the transforming plasmid(s).

Alternatively, the yeast can be co-transformed with a hybrid vector according to the invention and a second vector containing a genetic marker for yeast. If the two different vectors have DNA sequences in common (these can be bacterial sequences present on the vectors), recombination can take place leading to a fused selectable hybrid molecule.

The invention also relates to yeast hosts transformed with hybrid vectors containing a yeast acid phosphatase promoter and a yeast or a non-yeast polypeptide coding region.

## 4. Cultivation of transformed yeast cells and induction of polypeptide synthesis

To a varying extent, yeast cells transformed with autonomously replicating plasmids, for example, plasmids containing yeast 2μ plasmid DNA, tend to lose the introduced hybrid plasmid (cf. (16)). For this reason, such yeast cells have to be grown under selective conditions, i.e. conditions which require the expression of a plasmid-encoded gene for growth. Most selective markers currently in use are genes coding for enzymes of amino acid or purine biosynthesis. This makes it necessary to use synthetic minimal media deficient in the corresponding amino acid or purine base. However, some genes conferring antibiotic resistance may be used as well (e.g. genes conferring resistance to cycloheximide or to the amino-glycoside G 418 (21)). Yeast cells transformed with vectors containing antibiotic resistance genes may be grown in complex media containing the corresponding antibiotic whereby faster growth rates and higher cell densities can be reached.

Yeast cells transformed with DNA integrating into the chromosomes do not require selective growth conditions. These transformed cells are sufficiently stable to allow growth without selective pressure. For the above reason, these cells are advantageously grown in complex media.

Since the acid phosphatase promoter *PHO5* is repressible, the composition of the growth medium has to be adapted in order to obtain maximum levels of mRNA transcripts, i.e. the growth medium must contain low concentration of inorganic phosphate for depression of the *PHO5* promoter.

5. Isolation and purification of the expressed polypeptide

The invention also concerns a method for producing a yeast or a non-yeast polypeptide or a naturally occuring derivative thereof, such as human interferon or HBV surface antigen, comprising the steps of

(1) culturing a yeast strain transformed with a hybrid vector containing the yeast acid phosphatase *PHO5* promoter and a yeast or a non-yeast polypeptide coding region under appropriate nutrient conditions, and

(2) isolating and purifying said polypeptide or derivative thereof.

The transformed yeast strains according to the present invention are cultured in a liquid medium containing assimilable sources of carbon and nitrogen and inorganic salts.

Various carbon sources can be used. Examples of preferred carbon sources are assimilable carbohydrates, such as glucose, maltose, mannitol or lactose, or an acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate, which can be used either alone or in a suitable mixtures. Inorganic salts which may be used include, for example sulphates, chlorides, phosphates and carbonates of sodium, potassium, magnesium and calcium.

Additionally, the nutrient medium may also contain growth promoting substances and/or substances exerting a selection pressure in order to prevent the loss of the hybrid plasmid. Substances which promote growth include, for example, trace elements, such as iron, zinc, manganese and the like, or individual amino acids.

If the hybrid plasmid contains a gene conferring resistance to an antibiotic substance, cells containing such a hybrid plasmid will survive in a medium supplemented with the antibiotic substance whereas cells which have lost said hybrid plasmid as well as contaminating antibiotic-sensitive microorganisms will not. If the hybrid plasmid contains a gene providing for prototrophy in an auxotrophic yeast mutant, e.g. the *LEU2* or *HIS3* gene, a selection pressure can be exerted by omitting the gene product, such as leucine or histidine, in the nutrient medium.

Since the cultured yeast strain has been transformed with a hybrid plasmid containing the regulated acid phosphatase promoter *PHO5*, the content of inorganic phosphate must be reduced in the nutrient medium after the pre-culture phase in order to ensure maximum levels of mRNA transcripts and, consequently, maximum yields of polypeptides.

The cultivation is carried out employing conventional techniques. The culturing conditions, such as temperature, pH of the medium and fermentation time are selected in such a way that maximum levels of polypeptides are produced. A chosen yeast strain is preferably grown under aerobic conditions in submerged culture with shaking or stirring at a temperature of about 25° to 35°C, preferably at about 30°C, at a pH value of from 4 to 8, for example at approximately pH 7, and for about 4 to 20 hours, preferably until maximum yields of polypeptides are reached.

After the transformed yeast cells have been grown to a satisfactory cell density, the first step for the recovery of the expressed polypeptide consists in liberating the polypeptide from the cell interior. In most procedures the cell wall is first removed by enzymatic digestion with glucosidases (cf. section 3). Subsequently, the resulting spheroplasts are treated with detergents, such as Triton. Alternatively, mechanical forces, such as shearing forces (for example X-press, French press) or shaking with glass beads, may be used to break cells. The resulting polypeptide mixture can be enriched for the desired polypeptide by conventional means, such as precipitation with ammonium sulphate or trichloroacetic acid, gel electrophoresis, dialysis, chromatography, for example, ion exchange chromatography, size-exclusion chromatography, HPLC or reverse phase HPLC, and the like. The final purification of the pre-purified product can be achieved, for example, by means of antibody affinity chromatography. In principle, the purification steps (except the lysis of the cells) can be accomplished according to the method of Staehelin et al. (22) developed for the purification of human leukocyte interferon.

For example, the isolation and purification of the desired polypeptide can be performed using the following steps:

(1) lysis of the yeast cells with glucosidase,

(2) treatment with a detergent,

(3) removal of most of the non-proteinaceous material by treatment with polyethyleneimine,

(4) precipitation of the polypeptides by saturating the solution with ammonium sulphate,

(5) dialysis in an appropriate buffer mixture,

(6) column chromatography on DEAE-cellulose,

(7) affinity chromatography on a monoclonal antibody column, and

(8) molecular sizing on a suitable Sephadex®-column.

In order to obtain a sufficient pure product additional purification steps may turn out to be necessary, e.g. cation or anion exchange chromatography, adsorption on hydroxylapatite, reverse phase HPLC etc. On the other hand, one or more of the above steps may be omitted if possible, or the order of steps may be altered.

In the case where the desired polypeptide is secreted by the yeast cell into the periplasmatic space, a simplified protocol can be used: The polypeptide may be recovered without cell lysis by enzymatic removal of the cell wall or by treatment with chemical agents, e.g. thiol reagents or EDTA, which give rise to cell wall damages permitting the polypeptide to be released. In the case where the polypeptide is secreted into the culture broth, it can be recovered directly therefrom.

The polypeptides obtainable according to the present invention are useful and valuable in the treatment of human and animal diseases or in preventing them (e.g. interferon, HBV surface antigen, etc.) or can be used as foodstuffs, feed, feed additives or in enzymatic reactions (see 2 above). It is to be understood that the production of naturally occurring derivatives of said polypeptides, such as proteolytically cleaved polypeptides and/or glycosylated polypeptides, is also comprised by the present invention.

The invention concerns especially the DNA fragments, the hybrid vectors, the transformed yeast, and the processes for the preparation of polypeptides as described in the Examples.


Brief description of the drawings

In the following experimental part various embodiments of the present invention are described with reference to the accompanying drawings in which:

Figure 1 is a partial restriction endonuclease map of the plasmids pJDB207/*PHO5*, *PHO3* and pBR322/*PHO5*Bam-Sal used as sources of the *PHO5* gene or for DNA sequencing, respectively.

Figure 2 shows the localization of the *PHO5* and the *PHO3* acid phosphatase genes within a 5.1 Kb BamHI fragment isolated from a yeast gene library.

Figure 3 provides the DNA sequence of the promoter region of *PHO5*.

Figure 4 is a schematic diagram showing the construction of the plasmids p3OIFN2($8'_1$) and p3OIFN2'($8'_1$).

Figure 5 illustrates the ligation of the *PHO5* promoter DNA with the IFN-$8'_1$ cDNA in the construction of plasmid p3OIFN1($8'_1$).

Figure 6 schematically illustrates the construction of plasmid pJDB207/IFN2'($8'_1$).

Figure 7 is a schematic outline of the construction of recombinant DNA molecules containing Namalwa cDNA.

Figure 8 schematically illustrates the techniques used to synthesize the IFN mRNA specific 13mer DNA primer.

Figure 9 is a schematic diagram showing the identification of clones containing human lymphoblastoid IFN cDNA.

Figures 10 to 14 provide the DNA and corresponding amino acid sequences of the cDNA inserts of the plasmids CG-pBR322/HLycIFN-1'b, $-\beta_1$, $-4_1$, $-8'_1$, and $-5_1$.

Figure 15 depicts the construction of the plasmid CG-pBR(AP)/LyIFN-α-1 and figure 16 shows the DNA and the amino sequences of its cDNA insert.

Figure 17 depicts the construction of the plasmid CG-pBR(AP)/LyIFN-α-3 and figure 18 shows the DNA and the amino acid sequences of its cDNA insert.

Figure 19 shows the DNA and the amino acid sequences of the cDNA insert of the plasmid CG-pBR(AP)/LyIFN-α-2.

Figure 20 is a schematic outline of the construction of plasmid p31 containing a *PHO5* termination fragment.

Figure 21 shows the nucleotide sequence of the Sau3A-PstI *PHO5* transcription termination fragment.

Figure 22 is a schematic outline of the construction of plasmids p31/IF1($5_1$), p31/IF2($5_1$), p31/IF3($5_1$) and p31/IF2(1'b).

Figure 23 is a schematic diagram showing the construction of the plasmid p31/IF($8'_1$).

Figure 24 schematically illustrates the construction of a correct *PHO5*-HBVs junction in plasmid pBR322/*PHO5*/HBVsΔ14.

Figure 25 shows the DNA sequence in the vicinity of the *PHO5* promoter and HBVs coding region fusion point in plasmid pBR322/*PHO5*/HBVs.

Figure 26 is a schematic diagram showing the construction of the yeast expression plasmids pJDB207/*PHO5*/HBVsΔ14 and pJDB207/*PHO5*/HBVsΔ14t.

Figure 27 is a schematic diagram showing the construction of the yeast expression plasmids pJDB207/IF2(1'b)Δ and pJDB207/IF2($5_1$)Δ72.

Figure 28 displays the nucleotide sequences of plasmids pJDB207/IF2($5_1$) Δ72 and pJDB207/IF2($5_1$)Δ82 around the XhoI junction between the 3' nontranslated region of IFN-$5_1$ and the *PHO5* transcription termination region.

Figure 29 is a schematic diagram showing the construction of plasmid CG-pBR322/HLycIFN(α-3)-252.

Figure 30 shows the structures of plasmids CG-pBR322/HLycIFN(α-2)-261 and CG-pBR322/HLycIFN(α-1)-258.

Figure 31 displays a schematic outline of the process for deleting the *PHO5* signal sequence in expression plasmid p31 and specifically shows the construction of plasmid p31/R.

Figure 32 schematically shows the collection of clones obtained in the process outlined in figure 31.

Figures 33 and 34 display the nucleotide sequences of the BamHI-EcoRI restriction fragments containing the *PHO5/R* and *PHO5/Y* promoter regions.

Figures 35 to 37 schematically display the process of inserting IFN-α-3, -α-2 and -α-1 DNA into plasmid p31/R.

Figure 38 is a schematic diagram showing the construction of plasmid pJDB207R/IF(α-3).

The following Examples serve to illustrate the present invention but should not be construed as a limitation thereof.

Experimental part

The following abbreviations are used in the Examples:

EtBr: ethidium bromide
BSA: bovine serum albumin
DTT: 1,4-dithiothreitol (1,4-dimercapto-2,3-butanediol)
EDTA: ethylenediaminetetraacetic acid
SDS: sodium dodecyl sulphate
TNE: solution containing 100 mM NaCl, 10 mM Tris · HCl (pH 7.5), and 1 mM EDTA.
Tris · HCl: tris-(hydroxymethyl)-aminomethane, pH adjusted with HCl
PMSF: - phenylmethanesulphonylfluoride
TE: solution containing 10 mM Tris · HCl (pH 7.5) and 1 mM EDTA

Example 1: Construction of a yeast gene library

Thiry µg of total high molecular weight yeast DNA (23) from wild type *Saccharomyces cerevisiae* strain S288C is incubated for 30 min at 37°C with 2 units of EcoRI methylase (New England Biolabs) in 250 µl of EcoRI methylation buffer as recommended by the supplier. DNA is precipitated by ethanol, resuspended in 500 µl of 25 mM Tris · HCl pH 8.5, 2 mM MgCl$_2$ (EcoRI* buffer) (24) and digested with EcoRI (Boehringer) until the size distribution of the DNA fragments has a maximum in the 30—50 kb range (a XhoI digest of λDNA provides appropriate 33 kb and 17 kb markers). The yeast DNA digested under EcoRI* conditions is size-fractionated on a sucrose gradient (5—20% sucrose in 10 mM Tris · HCl pH 7.5, 1 mM EDTA) for 6 hrs at 38,000 rpm in a SW 40 rotor. Thirty fractions of 0.4 ml each are collected from the top of the gradient. Fraction 16 contains DNA fragments of 30—40 kb in size. The DNA of this fraction (3 µg) is precipitated with ethanol and ligated for 16 hours at 15°C in a total volume of 15 µl to 1 µg of cosmid vector pYcl (25), linearized by EcoRI. Ligation is carried out with 300 U T4 DNA ligase (New England Biolabs) using the buffer system described by the supplier. The DNA is packaged *in vitro* into bacteriophage λ (26) and the assembled phages are used to transduce *E. coli* strain HB101 ($r_K^-$, $m_K^-$, *leu*$^-$, *pro*$^-$, *recA*$^-$). The efficiency of transduction is about 500 ampicillin-resistant colonies per µg of pYcl vector. 3000 amp$^R$ colonies are picked and grown individually in the walls of microtiter dishes in LB medium [10 g Bacto-Tryptone (Difco), 5g Bacto Yeast Extract (Difco), 10 g NaCl] containing 100 µg/ml ampicillin.

Example 2: Isolation of the regulated acid phosphatase gene *PHO5*

Replicas of the gene library are grown on LB agar plates (LB medium plus 15 g/l agar) containing 100 µg/ml ampicillin. The cell material from 500 colonies is washed off the plates and pooled. DNA is isolated from individual pools using the following protocol:

The cells are harvested by centrifugation (Sorvall, GSA rotor, 10 min at 6000 rpm, 4°C), resuspended in 100 ml TE (10 mM Tris · HCl, 1 mM EDTA, pH 8.0) and centrifuged again under the above conditions. The cell pellet is resuspended in 3 ml Tsuc [50 mM Tris · HCl, pH 7.5, 25% (w/v) sucrose] and transferred to SS-34 polypropylene Sorvall tubes. All subsequent steps are carried out on ice: 0.3 ml of lysozyme solution (10 mg/ml, purchased from Worthington, 11,000 U/mg) is added, after 5 min 1.2 ml EDTA (500 mM, pH 8.0), and after another 5 min 4.8 ml detergent [0.1% Triton X-100 (Merck), 50 mM EDTA, 50 mM Tris · HCl, pH 8.0] are added. After 5 min the lysate is centrifuged in a precooled SS-34 rotor for 40 min at 4°C. The supernatant is carefully removed and solid CsCl is added (8.3 g CsCl to 8.7 ml of supernatant). After the addition of ethidium bromide (Sigma) (final concentration 1 mg/ml supernatant) the solution is transferred to 13.5 ml Quick Seal polyallomer tubes (Beckman) and centrifuged in a Beckman Ti50 rotor for 40 hrs at 40,000 rpm. Two fluorescent bands can be visualized with long wave UV (366 nm). The lower band contains supercoiled plasmid DNA which is collected by puncturing the tube from the side with a 2 ml syringe (18G needle). The ethidium bromide is removed by extracting 5 times with equal volumes of isopropanol (saturated with CsCl) and the product is transferred to 30 ml Corex tubes. 2.5 volumes of TE is added and the DNA is precipitated with ethanol. The solution is then kept for 12—15 hrs at −20°C. The precipitated DNA is collected by centrifugation in a Sorvall HB-4 rotor for 30 min at 12,000 rpm at 0°C and redissolved in 200 µl of TE. 50—100 µg of hybrid plasmid DNA are recovered from a 100 ml culture.

Plasmid DNA from these pools is used to transform *S. cerevisiae* strain AH216 (*a, his3, leu3, pho3, pho5*) according to the procedure described by Hinnen et al. (1). Yeast transformants are replicated on low

**EP 0 100 561 B1**

$P_i$-minimal medium [as "Difco yeast minimal medium without amino acids" supplemented with 20 g/l glucose, but prepared from the components according to the recipe of Difco (Difco Manual, Difco Laboratories, Detroit, USA) except that 0.03 g/l $KH_2PO_4$ plus 1 g/l KCl is used instead of 1 g/l $KH_2PO_4$] and stained for acid phosphatase activity by overlayering them with staining agar [1% Difco agar in 100 mM acetate buffer pH 4.0, 2 mg/ml Fast Blue B Salt (Serva) and 0.2 mg/ml α-naphthyl phosphate (Serva)]. Colonies with a functional *PHO5* gene srain red upon derepression of the gene on low $P_i$-medium. By repeated subpooling (17) of the gene library 3 independent clones exhibiting repressible acid phosphatase activity are obtained.

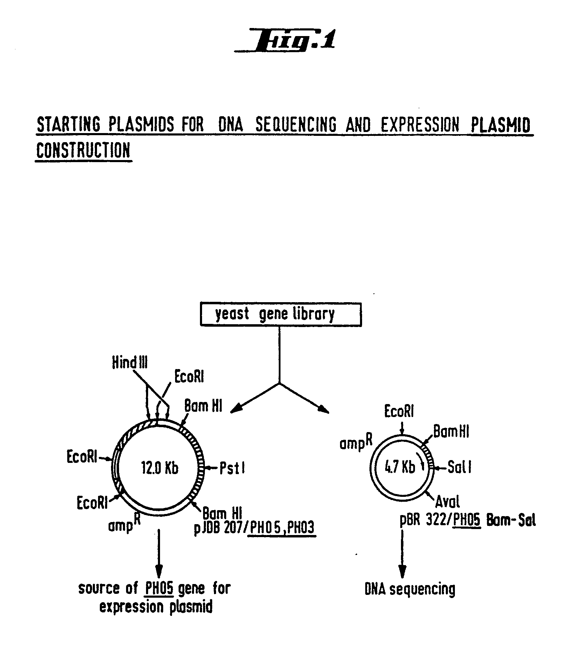

One of these clones (pG7) is further analysed. The hybrid plasmid has a size of 42 kb. EcoRI and BamHI fragments of pG7 are subcloned in pBR322/*HIS3* (16) and pJDB207 (28) respectively. Restriction digests are as recommended by the supplier (New England Biolabs) and ligations are performed in 20 μl with 150 U T4 DNA ligase (New England Biolabs) and 20 μg/ml of the individual digested plasmids (conditions as suggested by New England Biolabs). A 5.1 kb BamHI fragment which is part of a 8 kb EcoRI fragment is subcloned in yeast vector pJDB207 and, upon transformation of yeast strain AH216, this hybrid plasmid (pJDB207/*PHO5, PHO3*, see fig. 1) elicites high phosphatase activity under derepressed (low $P_i^-$) conditions (*PHO5* gene) and low levels of activity in normal yeast minimal medium (expression of the *PHO3* gene).

Example 3: Localisation of the *PHO5* gene and DNA sequence analysis

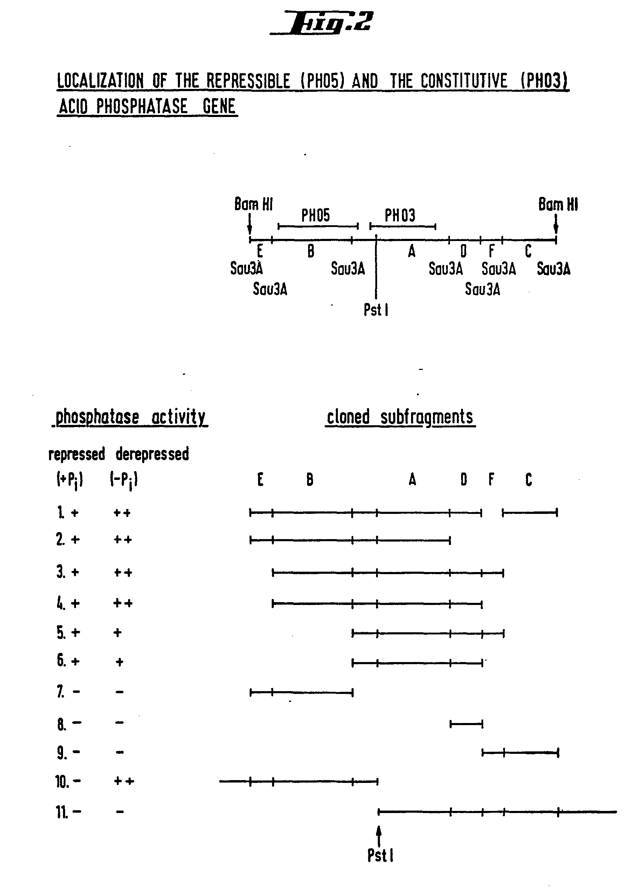

For the localisation of *PHO3* and *PHO5* within the BamHI fragment advantage is taken of the pattern of Sau3A restriction sites and a unique PstI site. Digestion of the BamHI fragment with restriction endonuclease Sau3A (New England Biolabs) generates 6 fragments (A-F, fig. 2). Subcloning of a partial Sau3A digest into the BamHI site of self-replicating yeast vector pJDB207 leads to plasmids with different combinations of Sau3A fragments. These plasmids are then used to transform the *pho3, pho5* mutant yeast *S. cerevisiae* AH216. Transformants are checked for acid phosphatase activity after growth on either low $P_i$- or normal minimal medium plates. Clones containing at least Sau3A fragments A and B (fig. 2, No. 1—4) express acid phosphatase at the same level (qualitative estimates after overlayering with acid phosphatase staining agar, as described in Example 2) as the entire 5.1 kb BamHI fragment. Expression is regulated normally by the concentration of inorganic phosphate in the medium. Clones with Sau3A-fragment A only (fig. 2, No. 5, 6) express low levels of acid phosphatase, which is not influenced by the inorganic phosphate concentration in the medium. This indicates that information carried by the Sau3A fragment A is sufficient for constitutive acid phosophatase (*PHO3*) expression. Sau3A fragment B (fig. 2, No. 7) alone does not lead to any expression of acid phosphatase under either repressed or derepressed conditions. However, a subclone with the complete sequence between the BamHI and PstI sites (fig. 2, No. 10) shows regulated, but not constitutive synthesis of acid phosphatase. This subclone must therefore contain the yeast *PHO5* gene (16).

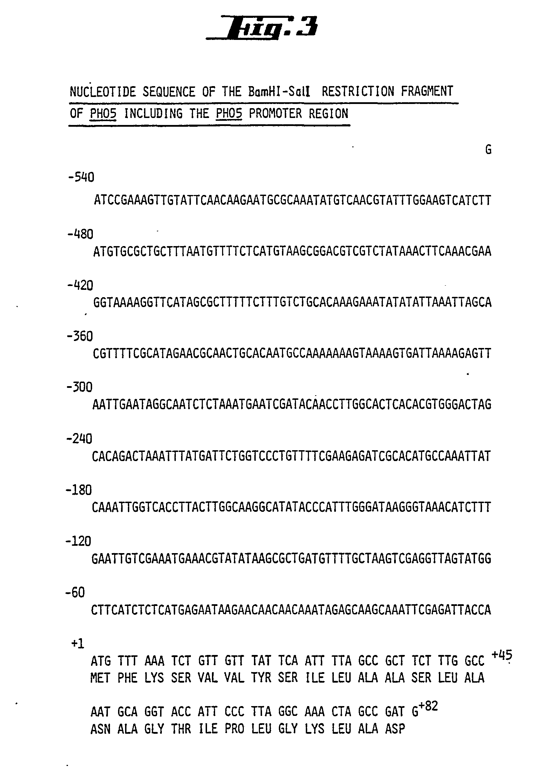

The exact localisation of the *PHO5* gene is determined by DNA sequencing using the method of Maxam and Gilbert (15). A 623 bp BamHI-Sall restriction fragment is cloned into plasmid pBR322 (see fig. 1), replacing the BamHI-Sall fragment which extends from position 375 to 650 (pBR322 nomenclature), using digestion and ligation conditions as described above (all enzymes are from New England Biolabs). DNA fragments of the BamHI-SalI DNA insert are asymmetrically labelled at their 5' ends at the following sites: BamHI (−541), Sau3A(−200) and SalI (+82), (for numbering see fig. 3a). The nucleotide sequence of the 623 bp BamHI-SalI DNA insert is depicted in fig. 3a. It reveals that the insert contains the *PHO5* promoter region and part of the *PHO5* phosphatase protein coding region.

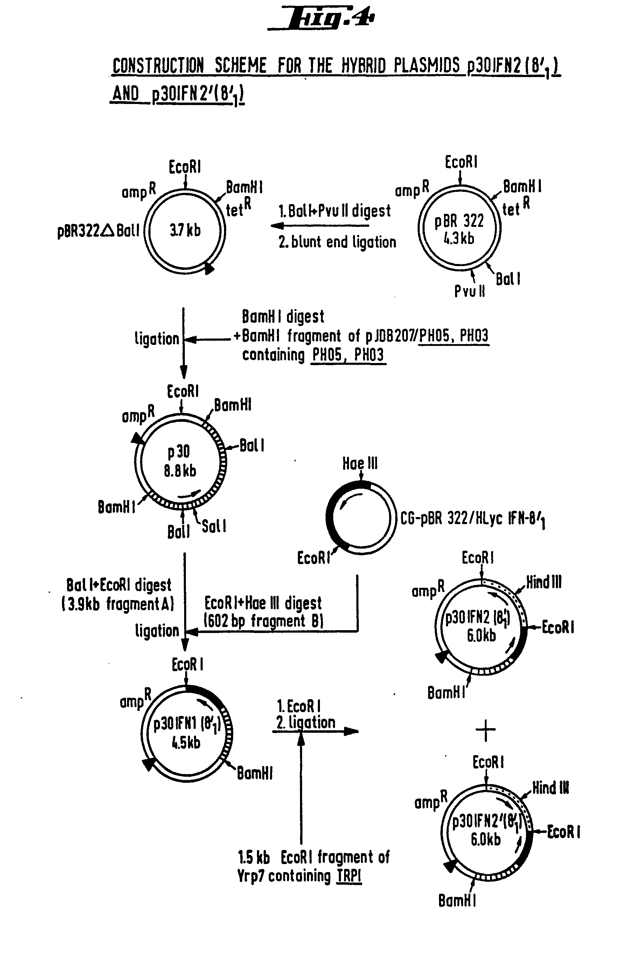

Example 4: Construction of plasmid p30 (see fig. 4).
a) Elimination of the Ball restriction site in plasmid pBR322 .

The scheme outlined in fig. 4 requires elimination of the unique Ball restriction site in plasmid pBR322. 3 μg of pBR322 are digested to completion with restriction endonucleases Ball (BrL) and PvuII (Bio-labs) according to the recommendations of the suppliers. The Ball/PvuII double digest of pBR322 results in two restriction fragments of 3738 bp and 622 bp in size. The two fragments are separated on a 1% low melting agarose gel (Sigma) in TBE (90 mM Tris · HCl pH 8.3, 2.5 mM EDTA, 90 mM boric acid) buffer. The DNA bands are stained with ethidiumbromide and visualized under long wave UV light at 366 nm. The piece of agarose containing the 3738 bp fragment is cut out from the gel, liquified at 65°C, adjusted to 500 mM NaCl and incubated at 65°C for 20 min. One volume of phenol (equilibrated with 10 mM Tris · HCl pH 7.5, 1 mM EDTA, 500 mM NaCl) is added. The aqueous phase is reextracted twice with phenol and once with chloroform. The DNA is precipitated with 2.5 volumes of cold absolute ethanol and collected by centrifugation. The DNA pellet is washed with cold 80% ethanol and then dried in vacuum. The DNA is resuspended in TE at a concentration of 0.15 mg/ml.

The isolated 3738 bp DNA fragment has two blunt ends resulting from the Ball and PvuII double digests. The DNA is circularized by blunt end ligation. 0.6 μg of DNA are incubated over night at room temperature in 30 μl of 60 mM Tris · CHI pH 7.5, 10 mM $MgCl_2$, 10 mM DTT, 4 mM ATP, and 900 U of T4 DNA ligase (Biolabs). 5 μl aliquots of the ligation mixture are added to 50 μl of calcium treated, transformation competent *E. coli* HB101 cells, prepared by the method of Mandel et al. (29). The mixture is kept on ice for 5 min, then incubated for 2 min at 37°C and left 10 min at room temperature before plating on LB agar plates containing 100 μg/ml of ampicillin. Six amp[R] colonies are picked and grown individually in 100 ml of LB (as

13

above but without agar) medium containing 100 µg/ml ampicillon. Plasmid DNA is prepared from the cells using the procedure described in Example 2. Restriction digests with HaeIII (purchased from Biolabs, digestion conditions as suggested by supplier), PvuII and BalI of the plasmids are analyzed on a 1.5% agarose gel in TBE buffer. The restriction pattern and the predicted size of the newly formed junction fragment indicates that the plasmids are identical and contain all of the pBR322 sequences except for the BalI—PvuII fragment. These plasmids lack the BalI restriction site and are referred to as pBR322ΔBalI.

b) Cloning of a yeast 5.1 kb BamHI restriction fragment containing *PHO5* and *PHO3* into pBR322ΔBalI

pJDB207/*PHO5,PHO3* (see fig. 1) contains a yeast 5.1 BamHI insert with the genes for regulated and constitutive yeast acid phosphatase *(PHO5* and *PHO3)*. pJDB207/*PHO5,PHO3* as well as plasmid pBR322ΔBalI are digested with restriction endonuclease BamHI. After complete digestion the enzyme is inactivated for 2 min at 65°C. Both DNAs are precipitated by ethanol and resuspended in 10 mM Tris · HCl pH 8.0 at a concentration of 0.2 mg/ml each. 0.5 µ of each of the two BamHI-digested DNAs are combined and ligated in 20 µl of ligation buffer (as suggested by New England Biolabs), containing 300 U of T4 DNA ligase, for 20 hrs at 15°C. 5 µl aliquots of the ligation mixture are added to 50 µl of calcium-treated *E. coli* HB101 cells and transformation is carried out as described in Example 4a. The transformed *E. coli* cells are tested for their resistance towards ampicillin and tetracyclin. Eight amp$^R$, tet$^S$ coloniss are isolated and grown in 100 ml of LB medium containing 100 µg/ml of ampicillin. Plasmid DNA is isolated from the cells (see Example 2). Restriction digsts with BamHI show that 4 plasmids contain a 5.1 kb insert besides the 3.7 kb vector fragment (pBR322ΔBalI). Restriction digests with SalI (New England Biolabs) determine the orientation of the inserted 5.1 kb fragment: two plasmids have the insert orientated as shown in figure 4. One of them is referred to as p30. The direction of transcription of the *PHO5, PHO3* genes in the 5.1 kb insert is anticlockwise as indicated in figure 4.

Example 5: Insertion of foreign DNA into p30 (see fig. 4)

a) Isolation of a 3.9 kb EcoRI-BalI fragment of p30 (fragment A)

10 µg of p30 DNA are digested with restriction endonuclease BalI. After extraction with phenol/chloroform, the DNA is precipitated with ethanol. The DNA is resuspended in 100 µl TE buffer. The restriction fragments are separated on a preparative 0.8% low melting agarose gel (Sigma). A 5.1 kb fragment, containing the vector part p30 is eluted from the gel as described in Example 4a. The DNA is purified by adsorbing the DNA on a DE52 (Whatman) ion exchange column in a low salt buffer (150 mM NaCl, 10 mM Tris · HCl pH 8.0, 1 mM EDTA) and then eluting it with a high salt buffer solution (1.5 M NaCl, 10 mM Tris · HCl pH 8.0 and 1 mM EDTA). The DNA is precipitated with ethanol and then further digested with EcoRI (Boehringer). The 3.9 kb EcoRI-BalI restriction fragment is again separated on a preparative 0.8% low melting agarose gel, recovered as described in Example 4a and ethanol precipitated. This DNA fragment is called fragment A.

b) Isolation of a 602 bp HaeIII-EcoRI fragment of CG-pBR322/HLycIFN-8'$_1$ (fragment B)

*E. coli* strain HB-101 CG-pBR322/HLycIFN-8'$_1$ (see Example 10E) is grown in 100 ml LB medium supplemented with 10 µg/ml tetracyclin and plasmid DNA is isolated as described in Example 2. Nine µg of HLycIFN-8'$_1$ DNA are completely digested with restriction endonuclease HaeIII. The restriction fragments are separated on a preparation 0.8% low melting agarose gel. A 940 bp HaeIII fragment is cut out and eluted from the agarose gel as described in Example 4a. The DNA is purified on DE52 as described in Example 5a and then further digested with EcoRI. The 602 bp EcoRI-HaeIII fragment is again separated on a preparative 0.8% low melting agarose gel, recovered as described in Example 4a and ethanol precipitated. This DNA fragment is called fragment B.

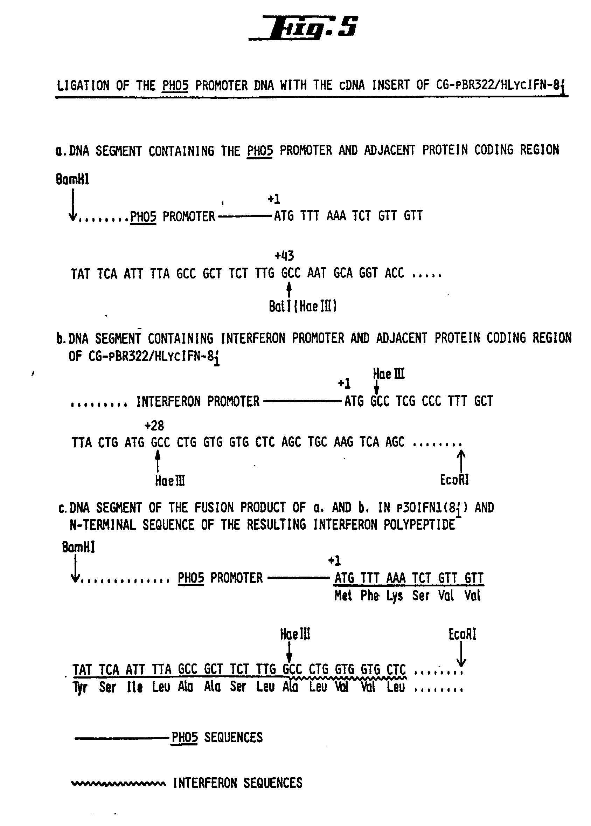

c) Ligation of fragments A and B (see fig. 5)

The two restriction fragments can be ligated enzymatically via the EcoRI sticky ends and the blunt ends of BalI and HaeIII respectively, thus creating a circular molecule with a unique EcoRI site and a BalI-HaeIII junction which is cleavable with HaeIII (but not with BalI).

The ligation is carried out in a buffer system containing 60 mM Tris · HCl pH 7.5, 10 mM MgCl$_2$, 10 mM · DTT, 4 mM ATP, 300 units of T4 DNA ligase for 16 hrs at 23°C at a DNA concentration of 20 µg/ml of fragment A and 3 µg/ml of fragment B in a total volume of 10 µl.

d) Transformation of *E. coli* HB101 with the ligated fragments

2 µl aliquots of the ligation mixture (see Example 5c) are added to 50 µl of calcium-treated *E. coli* UB101 cells (see Example 4a). The mixtures are then plated on LB agar plates supplemented with 100 µg/ml ampicillin. The plates are incubated at 37°C for 16 hrs. About 300 ampicillin resistant colonies of *E. coli* HB101 are prepared. Plasmid DNA from eight ampicillin resistant colonies is isolated, analysed and their structure is determined by comparing the mobility of the restriction fragments obtained after cleavage with EcoRI and HaeIII with standard DNA [bacteriophage λDNA digested with HindIII (New England Biolabs), p30 plasmid DNA digested with HaeIII and EcoRI]. After verification of the structure of the junctions, 5 plasmids are obtained which have the correct structure. One of these plasmids containing the *PHO5* promoter linked to the 8'$_1$-interferon polypeptide coding region (see fig. 5) is called p30IFN1(8'$_1$).

Example 6: Addition of replication origin and selective marker for yeast (see fig. 4)

a) Isolation of a 1.5 kb EcoRI fragment from plasmid Yrp7 and its ligation into plasmid p30IFN1(8'$_1$)

In order to facilitate the ligation reaction the 1.5 kb EcoRI restriction fragment is purified. Plasmid Yrp7 (4) is cut with EcoRI, the two fragments obtained are separated on a 0.8% agarose gel and the 1.5 kb fragment containing a yeast autonomously replicating segment and the yeast *TRP1* gene is purified and isolated as described in Example 4a. Ligation is carried out (as suggested by New England Biolabs) with 20 µg/ml of EcoRI cut p30IFN1(8'$_1$) and 10 µg/ml of the 1.5 kb EcoRI restriction fragment from Yrp7; 100 units of T4 ligase are used.

b) Transformation of *E. coli* JA 194 with the ligated fragments

Plasmids containing the *TRP1* yeast gene are directly selectable by transformation of the *E. coli trpC* mutant strain JA 194 (*trpC, leuB, B*$_1$). The *E. coli trpC* gene codes for the *E. coli* N - (5' - phosphoribosyl) - anthranilate isomerase. *E. coli trpC* mutants can be complemented by the yeast *TRP1* gene (4). Transformation of *E. coli* strain JA 194 is carried out as described for *E. coli* HB101 (see Example 4a) except for the following modification: before plating the mixtures onto agar plates the cells are allowed to recover in 1 ml of LB medium at 37°C for 60 min; the cells are washed once with *E. coli* M9 minimal medium (30) and plated onto M9 minimal medium plates supplemented with vitamine B1 (1 µg/ml) and L-leucine (20 mg/ml). The plates are incubated for 2 days at 37°C. Approximately 1000 tryptophan prototrophic *E. coli* colonies are recovered.

c) Isolation and characterization of hybrid plasmids

Trp$^+$ colonies are purified on LB plates supplemented with 100 µg/ml ampicillon. Individual colonies are picked and plasmids are isolated as described in Example 2. Purified plasmids are analyzed by measuring the size of the restriction fragments generated after cleavage with EcoRI, HindIII, PstI and BglII (Biolabs). Two different types of plasmids are obtained which contain the 1.5 kb EcoRI restriction fragment in the two possible orientations (see fig. 4). They are named p30IFN2(8'$_1$) and p30IFN2'(8'$_1$) as indicated in figure 4.

Example 7: Transformation of *Saccharomyces cerevisiae* RH971 and inducation of interferon production

Plasmids p30IFN2(8'$_1$) and p30IFN2'(8'$_1$) are each introduced into *Saccharomyces cerevisiae* strain RH971 (*a, trp1, leu2, his4*) in analogy as described by Hinnen et al. (1). One µg of plasmid DNA is added to 100 µl of a spheroplast suspension and the mixture is treated with polyethylene glycole as described (1). The spheroplasts are mixed with 10 ml regeneration agar and plated onto yeast minimal medium plates without leucine. After incubation for 3 days at 30°C, about 1000 transformed cells are obtained.

One single yeast colony from the yeast transformation plates [named *Saccharomyces cerevisiae* RH971/p30IFN2(8'$_1$) and /p30IFN2'(8'$_1$) respectively] is picked into 10 ml of yeast minimal medium in a 100 ml Erlenmeyer flask, and grown at 30°C at 200 rpm for 24 hrs to a density of about 2—3×10$^7$ cells/ml. The cells are washed once with 20 ml of low-P$_i$ minimal medium. Three ml of the resuspended cells are used to inoculate 300 ml low-P$_i$ minimal medium and 300 ml normal minimal medium, respectively, in 1000 ml Erlenmeyer flasks. Incubation is at 30°C at 160 rpm. Induction of the *PHO5* promoter is followed by measuring the appearance of acid phosphatase activity in whole cells as described by Toh-e et al. (31). The cells are grown to about 1—2×10$^7$ cells/ml (26—30 hrs of incubation).

Example 8: Preparation of yeast cell extracts and determination of the interferon titer

Cells from the 300 ml culture medium (see Example 7) at a density of 1—2×10$^7$/ml are collected by centrifugation in a Sorvall GSA rotor for 5 min at 8000 rpm at 4°C. The cells are washed once with 100 ml H$_2$O, resuspended in 6 ml ice cold lysis mix [0.1 M potassium phosphate buffer pH 7.4, 1% (v/v) Triton X-100,0.0001M PMSF (Merck)] and transferred to a 30 ml corex tube. The suspension is centrifuged again for 5 min in a Sorvall SS-34 rotor at 8000 rpm at 4°C and resuspended in 3 ml lysis mix at 0°C. Four g of glass beads (0.4 mm in diameter) are added to the cells and the suspension is shaken on a Vortex Mixer (Scientific Instruments Inc., USA) at full speed for 30 sec and then cooled for 1 min in an ice bath. This shaking procedure is repeated 5 to 10 times until more than 90% of the cells are broken (check under light microscope). Cell debris and glass beads are removed from the solution by centrifugation for 10 min at 8000 rpm at 4°C in a Sorvall HB-4 rotor. The supernatant is transferred to Eppendorf tubes, frozen in liquid nitrogen and stored at −60°C. Interferon activity is determined according to the procedure of Armstrong (32) using human CCL-23 cells and visicular stomatitis virus (VSV) as the challenge virus. The results are summarized in Table 1.

TABLE 1

Interferon activity in *Saccharomyces cerevisiae* strain RH971 after transformation with the recombinant plasmids p30IFN2(8′₁) and p30IFN2′(8′₁), respectively, and also with plasmid pJDB207/IFN2′(8′₁) (see Example 9)

| Plasmid | Interferon activity expressed in units/ml yeast cell extract | | |
|---|---|---|---|
| | p30IFN2(8′₁) | p30IFN2′(8′₁) | pJDB207/IFN2′(8′₁) |
| Repressed conditions (normal phosphate content) | 0 | 30 | 100 |
| derepressed conditions (low phosphate content) | 700 | 7000 | 50000 |

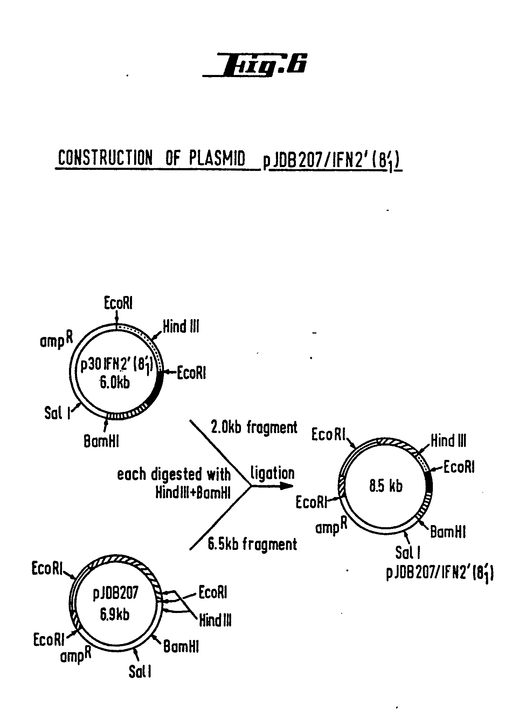

Example 9: Insertion of the interferon gene into the high copy number yeast 2μ vector pJDB 207 (see Fig. 6)

Plasmid p30IFN2′(8′₁) is digested with restriction endonucleases HindIII and BamHI according to the specifications of the supplier (Biolabs). Two fragments are generated of the size of 4.0 kb and 2.0 kb. The 2.0 kb restriction fragment is separated and purified by low melting agarose gel electrophoresis as described under step 4a.

Plasmid pJDB207 (28) is digested with restriction endonucleases HindIII and BamHI. Three fragments are generated. The 6.5 kb restriction fragment is separated as above.

0.3 μg of the 2.0 kb fragment (containing the *PHO5* promoter linked to the interferon protein coding region) is ligated for 15 hrs to the 6.5 kb vector fragment in a total volume of 20 μl using 300 U T4 DNA ligase under conditions described by the supplier (Biolabs). *E. coli* HB101 cells are transformed and ampicillin resistant colonies are selected. The plasmid DNA is isolated and the correct structure of the isolated plasmid DNA is verified by restriction digestions using HindIII and BamHI, with p30IFN2′(8′₁) and pJDB207 digested with the same enzymes as molecular weight standards. The new plasmid obtained is called pJDB207/IFN2′(8′₁).

Plasmid pJDB207/IFN2′(8′₁) is transformed into *S. cerevisiae* strain RH971 in analogy as described (1) selecting for leucine prototrophic colonies. One single leucine prototrophic yeast colony [named *Saccharomyces cerevisiae* RH971/pJDB207/IFN2′(8′₁)] is picked and grown as described in Example 7. The interferon titer is determined as described in Example 8. The results are depicted in Table 1.

Example 10: Production of *E. coli* strains transformed with recombinant plasmids containing the coding regions for human lymphoblastoid interferons

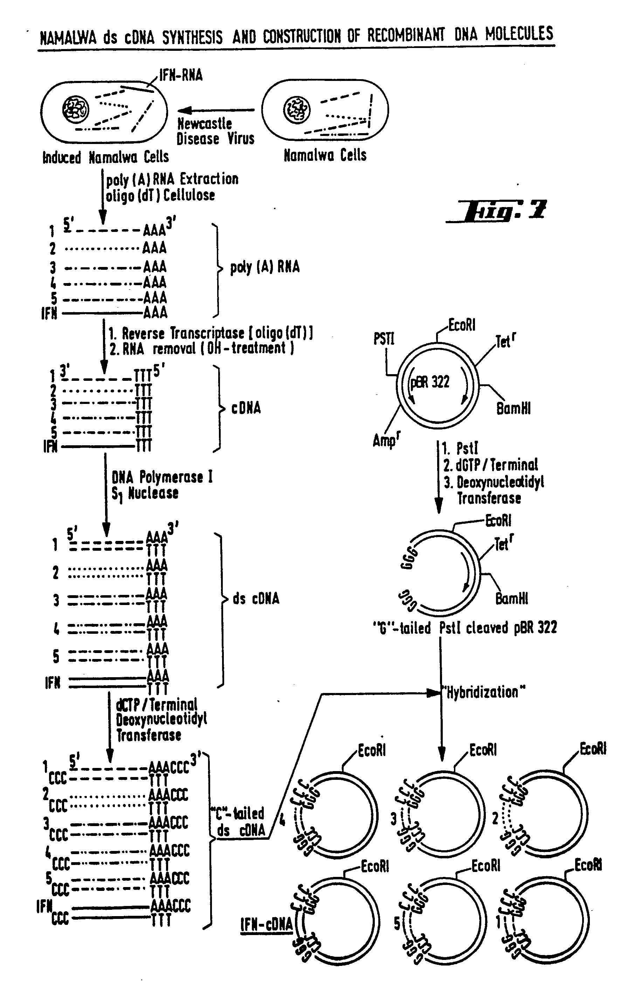

A. Isolation of poly(A) RNA enriched for HuIFN mRNA (figure 7)

a) Induction of the Namalwa cells

Namalwa cells are grown in culture medium RPMI 1640 containing 10% fetal calf serum at 37°C. When a cell density of $3 \cdot 10^6$ cells/ml is reached, the suspension is centrifuged at 800×g for 10 minutes at room temperature. The collected cells are resuspended in 200 ml of culture medium containing glutamine (0.027% by volume), penicillin (200 units/ml) and streptomycin (50 μg/ml). The cells are incubated for 90 minutes at 37°C with Newcastle disease virus (NDV 110) at a ratio of 190 HAU/10⁶ cells (HAU: haemagglutination units. By adding fresh culture medium the cell density is adjusted to $1.3 \cdot 10^6$ cells/ml and the cell suspension is shaken at 34°C at 100 rpm. After 12 h, $6 \cdot 10^9$ cells are harvested and resuspended in 50 ml phosphate-buffered saline ("PBS"; 1 l PBS contains 80 g NaCl, 2 g KCl, 14.4 g $Na_2HPO_4$ and 2 g $KH_2PO_4$). Before harvesting the cells, a sample is removed and the interferon activity is determined according to the procedure of Armstrong (32) using human CCL-23 cells and vesicular stomatitis virus (VSV) as the challenge virus. 4300 IFN units/ml are found.

b) Disruption of the cells and deproteinization

The cell suspension ($6 \cdot 10^9$ cells in 50 ml PBS) is added at room temperature to 800 ml lysis buffer consisting of 0.05 M Tris · HCl (pH 7.5), 0.1 M NaCl, 5 mM EDTA and 2% SDS (cryst. research grade, Serva). The lysate is digested with 0.2 mg/ml of preincubated (2 h at 37°C) protease (Protease P, type VI, Sigma) at room temperature for 1 h while stirring the solution. The solution is deproteinized by extracting 3 times with 500 ml phenol saturated with TNE and 5 times with 500 ml chloroform. 500 mg of nucleic acids are obtained as measured by absorbance at 260 nm.

c) Removal of contaminating DNA and RNA

The slightly viscous aqueous solution obtained as described above (step Ab) is adjusted to 0.3 M NaCl and 1 g of oligo(dT) cellulose (type 7, P-L Biochemicals) is added. After stirring for 30 min at room temperature the suspension is centrifuged in 1 l Sorvall bottles in a Sorvall RC-3 centrifuge at 4000 rpm for 10 min at room temperature and the oligo(dT) cellulose slurry is washed twice with 40 ml 2×TNE containing 0.5% SDS. The bound poly(A) RNA is then eluted by five successive washes with 2.5 ml $H_2O$. The yield is 720 µg poly(A) RNA as determined by measuring the optical density. The supernatant RNA solution from the first adsorption is adsorbed a second time to 1 g of oligo(dT) cellulose and eluted as described above, yielding 320 µg poly(A) RNA. The eluates are pooled, adjusted to TNE and the poly(A) RNA is precipitated with 67% ethanol at −20°C for 10 hours. The RNA is collected by centrifugation at 10 000 rpm in a Sorwall RC-5B centrifuge for 10 min at 0°C. The precipitate (1 mg) is redissolved in 1 ml of 1 mM EDTA.

The RNA is assayed for HuIFN mRNA activity by injection into oocytes of Xenopus laevis as follows:

50 nl of the RNA solution is injected into each of 20 oocytes. The oocytes are incubated in Barth medium (2 mM Tris, 88 mM NaCl, 1 mM KCl, 0.33 mM $Ca(NO_3)_2 \cdot H_2O$, 0.41 mM $CaCl_2 \cdot 2H_2O$, 0.82 mM $MgSO_4 \cdot 7H_2O$, 2.4 mM $NaHCO_3$, 0.01 mg/ml penicillin, 0.01 mg/ml streptomycin; the solution is adjusted to pH 7.6 with HCl) according to Gurdon (33), Barth (34) and Colman et al. (35). The injected oocytes are incubated for 42—48 hours and the incubation medium is removed, centrifuged for 5 min in an Eppendorf centrifuge, and the supernatant is stored at −20°C or −80°C until it is used for assay. The IFN activity is assayed essentially according to Armstrong (32), except that VSV is used as the challenge virus on Hep-2-cells (Flow Laboratories). The oocyte extract has a specific activity of 600 IU interferon per µg RNA injected.

d) Enriching the poly(A) RNA for HuIFN mRNA

The poly(A) RNA is passed through a Chelex-100 column (200—400 mesh, Bio-Rad) of 0.5 ml bed volume. The column is rinsed with 1 ml of 1 mM EDTA.

The eluate (1 mg poly(A) RNA in 2 ml EDTA) is heated for 2 min at 100°C and subjected to centrifugation through a sucrose density gradient (6 14 ml sucrose solutions increasing in sucrose concentration from 5% to 23% (m/v) and containing 50 mM Tris · HCl [pH 7.5], 0.2 m NaCl and 1 mM EDTA). The centrifugation is carried out in a TST 41 rotor (Kontron AG) at 35 000 rpm for 16 h at 5°C. 0.3 ml fractions are collected with an ISCO gradient collector. 2 volumes of ethanol are added to each fraction and the solution is allowed to stand for 10 h at −20°C. The precipitated mRNA is collected by centrifugation (Sorvall, HB-4 rotor at 0°C, 10 000 rpm for 10 min). The precipitate of each fraction is redissolved in 25 µl of 1 mM EDTA and each fraction is assayed for human IFN mRNA activity as described above (step Ac), except that only 10 oocytes are injected per RNA sample instead of 20. The results are given in Table 2.

TABLE 2:
HuIFN mRNA activity from fractions of sucrose-density gradient

| Fraction No. | IFN activity (units/ml) |
|---|---|
| 1—18 | — |
| 19 | 162 |
| 20 | 162 |
| 21 | 162 |
| 22 | 162 |
| 23 | not tested |
| 24 | 729 |
| 25 | not tested |
| 26 | 405 |
| 27 | not tested |
| 28 | 486 |
| 29 | not tested |
| 30 | 162 |
| 31 | not tested |
| 32 | 162 |
| 33 | not tested |
| 34 | 54 |
| 35—40 | not tested |

The fractions 23—29 are pooled and the poly(A) RNA is purified further as follows:

The poly(A) RNA solution is adjusted to 2×TNE in 0.5% SDS and applied on a 200 µl oligo(dT) cellulose column. The column is washed with 2 ml of 2×TNE in 0.5% SDS and the poly(A) RNA is eluted by 5 washes with 0.5 ml $H_2O$. The eluate is adjusted to TNE and the solution is extracted twice with an equal volume of phenol (saturated in TNE) and twice with an equal volume of chloroform. The poly(A) RNA is precipitated with 2 volumes of ethanol at −20°C for 10 hours and collected by centrifugation in a HB-4 rotor as described before.

The poly(A) RNA is dissolved in 100 µl of 0.5 mM EDTA. The yield is 40 µg as determined by measuring the optical density.

A portion of the poly(A) RNA is assayed for human IFN activity as described above by using 20 oocytes per assay. The poly(A) RNA preparation has a specific activity of 8100 IU interferon per µg RNA.

B. Preparation of double-stranded cDNA (figure 7)

Poly(A) RNA enriched for HuIFN mRNA (see step Ad) is used as a template to prepare double-stranded cDNA essentially as described by Efstratiadis et al. (36), Maniatis et al. (37) and Hoeijmakers et al. (38).

a) First strand synthesis

250 µl reaction mixture containing 40 mM Tris · HCl (pH 7.5), 30 mM NaCl, 5 mM MgCl$_2$, 0.5 mM DTT (Calbiochem.), 1 mM dGTP, dCTP, dTTP (P-L Biochemicals) and 1 mM $^{32}$P-dATP (Amersham, specific activity 50 000 cpm/nmole), 20 µg/ml oligo(dT)$_{12-18}$ (P-L Biochemicals), 40 µg/ml poly(A) RNA and 100 units of avian myeloblastosis virus (AMV) reverse transcriptase (Life Sciences, Inc., St. Petersburg, Florida) are incubated for 80 min at 37°C. The reaction is terminated by adjusting the solution to 10 mM EDTA and 0.1% SDS. The mixture is extracted once with 1 volume of phenol. The aqueous phase is reextracted with 1 volume of chloroform and applied on a 3 ml Sephadex G-50 (Pharmacia, fine) column. 0.1 ml fractions are collected. The radioactivity of each fraction is determined by measuring the Cerenkov radiation. Radioactive fractions are pooled and the nucleic acids are precipitated with 2 volumes of ethanol at −20°C for 10 h. The sample is centrifuged in a HB-4 rotor for 20 min at 10 000 rpm at 0°C. The precipitate is dissolved in 95 µl of H$_2$O. 5 µl of 10N NaOH is added and the mixture is incubated at 25°C for 40 min. After neutralization with 5M acetic acid, 50 µl H$_2$O and 2 volumes of ethanol are added and the sample is stored at −20°C for 10 hrs. The precipitate is collected by centrifugation as described before and redissolved in 200 µl of 0.1 mM EDTA. The yield of single-stranded cDNA is 3.7 µg. The size of the cDNA is 700—1500 nucleotides in length, as determined from its electrophoretic mobility in a 6% polyacrylamide gel in Tris-borate-EDTA (108 g of Tris, 9.3 g of disodium EDTA, and 55 g of boric acid per one l solution of pH 8.3) containing 7 M urea relative to marker DNAs of known length (39).

b) Second strand synthesis and S$_1$ endonuclease digestion

The obtained cDNA solution is heated at 100°C for 90 sec, chilled and incubated in a 400 µl reaction mixture comprising 0.1 M potassium phosphate buffer (pH 6.9), 10 mM MgCl$_2$, 10 mM DTT (Calbiochem), 1 mM dATP, 1 mM dCTP, 1 mM dTTP (P-L, Biochemicals), 1 mM $^3$H-dGTP (Amersham, specific activity 94 000 cpm/nmole) and 165 units/ml of E. coli DNA polymerase I (Biolabs, New England) for 8 h at 15°C. The reaction is terminated by adding EDTA and SDS to final concentrations of 10 mM and 0.1%, respectively. The mixture is extracted with phenol and chloroform, chromatographed over Sephadex G-50 (Pharmacia, fine, 2 ml bed volume) and ethanol precipitated as described above (step Ba).

The resulting DNA is treated in a 50 µl incubation mixture containing 0.25 M NaCl, 50 mM sodium acetate (pH 4.5) and 1 mM ZnSO$_4$ with 6 units of S$_1$ endonuclease (P-L Biochemicals) at 37°C for 30 min. The reaction is stopped with 0.1% SDS and 10 mM EDTA. The reaction mixture is deproteinized with 1 volume of phenol (saturated in 50 mM sodium acetate, pH 4.5) and chloroform. The aqueous phase is chromatographed on a 2 ml Sephadex G-50 (Pharmacia, fine) column in TNE. 100 µl fractions are collected and the Cerenkov radiation of each fraction is determined. The excluded fractions are pooled and the DNA is precipitated with 2 volumes of ethanol at −20°C for 10 h as described above. The precipitate is centrifuged in a HB-4 rotor (see above) and the collected precipitate is dissolved in a 100 µl solution containing 10 mM Tris · HCl (pH 7.5) and 0.5 mM EDTA. 4 µg of DNA are obtained.

The DNA is fractionated through a sucrose density gradient (5—23%) in 50 mM Tris-HCl (pH 7.5) and 1 mM EDTA in a TST-60 rotor (Kontron AG). Centrifugation is carried out at 55 000 rpm for 5 h at 15°C. The DNA, which sediments faster than a 800 base pair marker DNA, run in a parallel gradient, is pooled, adjusted to TNE and precipitated with 67% ethanol at −20°C for 10 hrs. 0.4 µg double-stranded cDNA are obtained.

C. Preparation of pBR 322—linked cDNA (figure 7)

a) Preparation of dCMP-elongated cDNA

The 3'-termini of 0.1 µg of the obtained ds cDNA are provided with poly(dC) tails in a 10 µl reaction volume containing 100 mM sodium cacodylate (pH 7.2), 2.4 mM CoCl$_2$, 50 µg BSA (Calbiochem.) per ml, 1 mM dCTP and 10 units of terminal deoxynucleotidyl transferase (P-L Biochemicals) per µg of ds cDNA. After incubation (20 min at 27°C), EDTA is added to 10 mM and the sample is stored at −20°C until use.

b) Preparation of Pst I cleaved, dGMP elongated pBR 322

10 µg of pBR 322 plasmid DNA is digested with 10 units of Pst I endonuclease (Biolabs) in a 100 µl solution containing 50 mM NaCl, 6 mM Tris · HCl (pH 7.5), 6 mM MgCl$_2$, 6 mM 2-mercaptoethanol and 100 µg/ml gelatine for 1 h at 37°C. The solution is extracted with 1 volume of phenol and chloroform. The solution is adjusted to TNE and the linearized DNA is precipitated with 2 volumes of ethanol at −20°C for 5 h.

The linearized plasmid DNA is elongated with dGMP in a 200 µl reaction volume containing 100 mM

18

sodium cacodylate (pH 7.2), 5 mM MgCl$_2$, 20 mM NaH$_2$PO$_4$, 50 µg BSA per ml, 1 mM dGTP and 100 units of terminal deoxynucleotidyl transferase (P-L Biochemicals). After incubation for 20 min at 37°C, EDTA is added to 10 mM and the reaction mixture is frozen at −20°C until use.

c) Annealing of dGMP-elongated pBR 322 to dCMP-elongated ds cDNA

A mixture of dCMP-elongated double-stranded cDNA (0.1 µg) and dGMP-tailed linearized pBR 322 (0.5 µg) in 500 µl TNE buffer is incubated at 65°C for one hour, at 46°C for one hour, at 37°C for one hour and at 20°C for one hour. The solution containing the pBR 322-linked cDNA is put on ice and used immediately for transformation.

D. Transformation of *E. coli* HB 101 with the annealed hybrid plasmid

Calcium treated *E. coli* HB 101 is prepared for transformation by the method of Mandel et al. (29).
10 µl of the reaction mixture containing the annealed pBR 322 hybrid plasmid DNAs prepared as described above (step Cc) are added to a mixture containing 150 µl calcium-treated *E. coli* HB 101 in 10 mM MgCl$_2$, 10 mM CaCl$_2$ and 10 mM Tris · HCl (pH 7.5) in a total volume of 200 µl.

The mixture is cooled in ice for 20 min, heated to 42°C for 1 min and incubated at 20°C for 10 min. 1 ml of tryptone medium (tryptone medium contains 10 g Bacto-Trypton (Difco); 1 g yeast extract (Difco); 1 g glucose, 8 g NaCl and 294 mg CaCl$_2$ · 2 H$_2$O in 1 l of distilled water) is added and the mixture is incubated for 30 min at 37°C by shaking at 300 rpm. The mixture is plated onto a 2 agar plates (Mc Conkey agar, Difco; 0.6 ml/plate) supplemented with 10 µg/ml of tetracycline (Sigma). The plates are incubated at 37°C for 12—17 hrs. About 5600 tetracycline resistant colonies of transformed *E. coli* HB 101 are prepared.

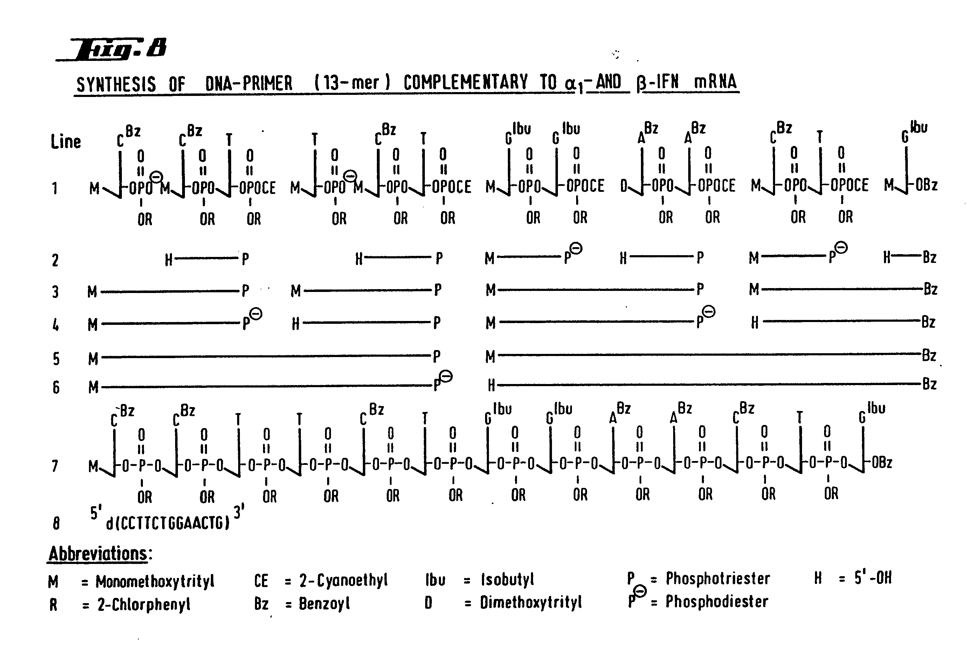

E. Identification of clones containing HuIFN cDNA
a) Synthesis of a 13-mer oligodeoxynucleotide primer (figure 8)

An oligodeoxynucleotide complementary to a stretch of 13 nucleotides which both HuIFN-α$_1$ and HuIFN-β mRNA share in common is chemically synthesized by the phosphotriester method (cf. Itakura et al. (40), de Rooij et al (41)). The individual steps of the synthesis are outlined in figure 8. The starting materials indicated in line 1 of figure 8 (mono- and dideoxynucleotides carrying protective groups) are known from the literature. The protective groups are split off by the methods described by Itakura et al.: the deblocking of 5'-monomethoxytrityl (M) or dimethoxytrityl (D) substituted hydroxyl groups is performed with acetic acid (80%) at room temperature, and the β-cyanoethyl phosphate groups are cleaved with 0.1 N sodium hydroxide in dioxane-water (4:1) at room temperature. The condensation of the building blocks is accomplished by using triisopropylbenzenesulfonyl chloride as an activating agent to afford oligodeoxynucleotides up to the fully protected 13-mer primer represented in line 7 of figure 8. The last step (complete removal of all protective groups) is achieved in the following manner:

A solution containing 64.6 mg of the fully protected 13-mer oligodeoxynucleotide in 3 ml dioxane and 1 ml acetonitrile is treated with 200 mg syn-p-nitrobenzaldoxime and 124 mg N$^1$, N$^1$, N$^3$, N$^3$ - tetramethylguanidine and allowed to stand for 27 hours. 10 ml ammonia (25%) is added and the solution is stored for 24 hours at 50°C. After the solvent has been evaporated in vacuo, the residue is dissolved in water, adjusted to pH 4 with acetic acid and the solution is extracted 20 times with chloroform. The aqueous solution is evaporated in vacuo and the residue is dissolved in 1 ml acetic acid (80%). The solution is allowed to stand for 1 hour, diluted with 6 ml water, extracted 3 times with chloroform and lyophilized. The third part of the raw product obtained is purified by chromatography on DEAE-Sephadex A 25 (column size: 10 · 1.3 cm) through a 200 ml 0.2—1.2 M triethylammonium bicarbonate gradient. Elution of the main fraction occurs at a gradient concentration of 0.87 M. The main fraction, which consists of the pure product as indicated by a HPLC test, is evaporated 3 times with water, filtered through 10 ml Dowex 50 W (NH$_4$-salt) and lyophilized. HPLC (permaphase AAX, column size 90 · 0.3 cm, 60°C, 2 ml/min; gradient: A=0.005 M KH$_2$PO$_4$, B=0.5 M KH$_2$PO$_4$, 0.5 M KCl, pH 4.5; 20% A→100% B in 30 min): t$_R$ 11.8 min.

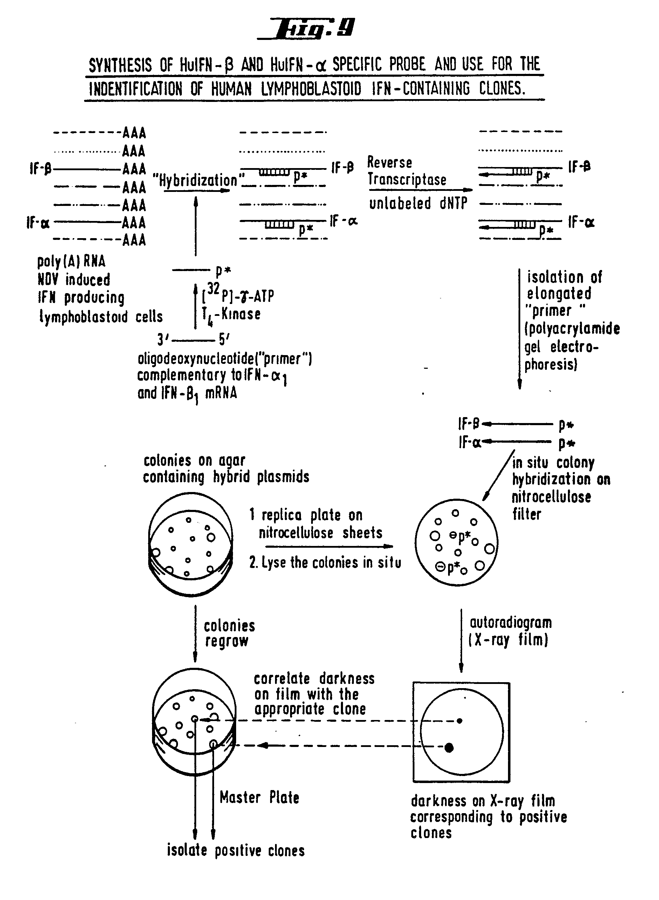

b) Preparation of a $^{32}$P-labeled human IFN-α and IFN-β specific CDNA probe (figure 9)

40 pmol of the synthetic 13-mer oligodeoxynucleotide primer (cf. step Ea) and 40 pmol of [γ-$^{32}$P]-ATP (5700 Ci · mmol$^{-1}$, Amersham) are combined in 100 µl of 50 mM Tris · HCl (pH 9.5), 10 mM MgCl$_2$ and 5 mM DTT. 50 units of T$_4$ polynucleotide kinase (P-L Biochemicals) are added and after 30 min at 37°C additional 20 units of the enzyme are added, and incubation is continued for another 15 min at 37°C. The aqueous solution containing the $^{32}$P-labeled primer is purified by phenol extraction. Further purification is accomplished by chromatography on a 4 ml Sephadex G-50 (Pharmacia, fine) column in 1 mM Tris · HCl (pH 8.0). 0.1 ml fractions are collected. The radioactivity of each fraction is determined by measuring the Cerenkov radiation. A specific activity of 4 · 10$^6$ Cerenkov cpm per mole of oligodeoxynucleotide is obtained. The $^{32}$P-labeled primer (40 pmol) is lyophilized, resuspended in 91 µl of H$_2$O containing 14 µg of poly(A) RNA (from induced Namalwa cells, prepared as described in step A) and heated for 60 sec at 100°C. 9 µl of 4 M KCl is added and the mixture is incubated at 25°C for 60 minutes. 450 µl reverse transcriptase mix is added such that the reaction volume comprises 40 mM Tris · HCl (pH 8), 4 mM MgCl$_2$, 1 mM DTT (Calbiochem, Inc.), 74 mM KCl, 1 mM each of dATP, dGTP, dCTP, dTTP (P-L Biochemicals) and 90 units of avian myeloblastosis virus (AMV) reverse transcriptase. The incubation is continued for 1 h at 37°C. The solution is extracted with 1 volume of phenol (saturated in TNE) and the nucleic acids are precipitated with 2 volumes of ethanol at −20°C for 10 h. The precipitate is collected by centrifugation (HB-4 rotor, 20 min, 10 000 rpm, 0°C) and dissolved in 20 µl dye mix containing 90% (v/v) formamide (Merck, pro analysis), 1 mM EDTA, 0.05% bromo-phenol blue and 0.05% xylene cyanol blue. The sample is heated at 90°C for 2 min and applied on a 5% polyacrylamide gel in Tris-borate-EDTA (cf. Peacock et al. (39). A single band is visible on the autoradiogram which migrates between the 267 bp and 435 bp $^{32}$P-labeled marker DNA fragments obtained from the Hae III digest of the plasmid pBR 322. The $^{32}$P-labelled cDNA fragment is extracted from the gel and purified as described by Mueller et al. (42). 20 000 Cerenkov cpm of the $^{32}$P-labeled human IFN-α and IFN-β specific cDNA probe are obtained.

c) Screening for colonies containing HuIFN cDNA (figure 9)

1650 of the transformant colonies prepared as described above (step D) are transferred to nitrocellulose filters BA 85 (Schleicher & Schuell, 8 cm diameter). The cells are lysed and their DNA is denatured and fixed to the filters in situ, according to Grunstein and Hogness (20). The filters bearing the colonies are prehybridized in 4×SET (a solution containing 0.15 M NaCl, 30 mM Tris · HCl (pH 8.0), 1 mM EDTA) 0.1% (w/v) Ficoll 400 (Pharmacia), 0.1% (w/v) polyvinylpyrrolidone (PVP-360, Sigma), 0.1% (v/v) BSA, 0.5% SDS, 50 µg/ml denatured calf-thymus DNA (prepared as follows: 5 mg calf-thymus DNA (type I, Sigma) is boiled for 10 min in 0.5 M NaOH to shear the DNA, neutralized with 5 M acetic acid and precipitated with 2 volumes of ethanol at −20°C. The precipitate is collected by centrifugation in a HB-4 rotor for 10 min at 0°C and redissolved in 50 µl 0.5 mM EDTA) at 65°C for 4h using 20 ml mixtures per filter and hybridized with $10^3$ Cerenkov cpm of the $^{32}$P-labeled probe per nitrocellulose filter in 5×SET, 0.02% (w/v) Ficoll, 0.01% polyvinylpyrrolidone, 0.02% (v/v) BSA, 0.2% SDS and 50 µg/ml denatured calf-thymus DNA. The hybridization is performed at 65°C for 36 h.

The filters are rinsed once in chloroform, twice in SET, 0.5% SDS at room temperature and twice in SET, 0.5% SDS for 1 h at 60°C and once with 3 mM Trizma base at room temperature for 1 h. The filters are dried by blotting on 3 MM-paper (Whatman), and an X-ray film (Fuji) is exposed to the filters using a screen (Ilford intensifying screen) at −80°C for 72 h.

Nine positive colonies are identified on the autoradiogram and are used for further investigation.

Since the primary clones of transformed cells occasionally contain more than one species of recombinant DNA molecules, the hybrid plasmid DNAs are isolated from the 9 positively hybridizing clones and used to retransform E. coli HB 101 as described before.

The hybrid plasmid DNA is isolated as follows: 1 colony is used to inoculate 10 ml of tryptone medium, supplemented with 10 µg/ml of tetracycline as above in a 25 ml Erlenmeyer flask. The culture is shaken for 15—18 hrs at 37°C at 300 rpm. The cells are harvested by centrifugation (Sorvall, HS-4 rotor, 10 min at 4000 rpm, 4°C). About 0.1 g of cells are obtained and are resuspended in 1 ml 50 mM Tris · HCl (pH 8.0). 0.25 ml of lysozyme solution (10 mg/ml in 50 mM Tris · HCl (pH 8.0), lysozyme is purchased from Sigma), are added and after incubation at 0°C for 10 min, 0.15 ml of 0.5 M EDTA (pH 7.5) is added. After another 10 min at 0°C, 60 µl of 2% Triton X-100 (Merck) is added. After 30 min at 0°C, the sample is centrifuged for 30 min at 15 000 rpm and 4°C in a Sorvall SA-600 rotor. The supernatant is deproteinized with 1 volume of phenol (saturated in TNE). The phases are separated by centrifugation (Sorvall HB-4 rotor) for 10 min at 5000 rpm at 4°C. The upper phase is extracted twice with 1 volume of chloroform. Pancreatic RNAse A (Sigma; 10 mg/ml in TNE, preheated 10 min at 85°C) is added to a final concentration of 25 µg/ml and the mixture is incubated for 40 min at 37°C. The solution is then adjusted to 1 M NaCl and 10% polyethylene glycol 6000 (Fluka, autoclaved for 20 min at 120°C) and incubated at −10°C for 2 hrs. The precipitate is collected in a Sorvall HB-4 rotor (20 min at 10 000 rpm, 0°C) and redissolved in 100 µl of TNE. The DNA solution is extracted with 1 volume of phenol and the DNA is precipitated with 2 volumes of ethanol at −80°C for 10 min.

The precipitate is collected by centrifugation in an Eppendorf centrifuge and the DNA is redissolved in 20 µl of 10 mM Tris · HCl (pH 7.5) and 0.5 mM EDTA. 8—10 µg of hybrid plasmid DNA are recovered from a 10 ml culture.

E. coli HB 101 is transformed with each of the nine isolated hybrid DNAs and the transformed cells are plated on agar plates containing tetracycline, as described before (step D). From each transformation, 3 tetracycline resistant clones are picked, 10 ml cultures are prepared and the hybrid DNAs are isolated from the cultures as described before.

All the DNA samples before and after retransformation are analyzed by cleavage with Pst I endonuclease and electrophoresis through a 1% agarose gel in 50 mM Tris-acetate (pH 7.8) and 1 mM EDTA. All the samples display identical cleavage patterns before and after retransformation.

One of the recloned recombinant DNA molecules gives 2 bands, one with the mobility of Pst I-cleaved pBR 322, the other with a mobility corresponding to about 1000 bp. It is denoted CG-pBR 322/HLycIFN-1′b.

Another recombinant DNA gives 3 bands, one with the mobility of Pst I-cleaved pBR 322, one with a mobility of about 600 bp and one with a mobility of about 150 bp. The recombinant DNA molecule in this clone is designated CG-pBR 322/HLycIFN-β$_1$.

d. Characterization of the clones CG-pBR 322/HLycIFN-1′b and CGp-BR 322/HLycIFN-β$_1$

The recombinant plasmid DNAs of the clones CG-pBR 322/HLycIFN-1′b and CG-pBR 322/HLycIFN-β$_1$ are isolated from the cultures as described above (step Ec) and characterized by establishing the nucleotide sequence of the cDNA insert using the method described by Maxam and Gilbert (15). Basically, the following approach is used:

The isolated recombinant plasmid DNA is digested with various restriction endonucleotides. The enzymes are applied essentially as described by the supplier (New England Biolabs), except that BSA is replaced by gelatin in the enzyme buffers. The solution containing the restricted DNA is deproteinized with phenol (saturated with TNE). The DNA is precipitated with ethanol, redissolved in 50 mM Tris-HCl (pH 8.0) at a DNA concentration of 50 µg/ml and incubated with 0.1 units of calf intestinal alkaline phosphatase (Boehringer) per pmole DNA 5′ ends for 30 min at 37°C. The enzyme is inactivated by heating the solution for 60 min at 65°C. The DNA is purified by DEAE-cellulose chromatography as described by Mueller et al. (42) and precipitated with ethanol. The DNA is then 5′-terminally labeled with $[\gamma\text{-}^{32}P]$-ATP ($>5000$ ci/mmole, Amersham) and T4 polynucleotide Kinase (P-L Biochemicals) essentially as described by Maxam and Gilbert (15) except that the DNA is not denatured before the Kinase reaction. In general, the specific activities amount to $1\text{—}3 \cdot 10^6$ cpm/pmole 5′-ends.

The labeled DNA fragments are cleaved with a second restriction endonuclease and the products are separated by electrophoresis through a 6%, 8% or 10% polyacrylamide gel in Tris-borate-EDTA buffer. The DNA fragments are extracted from the gel and purified as described by Mueller et al. (42). For the determination of the nucleotide sequences, the DNA fragments are chemically degraded and the products are separated by polyacrylamide gel electrophoresis as described by Maxam and Gilbert (15).

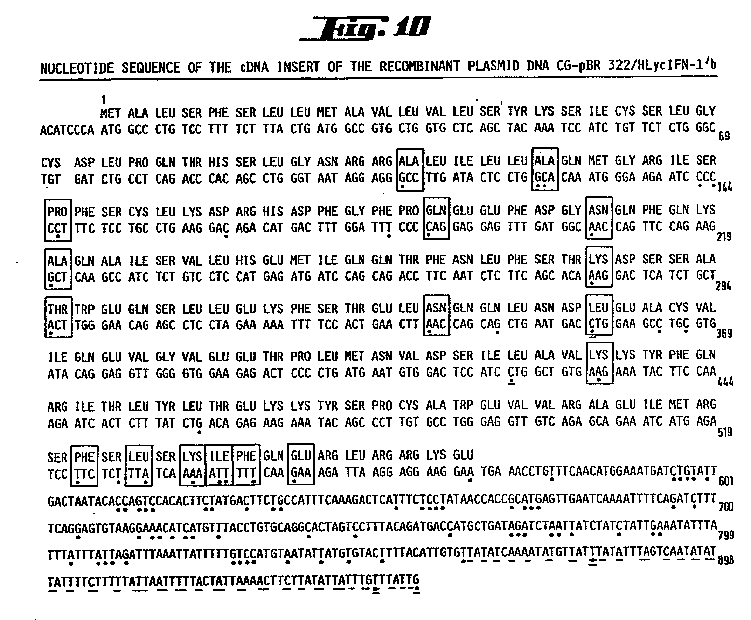

In particular, the isolated plasmid DNAs of the clone CG-pBR 322/HLyclFN-1′b are treated as follows. On the one hand, 5 µg of the plasmid DNA is digested with Bgl II, 5′ terminally labeled, and cleaved with Pvu II. The Pvu II-Bgl II* (*indicates the labeled site) and Bgl II-Pvu II* DNA fragments are isolated on a 6% polyacrylamide gel. On the other hand, 5 µg of the plasmid is digested with Alu I, 5′-terminally labeled, and cleaved with Pst I. The Pst I—Alu I* DNA fragment is isolated on a 8% polyacrylamide gel. The individual fragments are subsequently degraded and sequenced according to Maxam and Gilbert. The nucleotide sequence obtained is depicted in figure 10. A stretch of about 25—35 deoxyguanosine residues is preceding at the 5′-end of the cDNA insert. The nucleotide sequence shown is somewhat similar to that of IFN-α (type F) cDNA described by Goeddel et al. [(43), cf. also Weissmann (44)], nevertheless displaying a lot of distinct deviations (point mutations) some of which are affecting the resulting amino acids (cf. fig. 10).

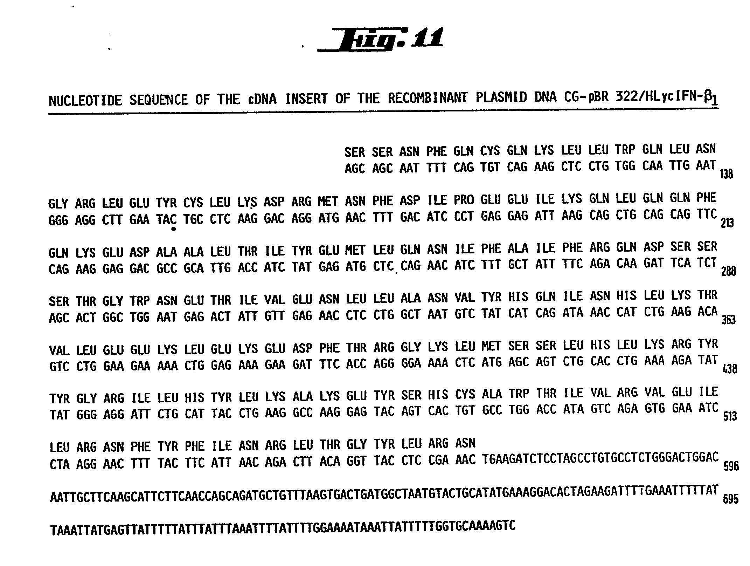

The isolated plasmid DNA of the clone CG-pBR 322/HLyclFN-$\beta_1$ is treated in a similar manner. 5 µg of the plasmid is digested with Pvu II and 5′-terminally labeled. One half of the mixture is cleaved with Pst I, and the rest with Bgl II. The Pst I-Pvu II* and Bgl II-Pvu II* fragments are isolated by electrophoresis on a 6% polyacrylamide gel and degraded as mentioned above. The nucleotide sequence (N-terminal sequence) is depicted in figure 11 and reveals that the cDNA insert starts at nucleotide number 102 of the IFN-$\beta_1$ cDNA as described by Taniguchi et al. (45). Therefore, the cDNA insert has the capacity to code for human IFN-$\beta_1$ lacking 11 amino acids at the N-terminus. The cDNA insert is flanked at its 5′ end by a stretch of about 20—25 deoxyguanosine residues and shows a point mutation at position 153, converting a C to a T residue without affecting the resulting amino acid.

e. Identification of clones containing recombinant DNA molecules cross-hybridizing to the inserts of CG-pBr 322/HLyclFN-1′b and CG-pBR 322/HLyclFN-$\beta_1$

The recombinant plasmid DNAs of the clones CG-pBR 322/HLyclFN-1′b and CG-pBR 322/HLyclFN-$\beta_1$ are isolated from the cultures as described above (step Ec). The CG-pBR 322/HLyclFN-1′b plasmid DNA (5 µg) is digested with Bgl II, 5′ terminally labeled, and cleaved with Pvu II. On the other hand, the isolated CG-pBR 322/HLyclFN-$\beta_1$ plasmid DNA (5 µg) is digested with Pvu II, 5′-terminally labeled, and cleaved with Bgl II. The Pvu II-Bgl II* (351 bp) DNA fragment (probe A) and the Pvu II*-Bgl II (368 bp) DNA fragment (probe B) are isolated from a 8% polyacrylamide gel as described above (step Ed) and used for *in situ* colony hybridization (see below). The restriction of the plasmid DNAs, the labeling, and the purification of the DNA fragments are accomplished in the same manner as described above (step Ed).

4000 of the transformant colonies prepared as described above (step D) are transferred to nitrocellulose filters BA 85 (Schleicher & Schuell, 8 cm diameter). The cells are lysed and their DNA is denatured and fixed to the filters *in situ*, according to Grunstein and Hogness (20). Hybridizations to the probes A and B (both probes are mixed) are performed as described before (step Ec). 6 positive colonies are identified by autoradiography, 3 of which, designated

*E. coli* HB 101 CG-pBR 322/HLyclFN-$4_1$,

*E. coli* HB 101 CG-pBR 322/HLyclFN-$5_1$ and

*E. coli* HB 101 CG-pBR 322/HLyclFN-$8′_1$

are used for further investigation. The plasmid DNAs of these clones are isolated, retransformed, and re-isolated as described above (step Ec, Ed).

In order to establish the nature of the inserts of the recombinant DNAs, the nucleotide sequences of the cDNA inserts (partial or complete) are established by using the general approach as described above (step Ed).

In particular, 5 µg of the isolated plasmid DNAs CG-pBR 322/HLyclFN-$4_1$ and CG-pBR 322/HLyclFN-$8′_1$ are each digested with Pvu II, 5′-terminally labeled and cleaved with Pst I. The DNA fragments are fractionated on a 8% polyacrylamide gel and the Pst I-Pvu II* (~120 bp) from $8′_1$ DNA and Pst I-Pvu II* (82 bp) from $4_1$ DNA are isolated as usual.

21

The isolated plasmid DNA CG-pBR 322/HLycIFN-$5_1$ is treated as follows. One the one hand, 5 µg of the plasmid DNA is digested with Hae III, 5'-terminally labeled and cleaved with Pst I. The Pst I-Hae III* (57 bp) DNA fragment is isolated on a 10% polyacrylamide gel. On the other hand, 5 µg of the plasmid is digested with EcoR I, 5'-terminally labeled and cleaved with Pst I. The Pst I-EcoR I* (235 bp) and EcoR I*-Pst I (~700 bp) DNA fragments are isolated on a 8% polyacrylamide gel. The various DNA fragments are subjected to sequence analysis according to Maxam and Gilbert (15).

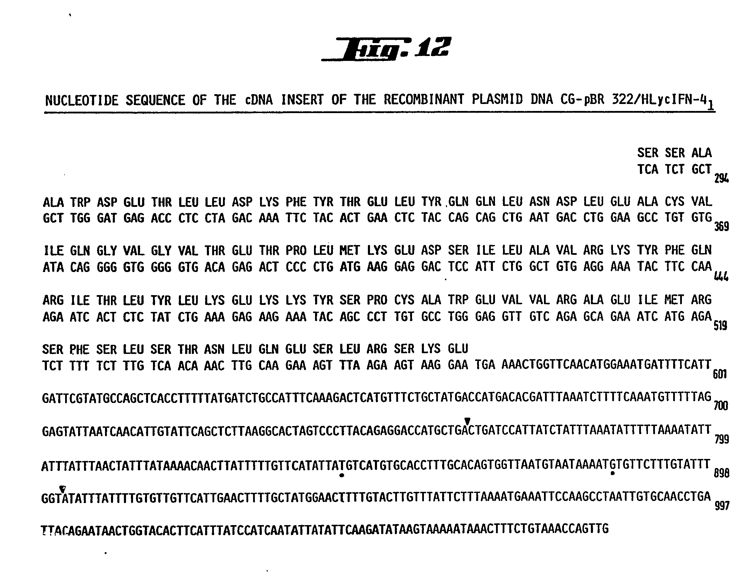

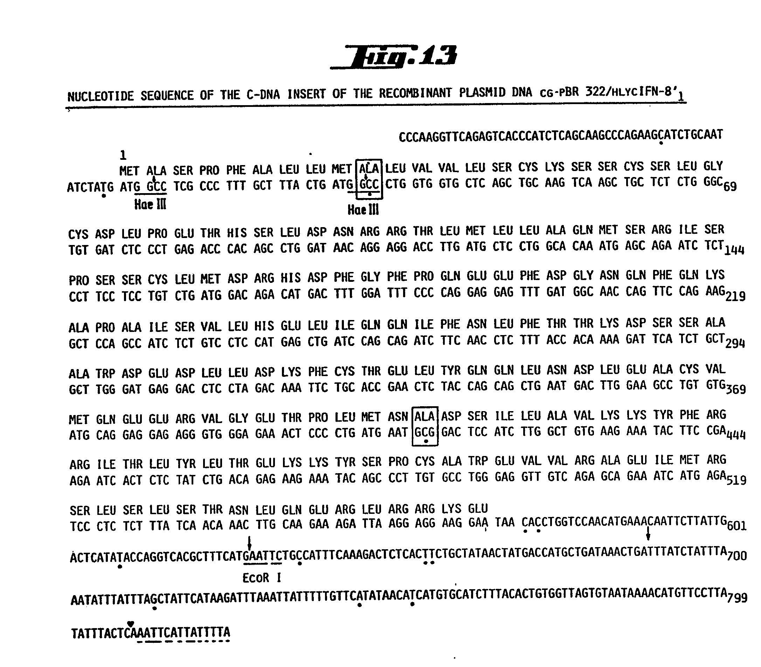

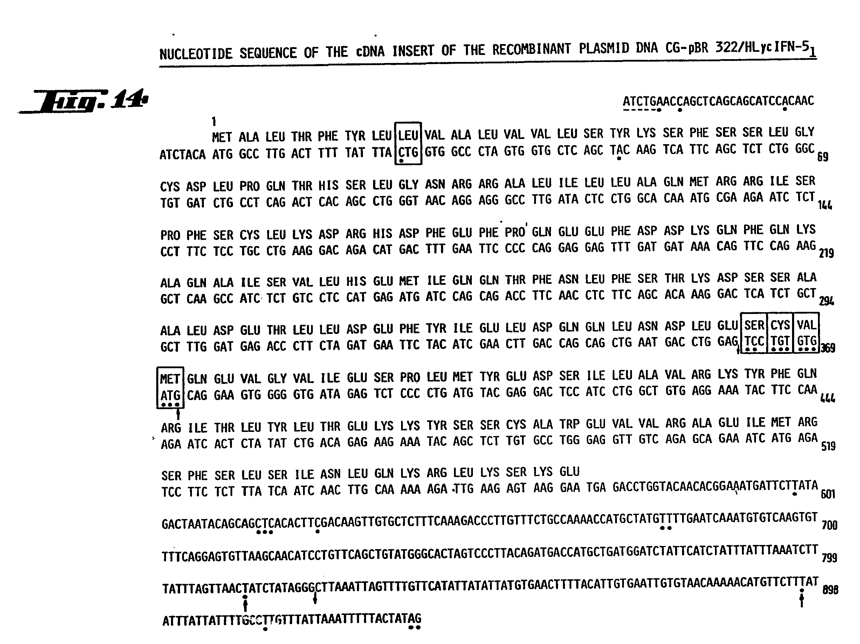

The nucleotide sequences of the cDNA inserts are depicted in figures 12—14. In figure 12, a partial nucleotide sequence of the cDNA insert of CG-pBR 322/HLycIFN-$4_1$ is shown. The insert is flanked at the 5' end by a stretch of 23 deoxyguanosine residues and comprises part of the IFN-$\alpha_2$ (Le) cDNA described by Streuli et al. (46). In the 3'-extracistronic region, there are some minor deviations (point mutations) and a stretch of additional 318 nucleotides. The nucleotide sequence of the cDNA insert of CG-pBR 322/HLycIFN-$8'_1$ is depicted in figure 13. The insert is flanked at the 5' end by a stretch of 20—23 deoxyguanosine residues and is similar but not identical, to the IFN-$\alpha$ (type D) cDNA described by Goeddel et al. [(43); cf. also Mantei et al. (27)]. Apart from differences in the cDNA regions preceding and following the IFN coding sequence, the IFN gene contains at positions 28—30 a GCC triplet and at positions 409—411 a GCG triplet coding for alanine instead of GTC and GTG, respectively, coding for valine. Finally, the nucleotide sequence of the cDNA insert of CG-pBR 322/HLyc IFN-$5_1$ (see figure 14) reveals a stretch of 17 deoxyguanosine residues at the 5' end. The nucleotide sequence is related to that of IFN-$\alpha$ (type B) cDNA described by Goeddel et al. (43). However, there are additional nucleotides at the 5' end of the cDNA insert of HLycIFN-5, point mutations, excisions and insertions in the extracistronic region and in the IFN coding sequence, especially at positions 22 and 361—372, are evident as well.

## F. Synthesis of human interferons by *E. coli* containing human IFN-specific recombinant DNA molecules

The 5 clones which have been shown to contain human IFN specific recombinant DNA molecules, namely

*E. coli* HB 101 CG-pBR 322/HLycIFN-1'b,
*E. coli* HB 101 CG-pBR 322/HLycIFN-$4_1$,
*E. coli* HB 101 CG-pBR 322/HLycIFN-$5_1$,
*E. coli* HB 101 CG-pBR 322/HLycIFN-$8'_1$, and
*E. coli* HB 101 CG-pBR 322/HLycIFN-$\beta_1$,

are tested for IFN activity, which, in each case, is accomplished in the following manner:

Cultures of the corresponding *E. coli* clone (30 ml suspensions) are grown in tryptone medium to an optical density ($OD_{650}$ of about 1. The cells are harvested and resuspended in 0.5 ml of an aqueous solution containing 30 mM NaCl and 50 mM Tris-HCl (pH 8.0). Lysozyme (Sigma) is added to 1 mg/ml. After 30 min at 0°C, the suspensions are frozen (liquid nitrogen) and thawed (at 37°C) 5 times, and centrifuged for 20 min at 20 000 rpm in a SS 34 Sorval rotor at 4°C. The supernatants are assayed for IFN activity using the cythopathic bioassay according to Armstrong (32) as described in step Ac. The following activities are found:

| Source of extract<br>*E. coli* HB 101 containing recombinant DNA | IFN activity<br>(IU/ml) |
| --- | --- |
| CG-pBR 322/HLycIFN-1'b | 0;0 |
| CG-pBR 322/HLycIFN-$4_1$ | 0;0 |
| CG-pBR 322/HLycIFN-$5_1$ | 10 000;10 000 |
| CG-pBR 322/HLycIFN-$8'_1$ | 100;100 |
| CG-pBR 322/HLycIFN-$\beta_1$ | 0;0 |

Possibly, clones exhibiting no measurable IFN activities contain recombinant DNAs in which the HuLyIFN-cDNA insert is in an improper orientation in regard to the direction of transcription. Therefore, the recombinant DNA of one such clone (CG-PBR 322/HLycIFN-1'b) containing a full length cDNA insert is reoriented as follows:

The plasmid DNA of the clone *E. coli* HB 101 CG-pBR 322/HLycIFN-1'b is isolated as described above (step Ec) and cleaved with Pst I. 0.5 µg of the cleaved DNA in 20 µl of a buffer mixture, containing 20 mM Tris-HCl (pH 7.8), 10 mM MgCl$_2$, 10 mM DTT, 25 mM NaCl and 50 µg/ml gelatin, is treated with 0.2 units of T4 DNA ligase (Biolabs) and 0.5 mM ATP for 2 h at 15°C. *E. coli* HB 101 is transformed with the cDNA mixture as described above (step D). Transformed colonies are selected on McConkey agar plates supplemented with tetracycline and subsequently, replica-plated to nitrocellulose filters. 4 bacterial colonies hybridizing to the $^{32}$P-labeled Pvu II-Bgl II* fragment (351 bp) of the recombinant DNA CG-pBR 322/HLycIFN-1'b (cf. step Ee) are designated *E. coli* HB 101 CG-pBR 322/HLycIFN-1'b$_1$ to -1'b$_4$. Extracts of the 4 clones are prepared and tested for IFN activity as described above. The following activities are found:

| Source of extract<br>E. coli HB 101 containing recombinant DNA | IFN activity<br>(IU/ml) |
|---|---|
| CG-pBR 322/HLycIFN-1′b$_1$ | 0;0 |
| CG-pBR 322/HLycIFN-1′b$_2$ | 0;0 |
| CG-pBR 322/HLycIFN-1′b$_3$ | 0;0 |
| CG-pBR 322/HLycIFN-1′b$_4$ | 30;30 |

Hence, the plasmid CG-pBR 322/HLycIFN-1′b$_4$ contains a cDNA insert capable of directing the synthesis of a polypeptide with IFN activity.

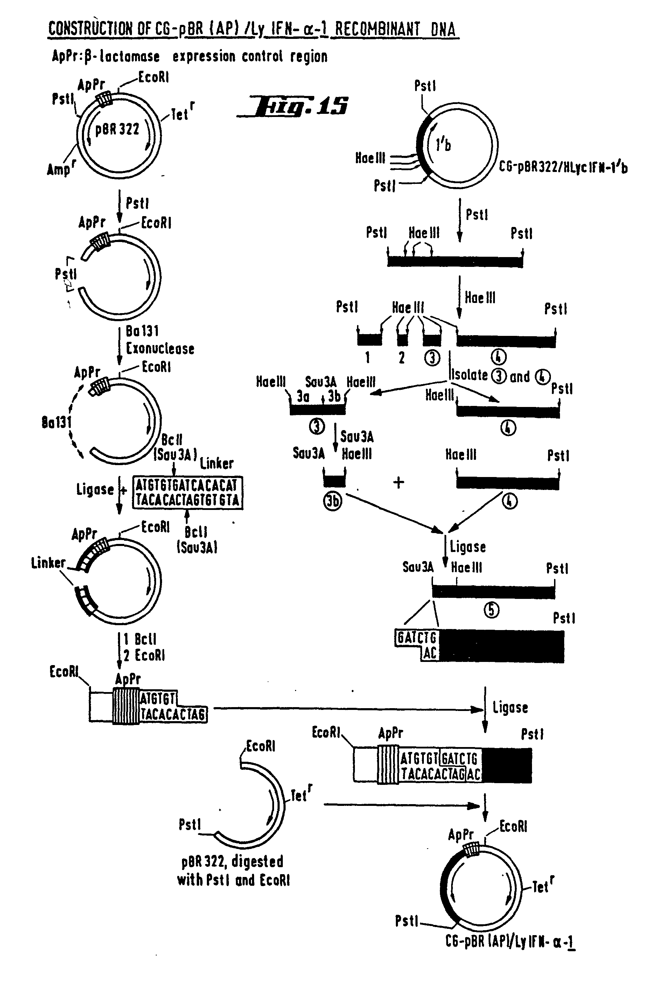

G. Construction of recombinant plasmids capable of producing high levels of polypeptides with IFN activity
I. Construction of CG-pBR (AP)/LyIFN-α-1 recombinant plasmid
In order to improve the IFN specific protein yield of the clone E. coli HB 101 CG-pBR 322/HLycIFN-1′b, the following construction is performed as indicated schematically in figure 15.

a. Preparation of the cDNA insert
The recombinant plasmid DNA (150 μg) of the clone E. coli HB 101 CG-pBR 322/HLycIFN-1′b is cleaved with Pst I (Biolabs) using standard procedures (cf. step Ed). Following phenol extraction and ethanol precipitation, the excised insert is isolated by means of sucrose density gradient centrifugation (5—23%) in 50 mM Tris-HCl (pH 8.0) and 1 mM EDTA. The centrifugation is performed as 35 000 rpm in a TST 41 rotor (Kontron AG) at 15°C for 16 h. 0.3 ml fractions are collected with an ISCO gradient collector at 1 ml/min. The fractions containing the small fragment (i.e. the insert) are pooled. The DNA is precipitated with ethanol as usual, and the precipitate is collected by centrifugation in a HB-4 rotor (Sorvall) at 10 000 rpm at 0°C for 10 min. The precipitate is redissolved in 60 μl 10 mM Tris-HCl (pH 7.5) and 0.05 mM EDTA. 30 μg DNA are recovered as determined by measuring the optical density.
The insert DNA (10 μg) is digested with Hae III (Biolabs) and the fragments are fractionated on a 2% agarose gel in a solution containing 50 mM Tris, 50 mM boric acid, 1 mM EDTA and 0.5 μg/ml ethidium bromide. The largest DNA fragments, Hae III-Pst I (869 bp) and Hae III-Hae III (82 bp, cf. figure 15, fragments 3 and 4 respectively), are each excised from the gel, squirted through a thin needle with a syringe into 5 ml of 0.15 M NaCl, 50 mM Tris · HCl (pH 8.0), 1 mM EDTA, and eluted overnight by shaking. The eluate is passed through a 100 μl DE-52 (Whatman) Pasteur-pipette column to adsorb the DNA. The column is washed with 2 ml of the same buffer and the DNA is eluted with 400 μl of a solution containing 1.5 M NaCl, 50 mM Tris (pH 8.0) and 1 mM EDTA. The DNA is precipitated with 2 volumes of ethanol at −20°C overnight. The precipitate is collected by centrifugation in an Eppendorf centrifuge.
The Hae III-Hae III DNA fragment (82 bp) is redissolved and digested with Sau 3A (Biolabs). The enzyme is heat-inactivated at 65°C for 30 min. 1 μg of the Hae III-Pst I DNA fragment (869 bp) is added, the solution is adjusted to 10 mM MgCl$_2$, 10 mM DTT and 0.5 mM ATP, and T4 DNA ligase (Biolabs) is added to 30 units/μl reaction volume. The solution is incubated for 10 h at 15°C. Following extraction with phenol and chloroform, the mixture is fractionated on a 2% agarose gel in Tris-borate-EDTA in the presence of ethidium bromide. The Sau 3A-Pst I DNA fragment (cf. figure 15, fragment 5) is extracted as described before, precipitated with ethanol and redissolved in 10 μl of a solution containing 10 mM Tris · HCl (pH 7.5) and 0.05 mM EDTA.

b. Preparation of the DNA fragment containing the β-lactamase regulatory region (ApPr) of pBR 322
The plasmid pBR 322 is cleaved with Pst I (cf. step Cb) and treated with 4 units/ml of the exonuclease Bal 31 (Bethesda Research Lab.) at 30°C for 4—10 min to remove the β-lactamase coding segment.
A chemical DNA linker of the formula

$$5′\text{-ATGTGTGATCACACAT-}3′$$

is synthesized using the method described above (step Ea). The linker is added to the Bal 31 treated pBR 322 DNA using conventional means. The resulting hybrid molecule is cleaved with the restriction endonucleases Bcl I (Biolabs) and EcoR I. The digestion products are fractionated on a 8% polyacrylamide gel in Tris-borate-EDTA as described before (step Ba). DNA fragments (ApPr DNA fragments), migrating between 184 bp and 234 bp marker DNAs, are isolated as described above (step Ia), and precipitated with ethanol as usual. The precipitate is redissolved in a solution containing 10 mM Tris · HCl (pH 7.5) and 0.05 mM EDTA.

c. Ligation of the ApPr DNA fragment to the cDNA insert
The solutions containing the ApPr DNA fragments and the cDNA insert are pooled. The mixture is adjusted to 10 mM MgCl$_2$, 10 mM DTT and 0.5 mM ATP, and incubated with 30 units/μl T4 DNA ligase (Biolabs) at 15°C for 12 h. Following extraction with phenol and chloroform, the mixture is fractionated on a 1% low melting agarose gel (Biorad). The obtained ApPr-cDNA fragment is joined to the large fragment of pBR 322 cleaved with both Pst I (Biolabs) and EcoR I (Biolabs) in the following manner. The gel piece, containing the ApPr cDNA fragment (about 20 µl) is mixed with the Pst I-EcoR I fragment of pBR 322, melted at 65°C for 2 min, cooled to 37°C, adjusted to 0.5 mM ATP, 10 mM DTT and 10 mM MgCl₂, and incubated with 30 units/µl of T4 DNA ligase (Biolabs) for 12 h at 15°C.

One tenth volume of a solution containing 100 mM Tris · HCl (pH 7.5), 100 mM CaCl₂ and 100 mM MgCl₂, is added, the solution is heated for 10 min at 65°C to inactivate the ligase and cooled to 37°C. The solution is then taken to transform Ca²⁺-treated E. coli HB 101 as described above (step D) and plated onto McConkey agar plates supplemented with 10 µg/ml tetracycline. The transformed colonies are screened for IFN activity (cf. step F). The clone synthesizing the highest level of IFN activity is selected and designated E. coli HB 101 CG-pBR(AP)/LyIFN-α-1. An activity of 40 000 (IU/ml) is found which represents a 1300 fold stimulation compared to the original clone E. coli HB 101 CG-pBR322/HLycIFN-1'b.

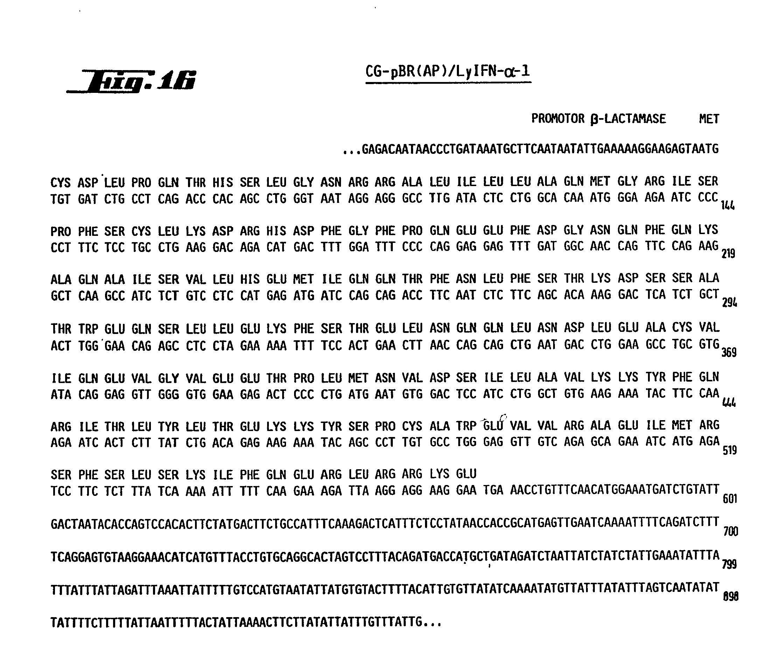

The recombinant plasmid DNA of the clone CG-pBR (AP)/LyIFN-α-1 is isolated from the culture as, described above (step 3c) and characterized by establishing the nucleotide sequence of the cDNA insert (IFN gene) and the β-lactamase regulatory region. The result is summarized in figure 16.

II. Construction of the recombinant plasmid CG-pBR (AP)/LyIFN-α-3

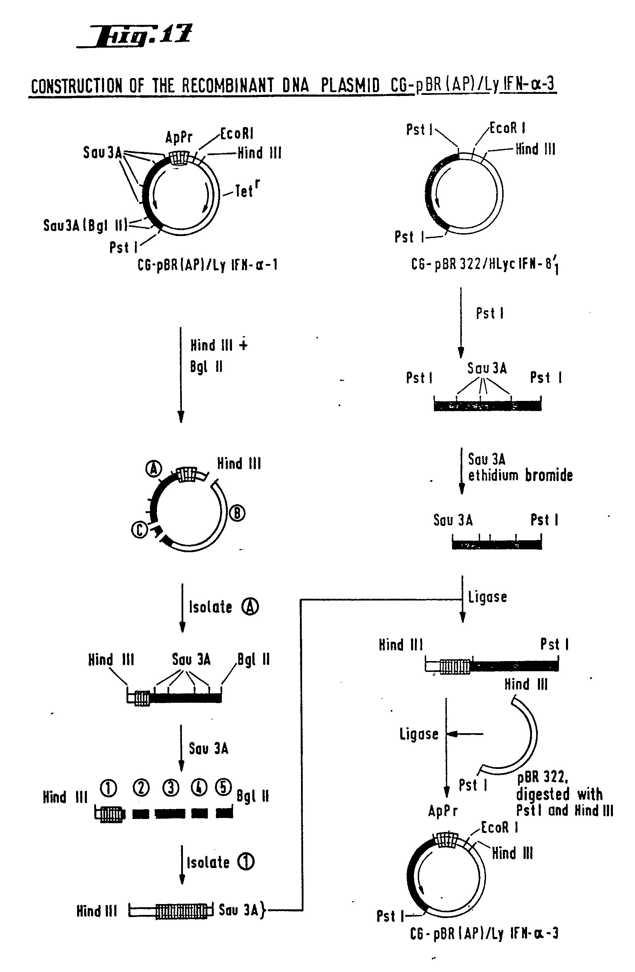

The IFN specific protein yields of the clone E. coli HB 101 CG-pBR 322/HLycIFN-8'₁ is improved as follows (cf. fig. 17):

a. Preparation of the DNA fragment containing the β-lactamase regulatory region from CG-pBR (AP)/LyIFN-α-1

CG-pBR (AP)/LyIFN-α-1 DNA (100 µg) is cleaved with Hind III (Biolabs) and Bgl II (Biolabs). Following phenol extraction and ethanol precipitation, the excised DNA fragment is isolated by means of sucrose density gradient centrifugation (5—23%) in 50 mM Tris · HCl (pH 8.0) and 1 mM EDTA. The centrifugation is performed at 58 000 rpm in a TST60 rotor (Kontron AG) at 15°C for 4 hours. 0.2 ml fractions are collected as described before. The fractions containing the small fragment (Hind III-Bgl III) are pooled and the DNA is precipitated with ethanol as usual. The precipitate is redissolved in 80 µl 10 mM Tris · HCl (pH 7.5) and 0.05 mM EDTA. 16 µg DNA are recovered as determined by measuring the optical density.

The DNA fragment (Hind III-Bgl II) (4 µg) is cleaved with Sau 3A (Biolabs) and the digestion products are fractionated on a 6% polyacrylamide gel in Tris-borate-EDTA as described before. The DNA fragments are stained in EtBr (0.5 µg/ml), the Hind III-Sau 3A DNA fragment (239 bp) is extracted and isolated as before. The DNA is precipitated with ethanol as usual. The precipitate is redissolved in 20 µl 10 mM Tris · HCl (pH 7.5) and 0.05 mM EDTA.

b. Preparation of the cDNA insert

The cDNA insert is excised from the recombinant plasmid CG-pBR 322/HLycIFN-8'₁ as described above (section Ia).

The cDNA insert (2 µg) is digested with 2.5 uints of Sau 3A (Biolabs) in 10 µg/ml EtBr and incubated at 37°C for 60 min. The digests are phenol extracted and the DNA is precipitated in ethanol as above. The DNA fragments are fractionated on a 1.2% agarose gel in a solution containing 50 mM Tris, 50 mM boric acid, 1mM EDTA and 0.5 µg/ml ethidium bromide.

The second largest DNA fragment (Sau 3A-PstI: 693 bp) is extracted from the gel and purified as described in section Ia. The DNA is redissolved in 20 µl 10 mM Tris-HCl (pH 7.5) and 0.05 mM EDTA.

c. Ligation of the Hind III-Sau 3A DNA fragment to the cDNA insert (Sau 3A-PstI)

Equal amounts of both DNA fragments (~50 ng) are incubated in a solution containing 10 mM MgCl₂, 10 mM DTT, 0.5 mM ATP and 30 units/µl T4 DNA ligase (Biolabs) at 15°C for 3 hours. The mixture is· incubated for 15 min. at 80°C and adjusted to 50 mM NaCl. The DNA mixture is digested with 0.5 units PstI (Biolabs) and 1 unit Hind III (Biolabs) for 20 min. at 37°C. The DNA is phenol extracted, ethanol precipitated and redissolved in 20 µl 10 mM Tris-HCl (pH 7.5) and 0.05 mM EDTA.

One half of the resulting mixture is ligated to the large Hind III-PstI DNA fragment of the plasmid pBR 322 (~100 ng) in 10 mM MgCl₂, 10 mM DTT, 0.5 mM ATP containing 30 units/µl of T4 DNA ligase (Biolabs) for 2h at 15°C.

One tenth volume of the solution is used to transform E. coli HB 101 as described in step D). The transformed colonies are used to test for IFN activity as described earlier (cf. step F).

The clone synthesizing the highest level of IFN activity is selected and designated E. coli HB 101 CG-pBR (AP)/LyIFN-α-3.

The IFN activity is determined as described above (step F). An activity of 70 000 (IU/ml) is found which represents a 700 fold stimulation compared to the original clone E. coli HB 101 CG-pBR 322/HlycIFN-8'₁.

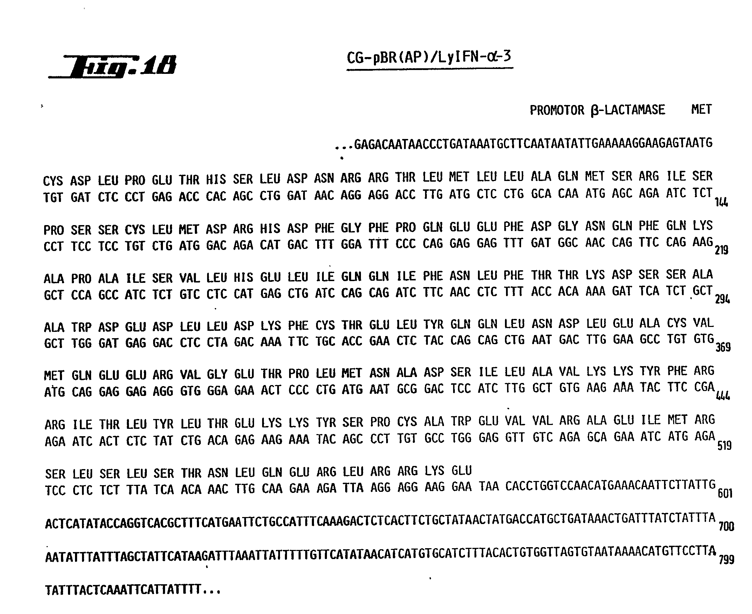

The recombinant plasmid DNA of the clone CG-pBR (AP)/LyIFN-α-3 is isolated from the culture as described above (step Cc) and characterized by establishing the nucleotide sequence of the cDNA insert (IFN gene) and the β-lactamase regulatory region. The result is summarized in figure 18.

The construction protocol for the plasmid CG-pBR (AP)/LyIFN-α-3 can be used for all α-IFN cDNA genes or appropriately cut chromosomal α-IFN genes in general.

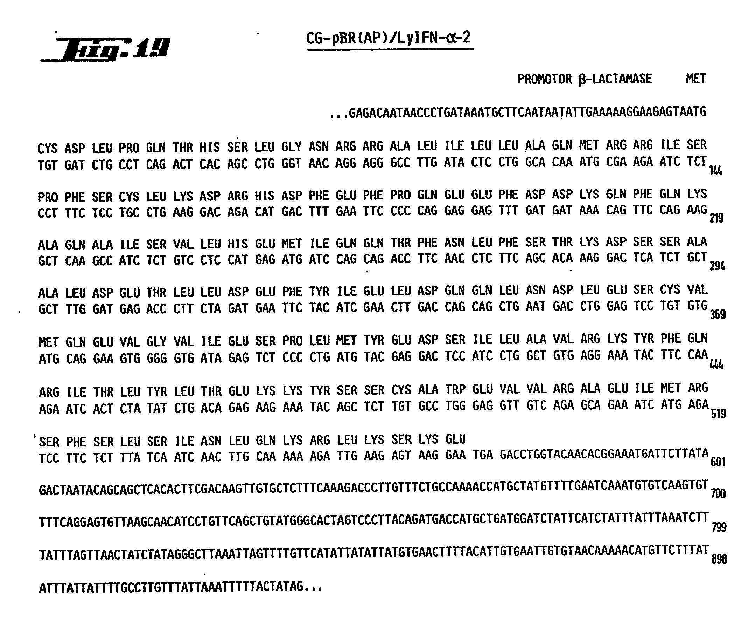

For example, starting from the plasmid CG-pBR 322/HLycIFN-5₁, the plasmid CG-pBR (AP)/LyIFN-α-2 is obtained in an identical manner as described for the plasmid CG-pBR(AP)/LyIFN-α-3. This new plasmid contains the DNA insert of CG-pBR 322/HLycIFN-$5_1$ and the β-lactamase regulatory region from CG-pBR (AP)/LyIFN-α-1. A clone designated *E. coli* HB 101 CG-pBR (AP)/LyIFN-α-2 is selected as described above. An IFN activity of 50 000 (IU/ml) is found which represents a 5 fold stimulation compared to the original *E. coli* HB 101 CG-pBR 322/HLycIFN-$5_1$. The nucleotide sequence of the cDNA insert and the β-lactamase regulatory region of the plasmid CG-pBR (AP)/LyIFN-α-2 is established as described above and depicted in fig. 19.

III. Deposition of prepared microorganisms

Micro-organisms and recombinant DNA molecules prepared as described in Example 10 are exemplified by cultures deposited in the culture collection of the Agricultural Research Culture Collection (NRRL) on September 14, 1981 and are assigned the following accession numbers:

*E. coli* HB. 101 CG-pBR 322/HLycIFN-$β_1$: NRRL B-12528
*E. coli* HB 101 CG-pBR 322/HLycIFN-$4_1$: NRRL B-12529
*E. coli* HB 101 CG-pBR 322/HLycIFN-1'b: NRRL B-12530
*E. coli* HB 101 CG-pBR 322/HLycIFN-$5_1$: NRRL B-12531
*E. coli* HB 101 CG-pBR 322/HLycIFN-$8'_1$: NRRL B-12532

Example 11:

The lymphoblastoid IFN-1'b and IFN-$5_1$ coding sequences of the *E. coli* plasmids CG-pBR322/HLycIFN-1'b and -$5_1$ (cf. Example 10) can be subcloned in plasmid p30 (cf. Example 4) in an analogous manner as described for IFN-$8'_1$ in Examples 5 and 6. A partial digestion with restriction endonucelase HaeIII is required. The yeast hybrid plasmids obtained in this way are p30IFN2(1'b), p30IFN2'(1'b), p30IFN2($5_1$) and p30IFN2'($5_1$).

These obtained hybrid plasmids can be used to transform *Saccharomyces cerevisiae* RH971 as described in Example 7. The following colonies containing a hybrid plasmid with a lymophoblastoid IFN cDNA insert can be selected:

*S. cerevisiae* RH971/p30IFN2(1'b)
*S. cerevisiae* RH971/p30IFN2'(1'b)
*S. cerevisiae* RH971/p30IFN2($5_1$)
*S. cerevisiae* RH971/p30IFN2'($5_1$)

Example 12:

In an analogous manner as described in Example 9, the following yeast hybrid plasmids can be obtained starting from the plasmids p30IFN2(1'b), -($5_1$), -($8'_1$) and p30IFN2'(1'b), -($5_1$), respectively:

pJDB207/IFN2(1'b), pJDB207/IFN2'(1'b), pJDB207/IFN2($5_1$), pJDB207/IFN2'($5_1$) and pJDB207/IFN2($8'_1$).

These hybrid plasmids can be transformed into *S. cerevisiae* strain RH971 selecting for leucine prototrophic colonies. The following colonies containing a hybrid plasmid with a lymphoblastoid IFN cDNA insert can be obtained:

*S. cerevisiae* RH971/pJDB207/IFN2(1'b)
*S. cerevisiae* RH971/pJDB207/IFN2'(1'b)
*S. cerevisiae* RH971/pJDB207/IFN2($5_1$)
*S. cerevisiae* RH971/pJDB207/IFN2'($5_1$)
*S. cerevisiae* RH971/pJDB207/IFN2($8'_1$)

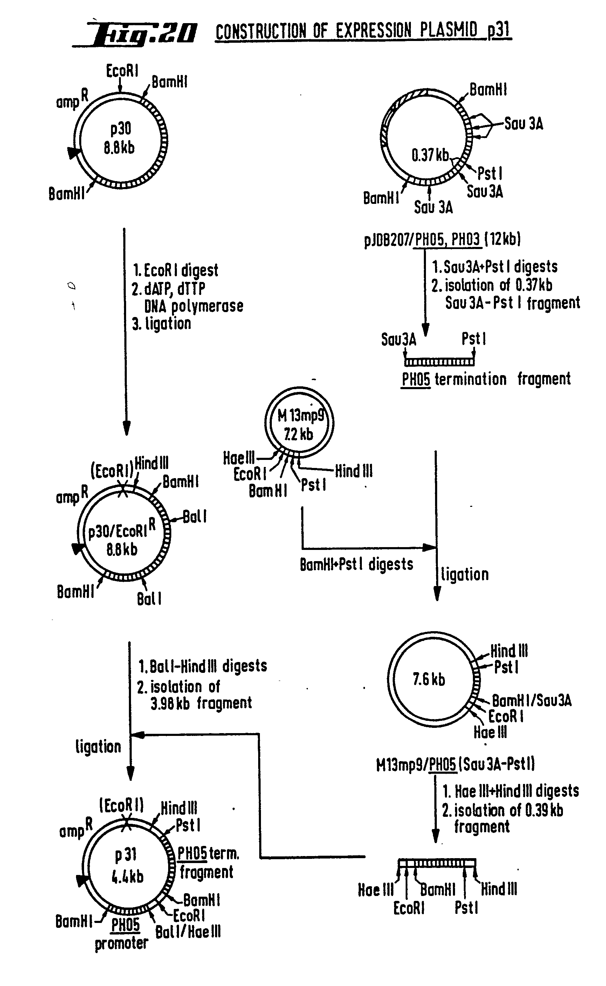

Example 13: Construction of an expression plasmid containing the *PHO5* promoter and *PHO5* transcription termination signals (see fig. 20)

a) Elimination of the EcoRI restriction site in plasmid p30:

The scheme outlined in fig. 20—22 requires elimination of the unique EcoRI restriction site in plasmid p30. Five μg of p30 DNA (cf. Example 4) are digested to completion with restriction endonuclease EcoRI (Boehringer). In order to fill in the resulting sticky ends, 1 μg of EcoRI digested p30 in 50 μl of 50 mM NaCl, 10 mMTris · HCl pH 7.5, 10 mM $MgCl_2$, 1 mM DTT, 0.25 mM dATP and 0.25 mM dTTP is incubated for 30 min 37°C with 1 unit of DNA polymerase (Klenow large fragment, BRL). The DNA recovered from ethanol precipitation is ligated as usual and used for transformation of compotent *E. coli* HB101 cells as described in Example 4. Clones that are resistant to EcoRI digest are referred to as p30/EcoRI$^R$.

b) Isolation of a 0.37 kb Sau3A-PstI *PHO5* transcription termination fragment:

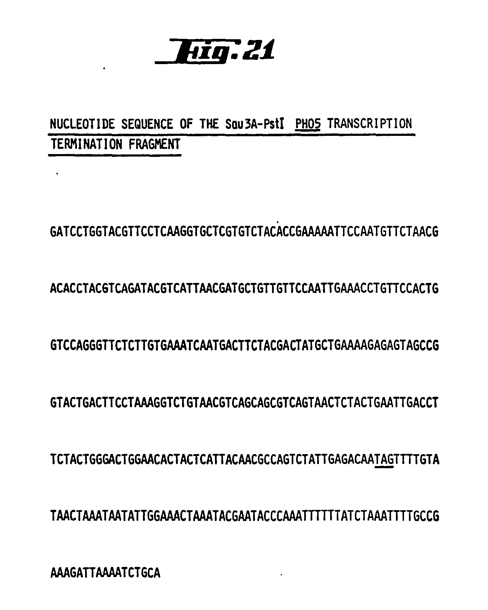

The *PHO5* transcript has been mapped by S1 nuclease mapping (48). The signals for transcription termination have been shown to be located in a 0.37 kb Sau3A-PstI fragment of the *PHO5* gene. The nucleotide sequence of the Sau3A-PstI fragment is given in fig. 21.

Five μg of pJDB207/*PHO5,PHO3* DNA (cf. Example 2) are digested to completion with restriction endonucleases Sau3A and PstI. The restriction fragments are separated on a vertical 1.5% low melting agarose gel in TBE buffer. The 0.37 kb Sau3A-PstI fragment is localized by ethidiumbromide staining and a gel block as small as possible is cut out containing this DNA fragment.

c) Cloning of the Sau3A-PstI *PHO5* fragment in M13mp9:

M13mp9 phage DNA is a useful cloning vector with a cluster of unique restriction sites (49). Five μg of M13mp9 DNA are digested to completion with restriction endonucleases BamHI and PstI. The larger 7.2 kb DNA fragment is separated from a very small fragment (8 bp) on a 0.8% low melting agarose gel. The gel block containing the large DNA fragment is cut out of the gel. Gel blocks with the 0.37 kb Sau3A-PstI fragment of pJDB207/*PHO5,PHO3* (cf. Example 13b) and the 7.2 kb BamHI-PstI fragment of M13mp9 are liquefied at 65°C, mixed in about equimolar amounts and diluted with $H_2O$ to lower the agarose concentration to 0.3%. Ligation is carried out in a 200 µl solution containing 60 mM Tris.HCl pH 7.5, 10 mM $MgCl_2$ 10 mM DTT, 1 mM ATP and 600 units of T4 DNA ligase (Biolabs). Transduction of competent cells of the strain *E. coli* JM101 (Ca++) is done according to the manual "M13 cloning and DNA sequencing system" published by New England Biolabs. Phages from a number of white plaques are grown and analyzed for the size of their DNA insert by cleavage with restriction endonucleases EcoRI and PstI.

A M13mp9 derived clone containing the Sau3A-PstI *PHO5* transcription termination fragment is isolated and referred to as M13mp9/*PHO5* (Sau3A-PstI).

d) Cloning of the *PHO5* transcription termination fragment in p30/EcoRI[R]:

The original *PHO5* transcription termination fragment cloned in phage M13mp9 (M13mp9/*PHO5*(Sau3A-PstI)) is recloned as a HaeIII-HindIII fragment in plasmid p30/EcoRI[R] cleaved with BalI and HindIII: M13mp9/*PHO5*(Sau3A-PstI) DNA is cleaved to completion with restriction endonucleases HaeIII and HindIII. The resulting two DNA fragments are separated on a 1.5% vertical low melting agarose gel in TBE buffer. The 0.39 kb fragment is isolated in a gel block cut out of the gel. p30/EcoRI[R] DNA is digested with BalI and HindIII. The large 3.98 kb fragment is separated on a 0.8% low melting agarose gel in TBE buffer and isolated by cutting a gel block containing the DNA fragment.

Gel blocks with the 0.39 kb HaeIII-HindIII *PHO5* transcription termination fragment and the 3.98 kb BalI-HindIII fragment of p30/EcoRI[R] are melted at 65°C and mixed in about equimolar amounts. Ligation and transformation of competent *E. coli* HB101 cells are as described in Example 4. DNA of transformed cells is analysed by cleavage with BalI and HaeIII. A clone containing the *PHO5* transcription termination fragment is further analyzed and referred to as p31 (see Figure 20).

Expression plasmid p31 contains the *PHO5* promoter region with part of the signal sequence of *PHO5* and adjacent to it a DNA fragment with the *PHO5* transcription termination signals. Foreign coding sequences to be expressed in this vector may conveniently be inserted between promoter and transcription termination sequences.

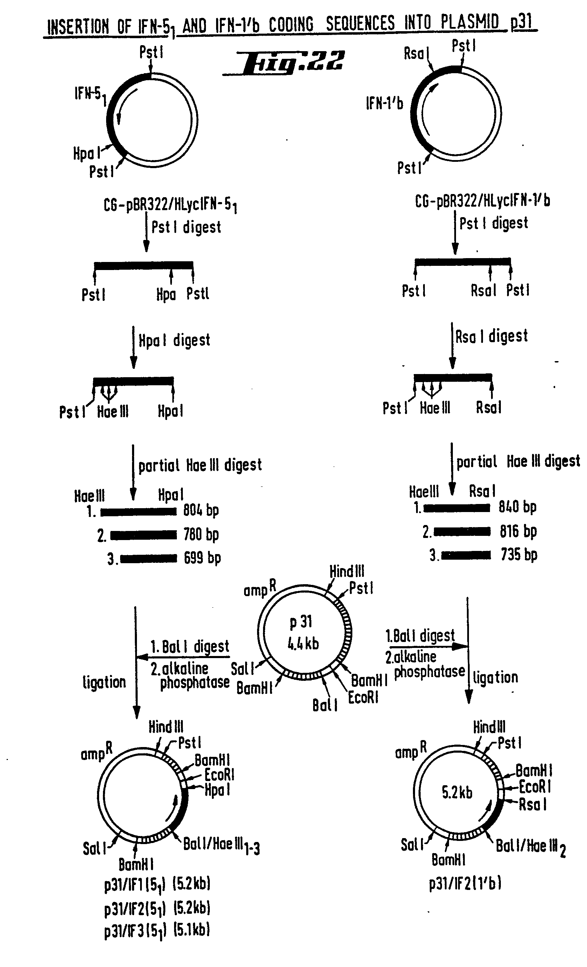

Example 14: Insertion of lymphoblastoid interferon-$5_1$ DNA into plasmid p31 (see Figure 22)
a) Isolation of HaeIII-HpaI fragments of plasmid CG-pBR322/HLycIFN-$5_1$

*E. coli* strain HB101 CG-pBR322/HLycIFN-$5_1$ (see Example 10E) is grown in 100 ml LB medium supplemented with 10 µg/ml tetracyclin and the plasmid DNA is isolated as described in Example 2. Ten µg of CG-pBR322/HLycIFN-$5_1$ DNA are completely digested with restriction endonucleases PstI and HpaI. The restriction fragments are separated on a preparative 0.8% low melting agarose gel. The PstI-HpaI fragment of about 860 bp containing the IFN-$5_1$ coding sequence is cut out of the gel and eluted from the agarose gel as described in Example 4a and purified by DE52 ion exchange chromatography as detailed in Example 5a.

The PstI-HpaI fragment contains 3 HaeIII sites: at position 41, 65 and 146 (from the ATG) in the IFN-$5_1$ coding sequence. Partial HaeIII digestion leads to three HaeIII-HpaI fragments of 699 bp, 780 bp and 804 bp, respectively. HaeIII digestion is carefully adjusted to obtain about equal amounts of all three fragments. The mixture of fragments is phenol extracted, ethanol precipitated and resuspended in 10 mM Tris pH8 at a concentration of 0.1 mg/ml.

b) Preparation of BalI cleaved, dephosphorylated plasmid p31

Six µg of p31 DNA (cf. Example 13d) are completely digested with restriction endonuclease BalI (BRL). After phenol extraction and ethanol precipitation the DNA is redissolved in 100 µl of 50 mM Tris pH 8.0 and passed through a 50 µl bed of equilibrated Chelex 100 (BioRAD) in a siliconized Pasteur pipet. The flow through and 450 µl of subsequent wash are combined. 0.4 units of calf intenstine alkaline phosphatase (Boehringer) are added. After 1 h of incubation at 37°C the enzyme is inactivated at 65°C for 1.5 hrs. The NaCl concentration in the incubation mixture is adjusted to 150 mM. The linearized dephosphorylated p31 DNA is purified by DE52 ion exchange chromatography (see Example 5a). After ethanol precipitation the DNA is resuspended in 10 mM Tris pH 8 at a concentration of 0.3 mg/ml.

c) Ligation of linearized, dephosphorylated p31 DNA to the HaeIII-HpaI fragments of IFN-$5_1$ DNA

0.6 µg of dephosphorylated p31 vector DNA cleaved with BalI is ligated to 0.5 µg of partial HaeIII-HpaI fragments of IFN-$5_1$ DNA (see Example 14a). Ligation is carried out in 10 µl of 60 mM Tris pH 7.5, 10 mM $MgCl_2$, 10 mM DTT, 4 mM ATP and 300 units of T4 DNA ligase (Biolabs) overnight at room temperature. A 1 µl aliquot of the ligation mixture is added to 50 µl of calcium treated transformation competent *E. coli* HB101 cells. The transformation protocol is as described in Example 4a.

Transformed, amp[R] colonies are grown individually in LB medium containing 100 µg/ml ampicillin. Plasmid DNA is prepared according to the method of Holmes et al., (50) and analysed by digestion with restriction endonuclease BstEII (one unique site in the *PHO5* promoter).

20 clones containing the IFN-$5_1$ insert are further analysed by BstEII-EcoRI double digests to determine the orientation and size of the insert. Among 8 clones with the insert in the right orientation all 3 expected insert sizes are found. The size corresponds to the three HaeIII$_{1-3}$-HpaI fragments created by partial HaeIII digest of the IFN-5$_1$ gene (cf. Example 14a). The clones are referred to as p31/IF1(5$_1$), p31/IF2(5$_1$) and p31/IF3(5$_1$) with IFN-5$_1$ inserts of 804 bp (HaeIII$_1$-HpaI insert), 780 bp (HaeIII$_2$-HpaI) and 699 bp (HaeIII$_3$-HpaI), respectively.

Example 15: Insertion of lymphoblastoid interferon-1'b DNA into plasmid p31 (see Fig. 22)
a) Isolation of HaeIII-RsaI fragments of plasmid CG-pBR322/HLycIFN-1'b:
Ten µg of CG-pBR322/HLycIFN-1'b DNA (see Example 10E) are digested with restriction endonucleases PstI and RsaI. The restriction fragments are separated on a 0.8% low melting agarose gel. A PstI-RsaI fragment of about 870 bp is isolated from the gel and purified as described above (Example 14a).
The PstI-RsaI fragment contains three HaeIII sites: at positions 13, 37 and 118 from the ATG of the IFN-1'b coding sequence. Partial HaeIII digestion leads to three HaeIII-RsaI fragments of 735 bp, 816 bp and 840 bp, respectively. The mixture of fragments is phenol extracted, ethanol precipitated and resuspended in 10 mM Tris pH 8.0 at a concentration of 0.1 mg/ml.

b) Ligation of linearized, dephosphorylated p31 DNA to HaeIII-RsaI fragments of IFN-1'b DNA
0.6 µg of dephosphorylated p31 vector DNA cleaved with BalI (see Example 14b) is ligated to 0.5 µg of partial HaeIII-RsaI fragments of IFN-1'b DNA. The ligation procedure, the transformation of competent E. coli, HB 101 cells with the ligation mixture and the selection of the transformed amp$^R$ colonies is carried out as described in Example 14c. Plasmid DNA is prepared according to the method of Holmes et al. (50) and analysed by digestion with restriction endonuclease BstEII
7 clones containing the IFN-1'b insert are analysed by BstEII-PvuII double digests. Two clones are shown to contain the HaeIII$_2$-RsaI fragment (816 bp) in the right orientation. This construction is referred to as p31/IF2(1'b).

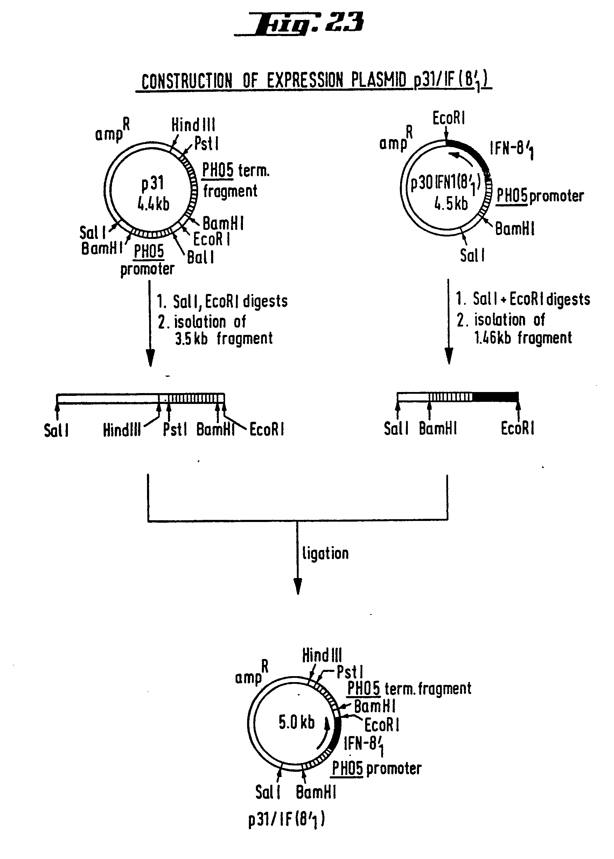

Example 16: Insertion of lymphoblastoid interferon-8'$_1$ DNA into plasmid p31 (see figure 23)
a) Isolation of a 1.46 kb SalI-EcoRI fragment of plasmid p30IFN1(8'$_1$)
Five µg of p30IFN1(8'$_1$) DNA (see Example 5d) is digested with restriction endonucleases SalI and EcoRI. A 1.46 kb SalI-EcoRI fragment, containing the PHO5 promoter linked to the protein coding region of IFN-8'$_1$ is separated on a 0.8% low melting agarose gel. The DNA band is localized by ethidium bromide staining and cut out of the gel.

b) Isolation of a 3.5 kb SalI-EcoRI fragment of plasmid p31
Five µg of p31 DNA (see Example 13d) are completely digested with restriction endonucleases SalI and EcoRI. The 3.5 kb vector fragment containing the PHO5 transcription termination sequence is separated on a 0.8% low melting agarose gel and the DNA band is cut out.

c) Ligation of a 1.46 kb SalI-EcoRI fragment of p30IFN1(8'$_1$) to a 3.5 kb SalI-EcoRI fragment of p31
Gel blocks with 0.67 µg of the 3.5 kb SalI-EcoRI fragment of p31 and 0.5 µg of the 1.46 kb SalI-EcoRI fragment of p30IFN1(8'$_1$) are ligated in 240 µl as described in Example 4a at 15°C overnight. 10 µl of the ligation mixture are used to transform competent E. coli HB101 cells.
Transformed, amp$^R$ colonies are grown individually in LB medium containing 100 µg/ml ampicillin. Plasmid DNA is prepared according to the method of Holmes et al. (50) and analysed by digestion with restriction endonuclease BstEII (one unique site in the PHO5 promoter).
A number of clones containing the IFN-8'$_1$ insert are analysed by BstEII-PvuII double digests. They all contain the 1.46 kb SalI-EcoRI fragment. The identical clones are referred to as p31/IF(8'$_1$).

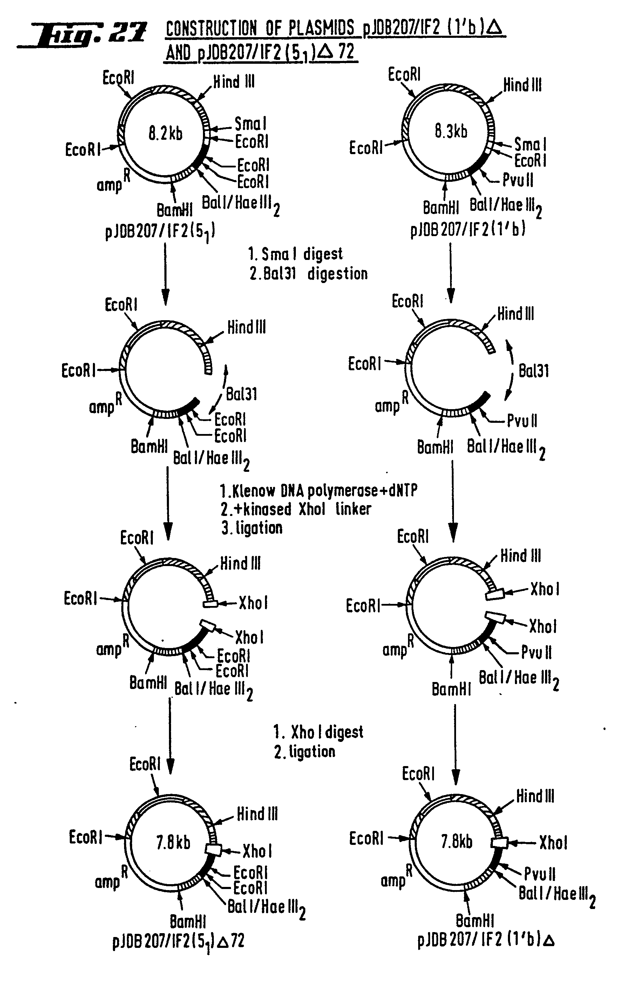

Example 17: Subcloning of gene constructions in the high copy number yeast vector pJDB207
The constructions described in Examples 14—16 contain the PHO5 promoter, different interferon coding regions and the PHO5 transcription termination signals in a tandem array, all inserted in a pBR322 derived vector. For expression in yeast the whole insert is subcloned as such in yeast vector pJDB207 (28) allowing selection for leucine prototrophic colonies (cf. Example 9 and fig. 6).
2 µg each of p31/IF(8'$_1$) DNA, p31/IF1(5$_1$) DNA, p31/IF2(5$_1$) DNA, p31/IF3(5$_1$) DNA and p31/IF2(1'b) DNA are digested with restriction endonucleases SalI and HindIII. The restriction fragments are separated on a preparative 0.8% low melting agarose gel. The small fragment (~2 kb in size) of each digest is cut out of the gel.
10 µg of pJDB207 DNA are digested with restriction endonucleases SalI and HindIII. The large 6.2 kb fragment is isolated from a preparative 0.8% low melting agarose gel. Gel blocks containing the DNA fragments are liquified at 65°C and diluted with H$_2$O to lower the agarose concentration to about 0.3%.
Each of the 2 kb SalI-HindIII fragments of the plasmids p31/IF(8'$_1$), p31/IF1(5$_1$), p31/IF3(5$_1$), p31/IF3(5$_1$) and p31/IF2(1'b) is mixed with an equimolar amount of the 6.2 kb HindIII-SalI fragment of pJDB207. Ligations are carried out in 100 µl for 4 hrs at 15°C. 10 µl of each ligation mixture are used to transform competent E. coli HB101 cells as described in Example 4a. Several amp$^R$ colonies from each experiment are grown individually in LB medium containing 100 µg/ml of ampicillin. The plasmid DNA is analysed for the size of the insert by cleavage with restriction endonucleases HindIII and SalI. The resulting clones with the correct inserts are named pJDB207/IF(8'₁), pJDB207/IF1(5₁,) pJDB207/IF2(5₁) (cf. fig. 27), pJDB207/IF3(5₁) and pJDB207/IF2(1'b) (cf. fig. 27).

Example 18: Transformation of *Saccharomyces cerevisiae* AH220 and induction of interferon production:

Plasmids pJDB207/IF(8'₁), pJDB207/IF1(5₁), pJDB207/IF2(5₁), pJDB207/IF3(5₁) and pJDB207/IF2(1'b) are each introduced into *Saccharomyces cerevisiae* strain AH220 (*a, trp1, leu2—3, leu2—112, his3, pho5, pho3*) using the transformation protocol described by Hinnen et al. (1) Transformed yeast cells are selected on yeast minimal medium plates deficient in leucine. Single transformed yeast colonies are picked and grown as described in Example 7. The different yeast colonies are referred to as

*Saccharomyces cerevisiae* AH220/pJDB207/IF(8'₁),
*Saccharomyces cerevisiae* AH220/pJDB207/IF1(5₁),
*Saccharomyces cerevisuae* AH220/pJDB207/IF2(5₁),
*Saccharomyces cerevisuae* AH220/pJDB207/IF3(5₁) and
*Saccharomyces cerevisiae* AH220/pJDB207/IF2(1'b)

Example 19: Preparation of yeast cell extracts and determination of the interferon titer:

Cell extracts are prepared and interferon activity is determined as described in Example 8. The results are summarized in Table 3.

TABLE 3

Interferon activity in *Saccharomyces cerevisiae* strain AH220 after transformation with the following recombinant plasmids:

| Plasmids | Interferon activity units/ml yeast cell extract |
|---|---|
| pJDB207/IF(8'₁) | $1 \cdot 10^7$ |
| pJDB207/IF1(5₁)<br>pJDB207/IF2(5₁)<br>pJDB207/IF3(5₁) | $7 \cdot 10^5$<br>$5 \cdot 10^5$<br>$3 \cdot 10^3$ |
| pJDB207/IF2(1'b) | $4 \cdot 10^3$ |

Example 20: Expression of hepatitis B virus surface (HBVs) antigen under the control of the yeast *PHO5* promoter

a) Construction of a fusion between the *PHO5* promoter and the HBVs protein coding region

5 µg DNA of plasmid pHBV130 (51) is digested with restriction endonuclease AvaI as recommended by the supplier (New England Biolabs). A fragment of 1336 base pairs is obtained which contains the entire protein coding region of HBVs, including 27 base pairs of a potential pre-HBVs sequence (see Fig. 2 in ref. 51; the AvaI fragment spans the DNA segment from the Xho site until an AvaI site 62 base pairs beyond the BamHI site located between the surface coding region and the core coding region). The 1336 base pair fragment is purified by soft agarose electrophoresis (0.8% agarose gel) as described in Example 4a.

5 µg of plasmid pBR322/*PHO5* Bam-Sal (see fig. 1) is cut with restriction endonucleases SalI and AvaI (position 1424 of pBR322) and the resulting 3.9 kb vector fragment containing pBR322 sequences together with the *PHO5* Bam-Sal segment is purified by soft agarose electrophoresis as described above.

1 µg of the 1336 base pair fragment is ligated to 3 µg of the 3.9 kb vector fragment in 50 µl of 60 mM Tris-HCl pH 7.5, 10 mM MgCl₂, 10 mM DTT, 1 mM ATP and 600 units of T4 DNA ligase (Biolabs) at 15°C for 4 hours. Transformation of *E. coli* HB101 to ampicillin resistance and plasmid isolation is carried out as described in Example 4a.

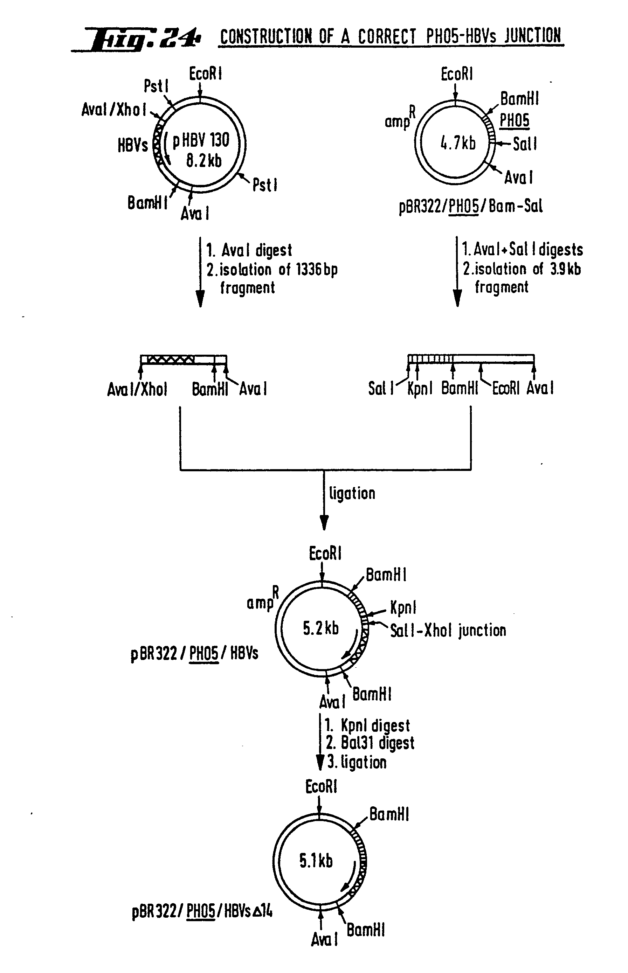

The correct structure of the plasmid is verified by restriction analysis. The new plasmid thus constructed is called pBR322/*PHO5*/HBVs (see fig. 24).

b) Adjustment of the *PHO5* promoter to the exact HBVs protein coding region

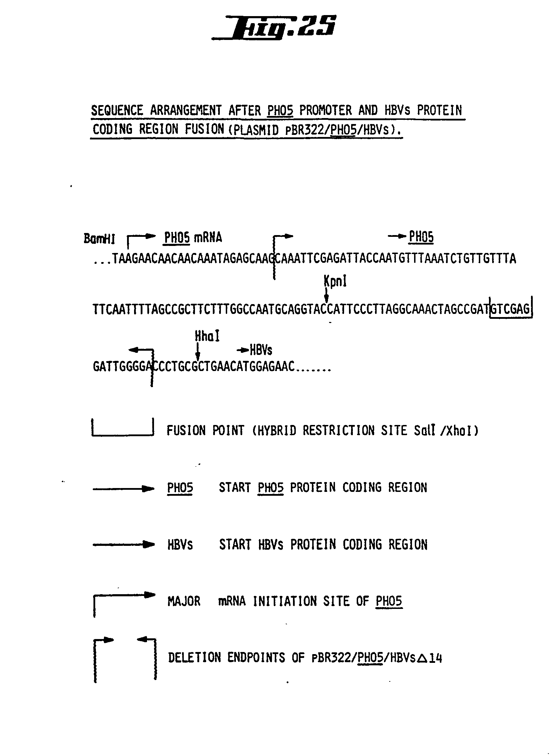

The fusion described creates a DNA sequence arrangement as depicted in fig. 25. The sequence data are taken from fig. 3 (*PHO5*) and from Pasek et al. (52; HBVs). The mRNA initiation site is determined by conventional S1 mapping (48) using the BamHI-SalI fragment of pBR322/*PHO5* Bam-Sal (fig. 1). In order to eliminate the *PHO5* protein coding region present in pBR322/*PHO5*/HBVs 5 µg of the plasmid is digested with restriction endonuclease KpnI (conditions specified by supplier, New England Biolabs) which produces a linearized plasmid.

4 µg of linearized plasmid is digested with 1 unit of exonuclease Bal31 (Bethesda Research Laboratory) at 30°C for 45 seconds in 12 mM CaCl₂, 12 mM MgCl₂, 300 mM NaCl, 20 mM Tris, 1 mM EDTA pH 8.1 in a total volume of 100 µl. The reaction is stopped by phenol extraction as described above. After ethanol precipitation the DNA is resuspended in 50 µl TE.

1 µg of DNA is recircularized by ligation with T4 DNA ligase in a volume of 20 µl (conditions see Example 4a). After transformation of *E. coli* HB101 to ampicillin resistance (see Example 4a) plasmid DNA is isolated as described and individual plasmid preparations are analysed by restriction analysis with the following enzymes: HaeIII, PstI, BstEII and HhaI. This analysis allows the determination of the presence of the HhaI site (6 base pairs before the start of the HBVs protein coding region) and gives a measure for the size of the deletion. The DNA sequence in the junction area is determined using the method of Maxam and Gilbert (15) (radioactive labelling at the BstEII site at position-374, see fig. 3). The endpoints of the deletion generated in one of the plasmids are indicated in fig. 24. This plasmid is called pBR322/*PHO5*/HBVsΔ14.

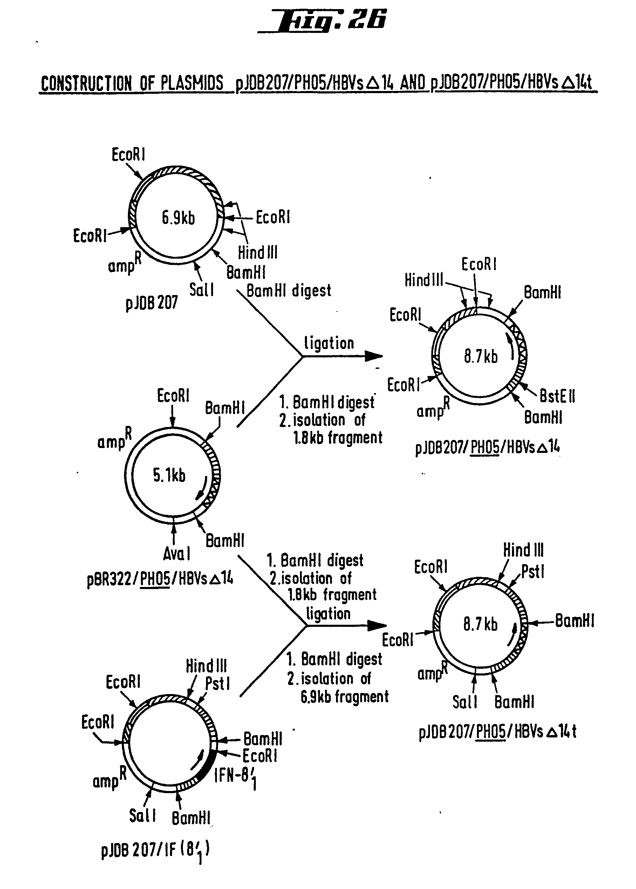

c) Transfer of the *PHO5*-HBVs fusion to the yeast plasmid pJDB207 (see fig. 26)

5 µg DNA of plasmid pBR322/*PHO5*/HBVsΔ14 is digested with restriction endonuclease BamHI (New England Biolabs, conditions as described by supplier). A 1.8 kb BamHI fragment is prepared by soft agarose gel electrophoresis (0.8% agarose) as described in Example 4a. 2 µg of the yeast vector pJDB207 is digested with the same enzyme. 1 µg of digested pJDB207 and 1 µg of the 1.8 kb BamHI restriction fragment is ligated in a total volume of 20 µl using the conditions described in Example 20a. Transformation of *E coli* HB101 to ampicillin resistance and isolation of plasmid DNA is carried out as described above (Example 4a). Individual plasmids are analyzed by BamHI restriction analysis and the orientation of the inserted BamHI fragment is determined by HindIII/BstEII double digestion. Fig. 26 outlines the construction. The plasmid obtained as indicated in fig. 26 is called pJDB207/*PHO5*/HBVsΔ14.

The plasmid is transformed into yeast strain AH220 as described in Example 7. Transformed yeast cells are selected, incubated in liquid medium and grown under derepressing conditions as described in Example 7. A single transformed yeast colony is referred to as *Saccharomyces cerevisiae* AH220/pJDB207/HBVsΔ14.

Preparation of cell extract is done as described in Example 8 and the amount of HBVs protein produced is determined using the Abbott radioimmuno assay (51). Under the assumption that the HBVs antigen produced by yeast reacts similarly to the antigen in human serum, about 2 µg HBVs antigen per ml yeast extract are found under derepressing conditions. Under repressing conditions the titer is below 0.001 µg/ml.

d) Transfer of the *PHO5*-HBVs fusion to a yeast plasmid containing a *PHO5* transcription termination sequence

5 µg of DNA of plasmid pJDB207/IF(8'₁) (Example 17) is digested with BamHI as described above and the 6.9 kb vector part is isolated by soft agarose electrophoresis (0.8% agarose) as described in Example 4a. 5 µg of pBR322/*PHO5*/HBVsΔ14 is digested with BamHI as described above and the 1.8 kb BamHI fragment is isolated by soft agarose gel electrophoresis. 1 µg of the 6.9 kb vector fragment is ligated with 1 µg of the 1.8 kb BamHI fragment in a total volume of 20 µl using the conditions described in Example 20a. Transformation of *E. coli* HB101 to ampicillin resistance and plasmid isolation is done as described in Example 4a. Plasmid analysis is performed by restriction endonuclease digestions. The plasmid obtained as indicated in fig. 26 is called pJDB207/*PHO5*/HBVsΔ14t. Transformation of yeast strain AH220 and selection of the transformed cells is done as described in Example 7. A single transformed yeast colony is referred to as *Saccharomyces cerevisiae* AH220/pJDB207/HBVsΔ14t.

Example 21

Hepatitis B virus (HBV) DNA sequences excised from plasmids

pBR322-PstI dG: HBV-KpnI dC;
pBR322-PstI dG: HBV-BamHI dC;
pBR322-PstI dG: HBV-BglII dC;
pBR322-PstI dG: HBV-EcoRI dC;
pBR322-BamHI: HBV-BamHI;
pBR322-EcoRI: HBV-EcoRI;
pBR322-PstI dG: HBV-KpnI dC,
pBR322-PstI' dG: pHBV114-PstI dC;
((pBR322-EcoRI HindIII: Lac promoter sequence)-HindIII: HBV114-HhaI HindIII linkers)-BamHI,
pUR2-EcoRI: HBV114-HhaI EcoRI linkers,
pUR2-EcoRI: HBV114-HhaI EcoRI linkers;
pBR322-PstI dG: pHBV114-AvaI dC, and
pBR322-PstI dG: pHBV114-TaqI dC

as described in European Patent Application 13828 can be inserted (preferably after appropriately adapting the termini) into plasmids p30 or p31 according to Examples 5, 14 or 15 and subsequently into plasmid pJDB207 according to Example 17. Transformation of *S. cerevisiae* is performed according to Example 18. Expression of polypeptides displaying HBV antigenicity is determined according to Example 20c.

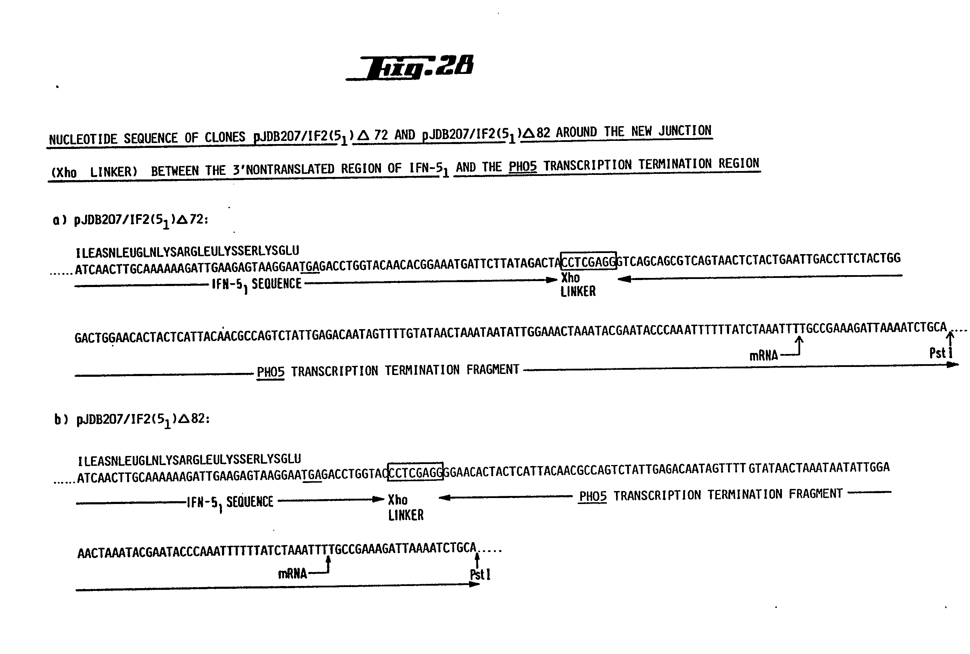

Example 22: Deletion of 3' nontranslated DNA sequences in plasmids pJDB207/IF2(5₁) and pJDB207/IF2(1'b) (see figures 27 and 28)

The construction of the plasmids pJDB207/IF2(5₁) and pJDB207/IF2(1'b) (cf. Example 17) resulted in a relative long 3' nontranslated region of about 440 bp and 480 bp, respectively. To shorten this region of the constructs the DNA is digested with exonuclease Bal31 from an unique SmaI site in the middle of this region. Xho linkers are introduced and the DNA is circularized by ligation.

a) Bal31 digestion of SmaI cleaved plasmids pJDB207/IF2(5₁) and pJDB207/IF2(1'b)

20 µg each of the plasmid DNAs are digested with restriction endonuclease SmaI. After extraction with phenol/chloroform, the DNA is precipitated with ethanol. The DNA is resuspended in 10 mM Tris pH 8.0 at a concentration of 0.5 mg/ml. 10 µg of the SmaI cleaved DNAs are each digested with 2 U of endonuclease Bal31 (BRL) in 100 µl of 20 mM Tris pH 8.0, 100 mM NaCl, 12 mM MgCl₂, 12 mM CaCl₂ and 1 mM EDTA. Aliquots of 3 µg of DNA are withdrawn after 90, 120 and 150 seconds of incubation at 30°C and are immediately mixed with 50 µl of phenol and 60 µl TNE. After extraction with phenol/chloroform and ethanol precipitation, the DNA is resuspended in 10 mM Tris pH 8.0 at a concentration of 100 µg/ml. To analyse the exconucleolytic digestion of Bal31, aliquots of 0.7 µg of DNA from each time point are digested with HindIII/EcoRI for pJDB207/IF2(5₁)-derived samples or with PvuII/HindIII for pJDB207/IF2(1'b)-derived samples. For further experiments the DNAs from the 90 second time points are used.

b) Addition of XhoI linkers to the Bal31 treated DNAs

2.2 µg each of plasmid DNA pJDB207/IF2(5₁) and pJDB207/IF2(1'b), after 90 sec. of Bal31 digestion (see Example 22a) are incubated for 1 hour at 37°C with 2.8 U of Klenow DNA polymerase (large fragment of polymerase I, BRL) in 35 µl of 60 mM Tris pH 7.5, 10 mM MgCl₂ and 0.1 mM dNTP's.

Three µg of XhoI linkers (5'-CCTCGAGG-3', Collaborative Research) are kinased in 50 µl of 6 mM Tris pH 7.5, 10 mM MgCl₂, 4 mM DTT, 0.5 mM ATP and 35 U of T4 polynucleotide kinase (Boehringer) for 30 min at 37°C.

0.67 µg of kinased XhoI linkers and 0.4 µg of Bal31 treated blunt end DNA of plasmid pJDB207/IF2(5₁) of pJDB207/IF2(1'b) are ligated over night at room temperature in 25 µl of 60 mM Tris pH 7.5, 10 mM MgCl₂ 5 mM DTT, 3.5 mM ATP and 450 U of T4 DNA ligase. The ligated DNA is separated from excess linkers by isopropanol precipitation in the presence of 10 mM EDTA, 0.3 M sodiumacetate pH 6.0 and 0.54 volumes of isopropanol. After 35 min. of incubation at room temperature the DNA is sedimented by centrifugation. The pellets are dried at the air and resuspended in 17 µl of 6 mM Tris pH 7.9, 150 mM NaCl, 6 mM MgCl₂ and 6 mM mercaptoethanol. The XhoI linkers ligated to the DNA are cleaved with XhoI, the DNA is precipitated with isopropanol as described before and circularized by ligation. After 6 hours of ligation at 15°C in 50 µl of 60 mM Tris pH 7.5, 10 mM MgCl₂, 10 mM DTT, 1 mM ATP and 600 U of T4 DNA ligase 10 µl of each ligation mixture are added to 100 µl of calcium-treated, transformation competent *E. coli* HB101 cells (see Example 4a).

72 transformed, amp^R colonies containing plasmids with an IFN-5₁ insert are grown individually in LB medium containing 100 mg/l ampicillin. Plasmid DNA is analysed by HaeIII digestion. The restriction pattern allows to judge the approximate size of the deletion introduced by Bal31. Two clones are further analysed and assayed for interferon activity. They are referred to as pJDB207/IF2(5₁)Δ72 and pJDB207/IF2(5₁)Δ82.

The nucleotide sequence on either side of the new junction (XhoI linker) between the 3' nontranslated region of the IFN-5₁ gene and the *PHO5* transcription termination region is given in fig. 28.

In an analogous manner 60 amp^R colonies containing plasmids with an IFN-1'b insert are grown individually in LB medium containing 100 mg/l ampicillin. Plasmid DNA is analysed as described above. One clone is selected and assayed for IFN activity. It is referred to as pJDB207/IF2(1'b)Δ.

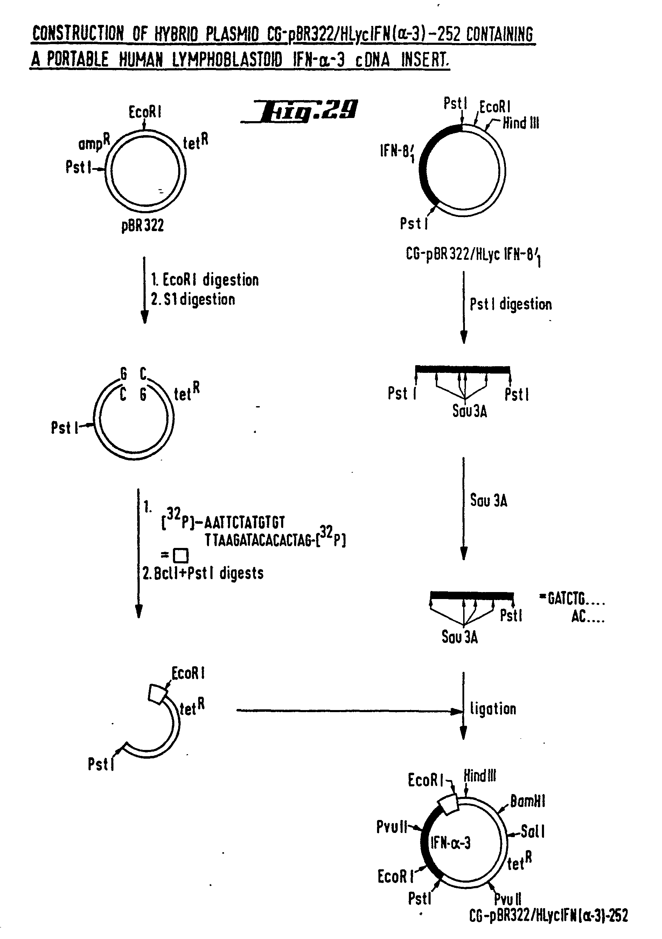

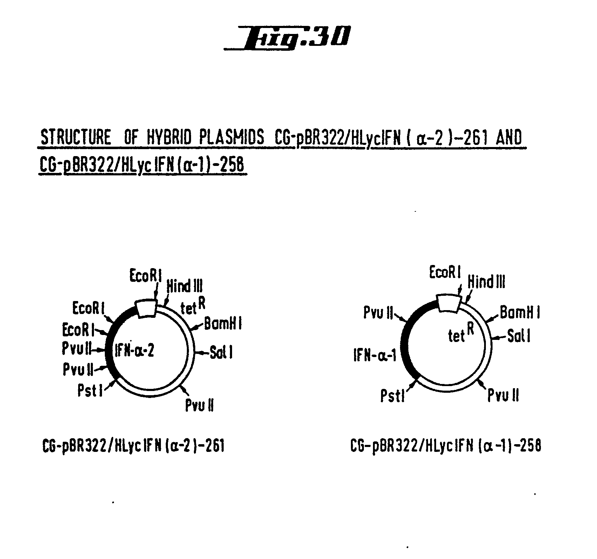

Example 23: Construction of recombinant plasmids, containing portable IFN-5₁, -8'₁ and -1'b cDNA inserts which can be used for direct expression of mature lymphoblastoid IFN (cf. figures 29 and 30)

a) Preparation of the cDNA inserts

The cDNA inserts are excised from the recombinant plasmids CG-BR322/HLycIFN-8'₁, CG-pBR322/HLycIFN-1'b, CG-pBR322/HLycIFN-5₁ by digestion of each of 150 µg of plasmid DNA with PstI (Biolabs) using the procedure as suggested by the supplier. Following phenol extraction and ethanol precipitation, the excised inserts are isolated by means of sucrose density gradient centrifugation (5—23%) in 50 mM Tris-HCl (pH 8.0) and 1 mM EDTA. The centrifugation is performed at 35 000 rpm in a TST 41 rotor (Kontron AG) at 15°C for 16 h. 0.3 ml fractions are collected with an ISCO gradient collector at 1 ml/min. The fractions containing the small fragment (i.e. the insert) are pooled. The DNA is precipitated with ethanol as usual, and the precipitate is collected by centrifugation in a HB-4 rotor (Sorvall) at 10 000 rpm at 0°C for 10 min. The precipitate is redissolved in 60 µl 10 mM Tris-HCl (pH 7.5) and 0.05 mM EDTA. About 30 µg DNA are recovered as determined by measuring the optical density.

2 µg of each of the cDNA inserts are digested with 2.5 units of Sau3A (Biolabs) in 10 µg/ml EtBr and incubated at 37°C for 60 min. The digests are phenol extracted and the DNA is precipitated in ethanol as above. The DNA fragments are fractionated on a 1.2% agarose gel in a solution containing 50 mM Tris, 50 mM boric acid, 1 mM EDTA and 0.5 µg/ml ethidium bromide.

The second largest DNA fragment (Sau 3A-PstI) from each of the 3 digests is excised from the gel, squirted through a thin needle with a syringe into 5 ml of 0.15 M NaCl, 50 mM Tris · HCl (pH 8.0), 1 mM EDTA, and eluted overnight by shaking. The eluate is passed through a 100 µl DE-52 (Whatman)

# EP 0 100 561 B1

Pasteur-pipette column to adsorb the DNA. The column is washed with 2 ml of the same buffer and the DNA is eluted with 400 µl of a solution containing 1.5 mM NaCl, 50 mM Tris (pH 8.0) and 1 mM EDTA. The DNA is precipitated with 2 volumes of ethanol at −20°C overnight. The precipitates are collected by centrifugation in an Eppendorf centrifuge and redissolved in 10 µl 10 mM Tris · HCl (pH 7.8), 0,05 mM EDTA.

b) Preparation of the acceptor plasmid DNA-fragment
1. Digestion of the plasmid pBR322 by EcoRI and $S_1$

10 µg of plasmid DNA pBR322 are digested with 15 units of EcoRI (Biolabs) for 60 min. at 37°C under conditions described by the supplier. Following phenol extraction and ethanol precipitation, the DNA is redissolved in 25 µl $H_2O$ and the staggered ends are removed by treatment of the DNA with 20 units of endonuclease $S_1$ (P-L Biochemicals) in 350 µl of a solution containing 250 mM NaCl, 30 mM sodium acetate (pH 4.5), 1 mM $ZnSO_4$ at 30°C for 30 min. The reaction is stopped by adding EDTA (pH 7.5) at 10 mM. The DNA is extracted with phenol, concentrated by ethanol precipitation and redissolved in 50 µl of 10 mM Tris · HCl (pH 7.8), 0.05 mM EDTA.

2. Ligation of a chemically synthesized DNA linker to pBR322 digested with EcoRI and $S_1$

Two oligodeoxynucleotides of the formulae

5′-AATTCTATGTGT-3′

and

5′-GATCACACATAGAATT-3′

are synthesized using the procedure described in Example 10 Ea.

The synthetic oligodeoxynucleotides are phosphorylated at their 5′-ends by incubating 80 pmoles of both oligodeoxynucleotides with 20 µCi [γ-$^{32}$P]-ATP (6700 Ci · mmol$^{-1}$, Amersham) in a 80 µl reaction volume, containing 0.1 mM rATP (Sigma), 50 mM Tris · HCl (pH 9.5), 10 mM $MgCl_2$, 5 mM DTT and 20 units of $T_4$ polynucleotide Kinase (P-L Biochemicals) for 30 min. at 37°C. The reaction is stopped by freezing the mixture at −80°C.

The resulting radioactively phosphorylated linker of the formula

[$^{32}$P]-AATTCTATGTGT

TTAAGATACACACTAG-[$^{32}$P]

is subsequently incubated with 6 µg of pBR322 cleaved with EcoRI and $S_1$ (see above) in a 200 µl reaction volume containing 0.5 mM rATP (Sigma) 0.1 mM DTT (Calbiochem), 20 mM Tris · HCl (pH 7.8), 10 mM $MgCl_2$ and 4 · 10$^3$ units of $T_4$ DNA ligase (Biolabs) by incubating the mixture for 2 h at 15°C.

The solution is deproteinized by phenol extraction and the DNA is concentrated by ethanol precipitation. The DNA is redissolved in 100 µl 10 mM Tris · HCl (pH 7.5), 0.05 mM EDTA and centrifuged through a sucrose gradient (5—23%) in 50 mM Tris · HCl (pH 8.0), 1 mM EDTA. The centrifugation is performed at 60 000 rpm in a TST 60 rotor (Kontron AG) at 15°C for 2.5 h. 0.2 ml fractions are collected with an ISCO gradient collector at 1 ml/min. The fractions containing the [$^{32}$P]-labeled plasmid DNA (fractions 11—14 out of 22 fractions) are pooled, the DNA is precipitated with ethanol and digested with 12 units of BclI (Biolabs) as recommended by the supplier. After phenol extraction and ethanol precipitation, the digested DNA is treated with 10 units of PstI (Biolabs) as recommended by the supplier. The phenol extracted digest is then centrifuged through a 5—23%) sucrose density gradient for 15 h at 35 000 rpm at 15°C in a TST 41 rotor (Kontron AG). 0.3 ml fractions are collected (see above) and the fractions containing the large [$^{32}$P]-labeled BclI—PstI DNA fragment (fractions 27—31 out of 42 fractions) are pooled and concentrated by ethanol precipitation. The DNA is redissolved in 20 µl Tris · HCl pH 7.8, 0.05 mM EDTA.

c. Ligation of the acceptor plasmid fragment of the cDNA inserts

2 µl of the acceptor plasmid DNA fragment (~100 ng) (see above) are incubated with each of 5 µl of cDNA inserts (~20 ng) (see above) in a reaction volume containing 20 mM Tris · HCl (pH 7.8), 10 mM $MgCl_2$, 0.1 mM rATP, 0.1 mM DTT and 400 units of $T_4$ DNA ligase in 10 µl for 3 h at 15°C.

5 µl of the reaction mixture are then added to a mixture containing 150 µl calcium-treated *E. coli* HB 101 in 10 mM $MgCl_2$, 10 mM $CaCl_2$ and 10 mM Tris · HCl (pH 7.5) in a total volume of 200 µl.

The mixture is cooled in ice for 20 min. heated to 42°C for 1 min. and incubated at 20°C for 10 min. 1 ml of tryptone medium (tryptone medium contains 10 g Bacto-Trypton (Difco); 1 g yeast extract (Difco); 1 g glucose, 8 g NaCl and 294 mg $CaCl_2$ · 2 $H_2O$ in 1 l of distilled water) is added and the mixture is incubated for 30 min. at 37°C by shaking at 300 rpm. The mixture is plated onto 2 agar plates (Mc Conkey agar, Difco; 0.6 ml/plate) supplemented with 10 µg/ml of tetracycline (Sigma). The plates are incubated at 37°C for 12—17 h. Approximately 1000 colonies are obtained per transformation mixture. 4 colonies are picked from each transformation mixture for further analysis.

31

d) Restriction analysis of the hybrid plasmids

In order to further analyse the potential hybrid plasmids, the plasmid DNA is isolated from 12 colonies (4 from each of the 3 ligation mixtures, see above).

The hybrid plasmid DNA is isolated as follows: 1 colony is used to inoculate 10 ml of tryptone medium, supplemented with 10 µg/ml of tetracycline as above in a 25 ml Erlenmeyer flask. The culture is shaken for 15—18 h at 37°C at 300 rpm. The cells are harvested by centrifugation (Sorvall, HS-4 rotor, 10 min. at 400 rpm, 4°C). About 0.1 g of cells are obtained and are resuspended in 1 ml 50 mM Tris · HCl (pH 8.0). 0.25 ml of lysozyme solution (10 mg/ml in 50 mM Tris-HCl (pH 8.0), lysozyme is puchased from Sigma) are added and after incubation at 0°C for 10 min., 0.15 ml of 0.5 M EDTA (pH 7.5) is added. After another 10 min. at 0°C, 60 µl of 2% Triton X-100 (Merck) is added. After 30 min. at 0°C, the sample is centrifuged for 30 min. at 15000 rpm and 4°C in a Sorvall SA-600 rotor. The supernatant is deproteinized with 1 volume of phenol (saturated in TNE). The phases are separated by centrifugation (Sorvall HB-4 rotor) for 10 min. at 5000 rpm at 4°C. The upper phase is extracted twice with 1 volume of chloroform. Pancreatic RNAs A (Sigma; 10 mg/ml in TNE, preheated 10 min. at 85°C) is added to a final concentration of 25 µg/ml and the mixture is incubated for 40 min. at 37°C. The solution is then adjusted to 1 M NaCl and 10% polyethylene glycol 6000 (Fluka, autoclaved for 20 min. at 120°C) and incubated at −10°C for 2 h. The precipitate is collected in a Sorvall HB-4 rotor (20 min. at 10 000 rpm, 0°C) and redissolved in 100 µl of TNE. The DNA solution is extracted with 1 volume of phenol and the DNA is precipitated with 2 volumes of ethanol at −80°C for 10 min.

The precipitate is collected by centrifugation in an Eppendorf centrifuge and the DNA is redissolved in 20 µl of 10 mM Tris · HCl (pH 7.5) and 0.5 mM EDTA. 8—10 µg of hybrid plasmid DNA are recovered from 10 ml culture.

All of the plasmid DNAs are analysed by the following double digests: 0.5 µg of each DNA is digested with HindIII (Biolabs) and PvuII (Biolabs), HindIII and PstI (Biolabs), HindIII and BamHI (Biolabs), EcoRI (Biolabs) and PstI using standard protocols, and fractionated on a 1.5% agarose gel according to size in 40 mM Tris · acetate (pH 7.8), 1 mM EDTA containing 0.5 µg/ml EtBr.

The hybrid plasmids having the desired restriction enzyme pattern are selected. The result is summarized in figures 29 and 30. Plasmid DNA, containing the insert derived from pBR322/HLyclFN-8'$_1$ or pBR322/HLyclFN-5$_1$ or pBR322/HLyclFN-1'b are denoted CG-pBR322/HLyclFN(α-3)-252 and CG-pBR322/HLyclFN(α-2)-261 and CG-pBR322/HLyclFN(α-1)-258, respectively.

In order to further confirm the structure at the junction point between the linker and the start of the coding sequence of the IFN cDNAs, the nucleotide sequence is determined at this area. In particular, 2 µg of the isolated plasmid DNA CG-pBR322/HLyclFN(α-1)-258 is digested with EcoRI, 5°-terminally labeled and cleaved with PstI. The DNA fragments are fractionated on a 6% polyacrylamid gel and the EcoRI*-PstI (9.4 bp) DNA fragment is extracted as described above. The DNA fragment is subjected to sequence analysis according to Maxam and Gilbert (15).

The structure at the junction point between the linker and the start of the coding sequence of the IFN cDNAs in plasmids CG-pBR322/HLyclFN(α-2)-261 and CG-pBR322/HLyclFN(α-3)-252 is confirmed analogously.

In the plasmids CG-pBR322/HLyclFN(α-1)-258, (α-2)-261 and (α-3)-252 the IFN coding sequences are preceded by the following nucleotide segment containing an EcoRI restriction site.

$EcoRI$ $Sau3A$

-NGAATTCTATGTGTGATC...

-NCTTAAGATACACACTAG...

——————→

IFN gene

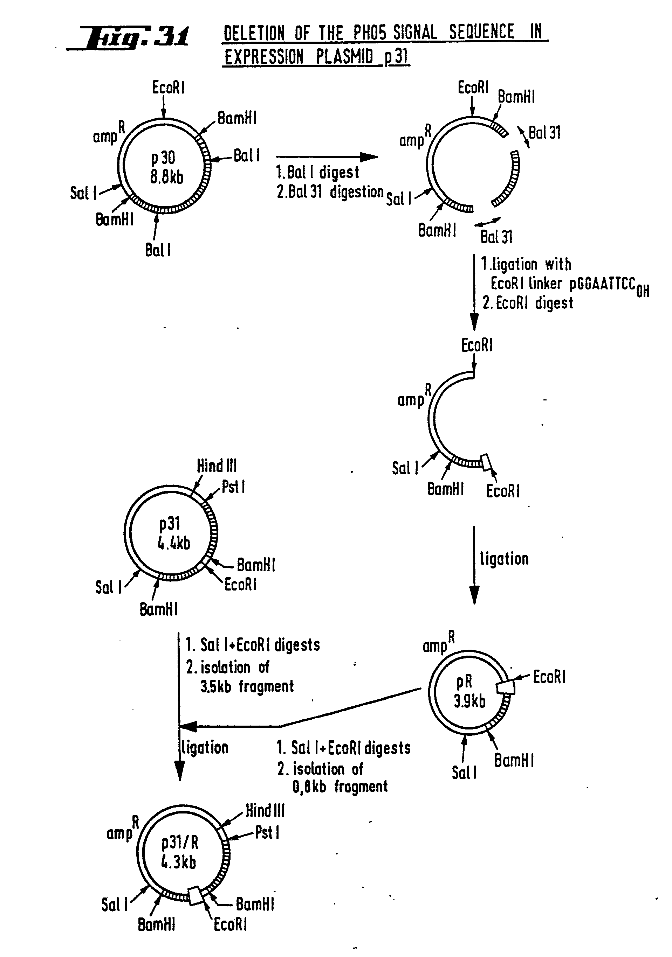

Example 24: Deletion of the PHO5 signal sequence in the expression plasmid p32 (see figure 31)

Expression plasmid 31 contains the PHO5 promoter sequence including the mRNA start sites, the translation start codon ATG of acid phosphatase and additional 40 nucleotides coding for part of the acid phosphatase signal sequence. In this construction, the nucleotides for the signal sequence and the ATG are eliminated by Bal31 digestion. EcoRI linkers are introduced to allow joining of the PHO5 promoter to appropriate mature coding sequences (e.g. interferon genes).

a) Bal31 digestion of Ball cleaved plasmid p30

20 µg of p30 DNA (see Example 4B) are digested with restriction endonuclease Ball, resulting in 2 fragments of 3.7 and 5.1 kb. After extraction with phenol/chloroform, the DNA is precipitated with ethanol. The DNA is resuspended in 10 mM Tris pH 8.0 at a concentration of 0.5 µg/ml. 9 µg of Ball cleaved p30 DNA are digested with 2U of exonuclease Bal31 (BRL) in 100 µl of 20 mM Tris pH 8.0, 100 mM NaCl, 12 mM MgCl$_2$, 12 mM CaCl$_2$ and 1 mM EDTA. Aliquots of 2 µg DNA each are withdrawn after 15 sec., 30 sec. 45 sec. and 60 sec. of incubation at 30°C and are immediately mixed with 50 µl phenol and 60 µl TNE. After extraction with phenol/chloroform and ethanol precipitation, the DNA is resuspended in 10 mM Tris pH 8.0 at a concentration of 100 µg/ml. To analyse the extent of exonucleolytic cleavage by Bal31 0.5 µg of DNA from each time point are digested with endonuclease BamHI and analysed on a 1.5% agarose gel in Tris-borate buffer pH 8.3. On the average 70 bp are removed from each end of the fragment after 45 sec. of Bal31 digestion. For further experiments DNA from the 45 second time point is used.

b) Addition of EcoRI linkers to the Bal31 treated DNA

Two $A_{260}$ units of EcoRI linkers (5'-GGAATTCC-3', BRL) are resuspended in 250 µl of 10 mM Tris pH 8, 1 mM EDTA. Two µg of EcoRI linkers are kinased in 75 µl of 60 mM Tris pH 7.5, 10 mM $MgCl_2$, 15 mM DDT, 10 µM ATP and 33 U of T4 polynucleotide kinase (Boehringer). After 1 h at 37°C the mixture is allowed to cool to room temperature and is then stored at −20°C.

The annealed, double stranded EcoRI linkers are ligated with their blunt ends to the Bal31 treated DNA fragments. Half a microgram of Bal31 treated DNA (see Example 24a) is incubated for 16 hours at room temperature with a 50 fold excess of kinased EcoRI linkers in 20 µl of 60 mM Tris pH 7.5, 10 mM $MgCl_2$, 10 mM DTT, 4 mM ATP and 600 U of T4 DNA ligase (Biolabs). After inactivation of the T4 DNA ligase (10 min at 65°C) the excess of EcoRI linkers is cleaved by 50 U of EcoRI (Boehringer) in a volume of 50 µl. The DNA is extracted with phenol/chloroform, precipitated by ethanol and resuspended in 10 mM Tris, 1 mM EDTA.

Restriction endonuclease EcoRI not only cleaves the terminally added EcoRI linkers of both BalI fragments (3.7 kb and 5.1 kb) but also at an internal EcoRI site in the 5.1 kb fragment giving rise to a 3.9 kb and a 1.2 kb fragment. The 3.7 kb and 3.9 kb fragments are separated from the 1.2 kb fragment on a 0.8% low melting agarose gel (Sigma) in 90 mM Tris-HCl pH 8.3, 90 mM boric acid and 2.5 mM EDTA. The DNA bands are stained with ethidium bromide and visualized under long wave UV light at 366 nm. The two large DNA fragments of 3.7 kb and 3.9 kb and not separated. They are cut out of the gel in a single gel block and are extracted as described in Example 4a.

The linear fragments terminating in EcoRI sticky ends are circularized by ligation. About 0.25 µg of fragments are ligated in 100 µl of 60 mM Tris pH 7.5, 10 mM $MgCl_2$, 10 mM DTT, 1 mM ATP and 600 U T4 DNA ligase for 4 hours at 15°C.

Ten µl aliquots of the ligation mixture are added to 100 µl of calcium treated, transformation competent *E. coli* HB101 cells (see Example 4a). 35 transformed, amp[R] colonies are grown individually in LB medium containing 100 µg/ml of ampicillin. Plasmid DNA is prepared according to the method of Holmes *et al.* (50) and is analysed by EcoRI/BamHI double digestion.

c) Nucleotide sequence analysis to determine the position of the EcoRI linker addition

Most of the 35 clones will differ from each other in the position of EcoRI linker addition in the *PHO5* promoter region depending on the degree of Bal31 digestion of the individual DNA molecules. For the nucleotide sequence analysis plasmid DNA is digested with EcoRI. After extraction with phenol/chloroform the restricted DNA is precipitated with ethanol. The DNA is dephosphorylated and 5'-terminally labeled as described in Example 10Ed. The labeled DNA fragments are cleaved with a second restriction endonuclease, BamHI. The products are separated on a 0.8% low melting agarose gel. The 0.5—0.6 kb 5'-labeled EcoRI-BamHI fragment is isolated from low melting agarose as described in Example 4a. For the determination of the nucleotide sequence adjacent to the EcoRI linker the different DNA fragments are chemically degraded and the products are separated by polyacrylamide gel electrophoresis as described by Maxam and Gilbert (15).

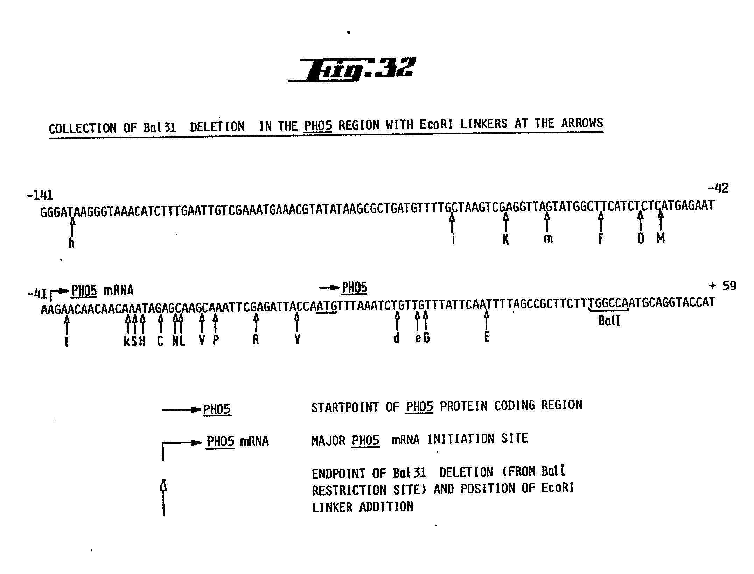

The different clones and the position of the corresponding last nucleotide of the *PHO5* sequence (then followed by an EcoRI linker) is listed in Tab. 4 (see also figure 32).

TABLE 4

| Clone | Position of last nucleotide of the *PHO5* sequence |
|---|---|
| pE | +25 |
| pG | +16 |
| pe | +15 |
| pd | +12 |
| pY | −4 |
| pR | −10 |
| pP | −16 |
| pV | −18 |
| pL | −21 |
| pN | −22 |
| pC | −24 |
| pH | −27 |
| pS | −28 |
| pk | −29 |
| pl | −38 |
| pM | −50 |
| pO | −53 |
| pF | −59 |
| pm | −67 |
| pK | −73 |
| pi | −81 |
| ph | −137 |

d) Isolation of a 0.53 kb BamHI-EcoRI fragment containing the *PHO5*/R promoter:

Plasmid pR contains the *PHO5*/R promoter on a 534 bp BamHI-EcoRI fragment. According to the numbering in fig. 3a, the fragment covers *PHO5* promoter sequences from nucleotide—541 (BamHI site) to nucleotide—10. An EcoRI linker, ligated to nucleotide—10 (see Example 24b) contributes two G-residues upon EcoRI cleavage.

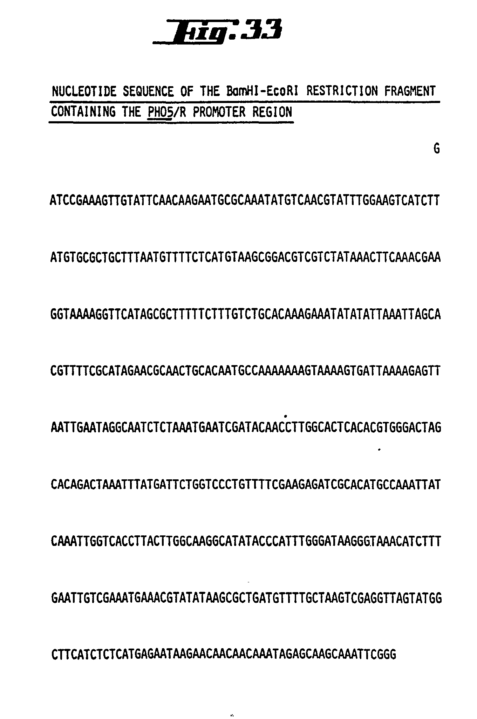

Plasmid pR is digested with restriction endonuclease BamHI and EcoRI. The 0.53 kb BamHI-EcoRI fragment is separated on a 0.8% low melting agarose gel and isolated as described in Example 4a. The nucleotide sequence is given in fig. 33.

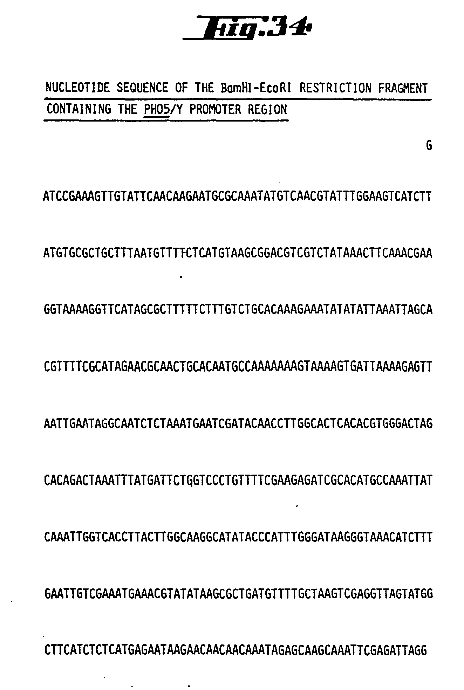

In an analogous manner plasmid pY is digested and a 0.53 kb BamHI-EcoRI fragment is isolated containing the *PHO5*/Y promoter. The nucleotide sequence is given in fig. 34.

e) Replacement of the SalI-EcoRI fragment in plasmid p31 by a SalI-EcoRI fragment of the new constructions

Five μg of plasmid p31 (cf. Example 13d) are digested with restriction endonuclease SalI. The restricted DNA is precipitated with ethanol and resuspended in 50 μl of 100 mM Tris pH 7.5, 50 mM NaCl, 5 mM MgCl$_2$. The DNA is digested with EcoRI to completion. The restriction fragments are separated on a 0.8% low melting agarose gel in Tris-borate-EDTA buffer pH 8.3. A 3.5 kb DNA fragment is isolated in a small gel block containing the DNA band.

Five μg each of clones pR and pY (cf. Table 4 and fig. 32) are digested with SalI and EcoRI in the same way as described above. The 0.8 kb DNA fragments are isolated in small blocks of low melting agarose gel.

0.67 μg of the 3.5 kb SalI-EcoRI fragment of vector p31 is ligated to 0.34 μg of the 0.8 kb SalI-EcoRI fragment of plasmid pR or pY, respectively. Appropriate gel blocks, containing the DNA fragments are mixed and melted at 65°C. The liquified gel is diluted three times. Ligation is performed in a total volume of 240 μl of 60 mM Tris pH 7.5, 10 mM MgCl$_2$, 10 mM DTT, 1 mM ATP with 750 U of T4 DNA ligase (Biolabs) overnight at 15°C. A 2 μl aliquot of each of the ligations is added to 100 μl of calcium treated, transformation competent *E. coli* HB101 cells (see Example 4a).

8 transformed, amp[R] colonies each are grown individually in LB medium containing 100 μg/ml ampicillin. Plasmid DNA is analysed by restriction analysis. The clones of each group are identical. One clone each is further used and referred to as p31/R or p31/Y, respectively (fig. 31).

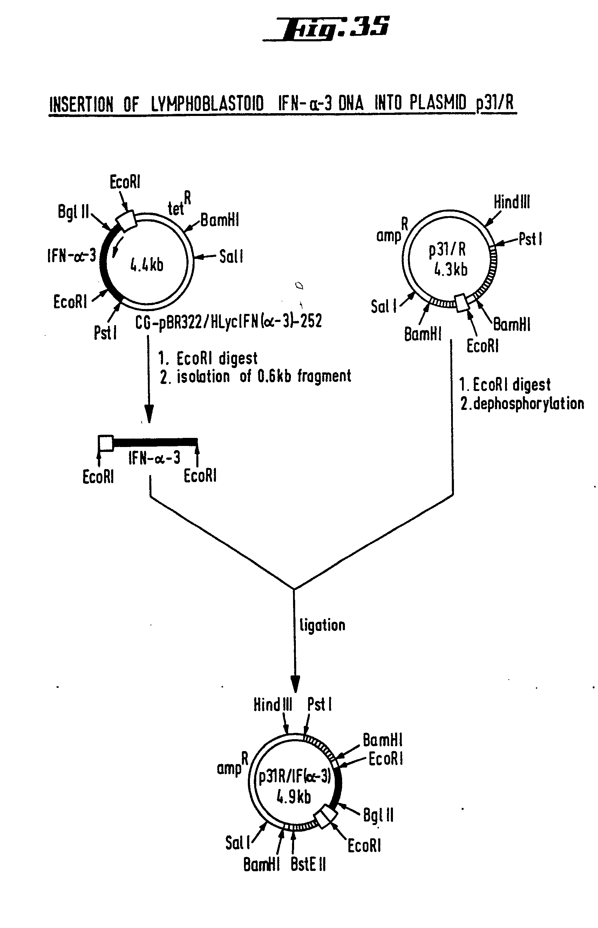

Example 25: Insertion of lymphoblastoid interferon-α-3 DNA into plasmid p31/R or p31/Y (cf. figure 35)

This construction joins the *PHO5* promoter region to the gene coding for mature interferon-α-3. Neither of the signal sequences of *PHO5* or interferon is present but there is an EcoRI linker introduced at the site of the junction.

34

Plasmid p31/R (see Example 24e) contains the *PHO5* promoter sequence which is terminated 9 nucleotides before the ATG of the acid phosphatase gene by an EcoRI linker 5'-GGAATTCC-3'. The lymphoblastoid interferon-α-3 gene in plasmid CG-pBR322/HLycIFN(α-3)-252 (see Example 23) is specifically adapted for the junction to the EcoRI linker in the *PHO5* promoter. The additional nucleotides at the 5' end of the coding sequence of mature interferon-α-3 provide an EcoRI restriction site and an ATG, necessary for the translation of the interferon gene (cf. figure 29). Essentially the same construction is also done with plasmid p31/Y (see Example 24e).

a) Preparation of EcoRI cleaved, dephosphorylated plasmid p31/R

Five µg of plasmid p31/R are digested with restriction endonuclease EcoRI (Boehringer) to completion. After extraction with phenol/chloroform, the DNA is precipitated with ethanol and resuspended in 100 µl of 50 mM Tris pH 8.0. Passage of the DNA through Chelex 100 (BioRAD), dephosphorylation by calf intestine alkaline phosphatase (Boehringer) and purification of the dephosphorylated DNA by DE52 ion exchange chromatography is as described in Example 14b. The DNA is resuspended in 10 mM Tris-HCl pH 7.5, 1 mM EDTA at a concentration of 0.2 mg/ml.

b) Isolation of a 0.6 kb EcoRI fragment of plasmid CG-pBR322/HLycIFN(α-3)-252 containing the IFN-α-3 coding sequence

Ten µg of plasmid CG-pBR322/HLycIFN(α-3)-252 are digested with restriction endonuclease EcoRI. The digest results in 2 fragments of 3.8 kb and 0.6 kb. The 0.6 kb fragment contains the interferon-α-3 coding region. The fragment is isolated on a 0.6% low melting agarose gel in Tris-borate-EDTA buffer. The gel piece containing the 0.6 kb DNA fragment is cut out of the gel and used for ligation.

c) Ligation of linearized, dephosphorylated p31/R DNA and the 0.6 kb EcoRI fragment of IFN-α-3 DNA

1.5 µg of dephosphorylated p31/R vector DNA cleaved with EcoRI is ligated to 0.19 µg of the 0.6 kb EcoRI fragment of IFN-α-3. The latter fragment is contained in a small block of low melting agarose gel which is melted at 65°C. The liquidified gel is diluted two times. Ligation is performed in a total volume of 220 µl 60 mM Tris pH 7.5, 10 mM MgCl$_2$, 10 mM DTT, 1 mM ATP with 800 U of T4 DNA ligase (Biolabs) overnight at 15°C. A 10 µl aliquot of the ligation mixture is added to 100 µl of calcium treated, transformation competent *E. coli* HB101 cells (see Example 4a).

6 transformed, amp$^R$ colonies are grown individually in LB medium containing 100 µg/ml ampicillin. Plasmid DNA is prepared according to the method of Holmes *et al.* (50) and is analysed by BglII/BstEII double digests to determine the orientation and the size of the insert. One of these clones is referred to as p31R/IF(α-3).

The same construction is done with p31/Y (See Example 24e). Plasmids from 6 transformed amp$^R$ colonies are analysed. 2 clones have the right orientation of the insert. One of them is referred to as p31Y/IF(α-3).

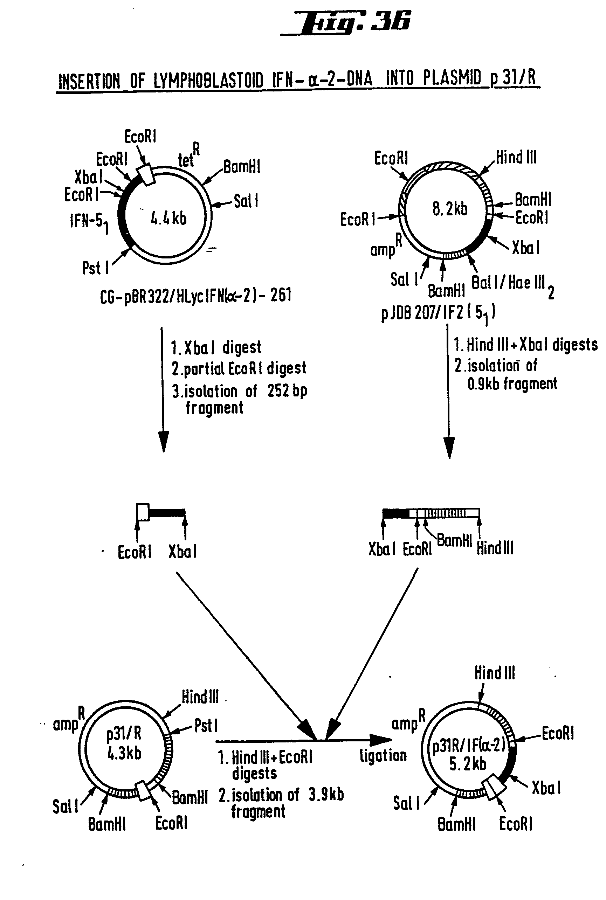

Example 26: Insertion of lymphoblastoid interferon-α-2 DNA into plasmid p31/R (see figure 36)

This construction joins the *PHO5* promoter to the mature interferon-α-2 coding region.

a) Isolation of a 3.9 kb HindIII-EcoRI fragment of vector p31/R

Ten µg of vector p31/R are digested with HindIII to completion. The buffer is adjusted with 0.1 volume of 1 M Tris pH 7.5. The hindIII-cleaved p31/R DNA is then digested with EcoRI. The 3.9 kb HindIII-EcoRI fragment is isolated from a 0.8% low melting agarose gel in a gel block cut out of the gel.

b) Isolation of a 0.9 kb XbaI-HindIII fragment of pJDB207/IF2(5$_1$)

Five µg of plasmid pJDB207/IF2(5$_1$) (cf. Example 17) are digested with HindIII to completion. The buffer is adjusted with 0.1 volume of 1 M Tris pH 7.9. The HindIII-cleaved plasmid is then digested with XbaI. The 0.9 kb XbaI-HindIII fragment contains part of the interferon-α-2 coding sequence and the downstream *PHO5* transcription termination signals. The 0.9 kb fragment is separated on a 0.8% low melting agarose gel and cut out of the gel.

c) Isolation of a 252 bp EcoRI-XbaI fragment of plasmid CG-pBR322/HLycIFN(α-2)-261 containing part of the IFN-α-2 coding sequence

Ten µg of plasmid CG-pBR322/HLycIFN(α-2)-261 (see Example 23) are digested with XbaI in 100 µl of 6 mM Tris pH 7.9, 150 mM NaCl, 6 mM MgCl$_2$ and 6 mM mercaptoethanol. After complete digestion with XbaI the linearized plasmid DNA is partially digested with 3 U of EcoRI (Boehringer). After 20 min at 37°C the digestion is stopped by freezing at −70°C. The DNA fragments are analysed on a 1.5% agarose gel in Tris-borate-EDTA buffer pH 8.3. The 252 bp EcoRI-XbaI fragment contains the 5' part of the mature interferon-α-2 coding sequence (up to the XbaI site) with the specific linker for the junction with the *PHO5* promoter. The 252 bp fragment is isolated in a small gel block from a 0.8% low melting agarose gel.

d) Ligation of DNA fragments

Three DNA fragments described in Examples 26 a-c, having appropriate sticky ends are ligated in one reaction:

0.67 µg of the 3.9 kb HindIII-EcoRI fragment of vector p31/R, 0.16 µg of the 0.9 kb XbaI-HindIII fragment of pJDB207/IF2($5_1$) and about 70 ng of the 250 bp EcoRI-XbaI fragment of CG-pBR322/HLycIFN(α-2)-261 are ligated. All three DNA fragments are contained in small gel blocks of low melting agarose. The three pieces of agarose gel are pooled, melted at 65°C and diluted three times. The ligation is done in a total volume of 450 µl of 60 mM Tris pH 7.5, 10 mM MgCl$_2$, 10 mM DTT, 1 mM ATP with 1200 U of T4 DNA ligase at 15°C for 16 hours. A 10 µl aliquot of the ligation mixture is added to 100 µl of calcium treated, transformation competent E. coli HB101 cells (see Example 4a).

12 transformed, amp$^R$ colonies are grown individually in LB medium containing 100 µg/ml of ampicillin. Plasmid DNA is prepared according to the method of Holmes et al. (50) and is analyzed by BamHI/HindIII double digestion. All clones show an identical digestion pattern. One of them is referred to as p31R/IF(α-2).

Instead of the 0.9 kb XbaI-HindIII fragment of pJDB207/IF2($5_1$) also 0.5 kb XbaI-HindIII fragment of plasmid pJDB207/IF2($5_1$)Δ72 or pJDB207/IF2($5_1$)Δ82 (see example 22) can be used for ligation. Also, instead of the 3.9 kb HindIII-EcoRI fragment of vector p31/R the ligation is carried out with the 3.9 kb HindIII-EcoRI fragment of vector p31/Y.

Conditions for ligation of the DNA fragments and the transformation of E. coli HB101 are the same as described above.

12 transformed, amp$^R$ colonies of each ligation are grown individually in LB medium containing 100 µg/ml ampicillin. Plasmid DNA is analysed by BamHI/HindIII double digestion. The resulting clones are referred to as p31R/IF(α-2)Δ72, p31R/IF(α-2)Δ82, p31Y/IF(α-2), p31Y/IF(α-2)Δ72 and p31Y/IF(α-2)Δ82.

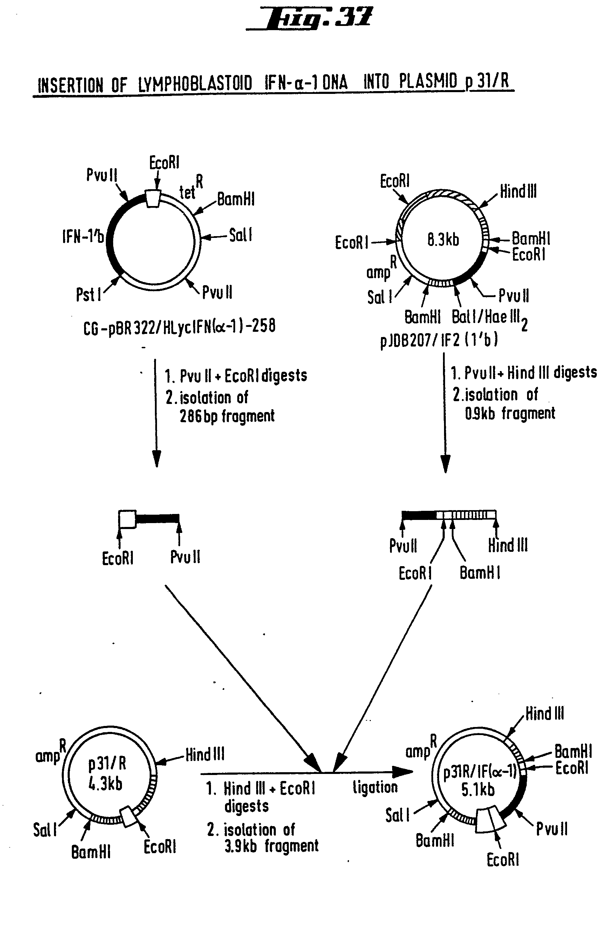

Example 27: Insertion of lymphoblastoid interferon-α-1 into plasmid p31/R (see figure 37)

a) Isolation of a 3.9 kb HindIII-EcoRI fragment of vector p31/R:

Ten µg of vector p31/R are digested with HindIII and EcoRI as described in Example 26a. The resulting 0.4 kb and 3.9 kb fragments are separated on a preparative 0.8% low melting agarose gel. The 3.9 kb HindIII-EcoRI fragment is eluted from the gel as described in Example 4a. The DNA is purified by DE52 (Whatman) ion exchange chromatography (see Example 5a), precipitated with ethanol and resuspended in 10 mM Tris pH 8.0, 1 mM EDTA at a concentration of 0.1 mg/ml.

b) Isolation of 0.9 kb PvuII-HindIII fragment of pJDB207/IF2(1'b):

Five µg of plasmid pJDB207/IF2(1'b) (cf. Example 17) are digested with PvuII and HindIII. The resulting fragments of 0.9 kb and 7.3 kb are separated on a preparative 0.8% low melting agarose gel. The 0.9 kb fragment is eluted from the gel and purified as described in Example 27a. The DNA is resuspended in 10 mM Tris pH 8.0, 1 mM EDTA at a concentration of 0.05 mg/ml.

c) Isolation of a 286 bp EcoRI-PvuII fragment of plasmid CG-pBR322/HLycIFN(α-1)-258 containing part of the IFN-α-1 coding sequence

Ten µg of plasmid CG-pBR322/HLycIFN(α-1)-258 (see Example 23) are digested with PvuII and EcoRI. A 286 bp restriction fragment is separated from a 4.2 kb fragment on a preparative 0.8% low melting agarose gel. The 286 bp EcoRI-PvuII fragment is eluted from the gel and purified as described in Example 27a. The DNA is resuspended in 10 mM Tris pH 8.0, 1 mM EDTA at a concentration of 0.03 mg/ml.

d) Ligation of DNA fragments

0.5 µg of the 3.9 kb HindIII-EcoRI fragment of vector p31/R, 0.25 µg of the 0.9 kb PvuII-HindIII fragment of pJDB207/IF2(1'b) and 0.1 µg of the 286 bp EcoRI-PvuII fragment of plasmid CG-pBR322/HLycIFN(α-1)-258 are ligated for 16 hrs at 15°C in 20 µl of 60 mM Tris pH 7.5, 10 mM MgCl$_2$, 10 mM DTT, 4 mM ATP and 600 U of DNA ligase. A 3 µl aliquot of the ligation mixture is added to 100 µl of calcium treated, transformation competent E. coli HB101 cells (see Example 4a).

12 transformed amp$^R$ colonies are grown individually in LB medium with 100 µg/ml of ampicillin. Plasmid DNA is analysed by BamHI/HindIII double digestion. One clone giving rise to a 1.4 kb BamHI fragment and a 390 bp BamHI-HindIII fragment, is further analysed and referred to as p31R/IF(α-1).

The 3.9 kb HindIII-EcoRI fragment of vector p31/R can be replaced by the HindIII-EcoRI fragment of vector p31/Y. Also, instead of the 0.9 kb PvuII-HindIII fragment of pJDB207/IF2(1'b), a 0.45 kb PvuII-HindIII fragment of pJDB207/IF2(1'b)Δ can be used for the ligation. The resulting clones are analysed as described above. The clones are referred to as p31R/IF(α-1)Δ, p31Y/IF(α-1) and p31Y/IF(α-1)Δ.

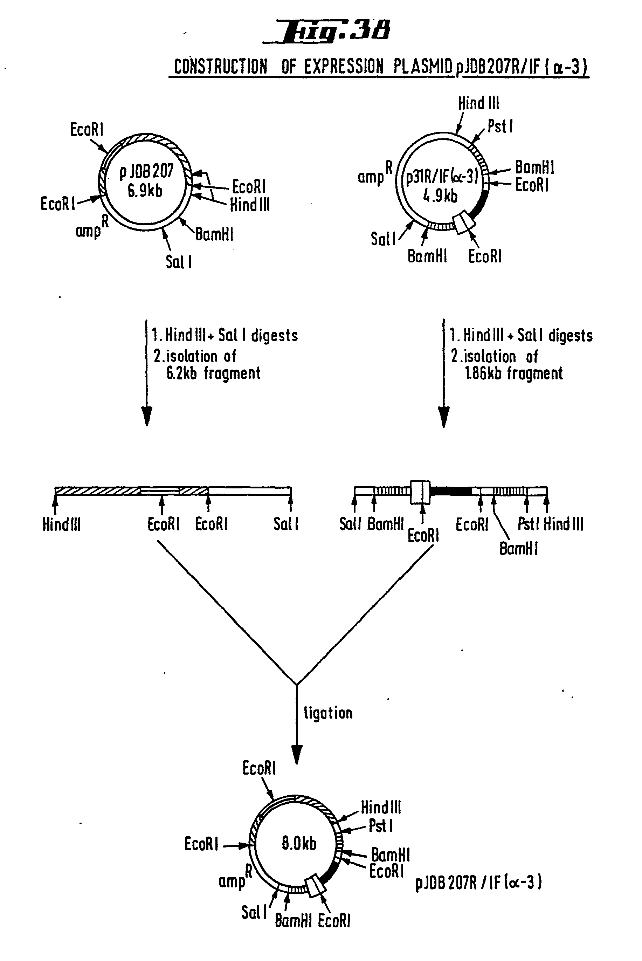

Example 28: Subcloning of gene constructions in the high copy number yeast vector pJDB207 (see fig. 38)

The constructions described in Example 25—27 contain the PHO5 promoter, different interferon coding regions and the PHO5 transcription termination signals in a tandem array, all inserted in a pBR322 derived vector. SalI-HindIII fragments containing the whole array are ligated into the 6.2 kb SalI-HindIII fragment of pJDB207 as described in Example 17.

2 µg of plasmid p31R/IF(α-3) are digested with restriction endonucleases SalI and HindIII. The restriction fragments are separated on a preparative 0.8% low melting agarose gel. The small fragment (1.8 kb in size) is cut out of the gel.

Plasmid pJDB207 is digested with restriction endonucleases SalI and HindIII and the large 6.2 kb fragment is isolated as described in Example 17.

Ligation of the DNA fragments and transformation of competent *E. coli* HB101 cells is carried out as described in Example 17. 8 amp$^R$ colonies are grown individually in LB medium containing 100 µg/ml of ampicillin. The plasmid DNA is analysed for the size of the insert by cleavages with restriction endonucleases HindIII and SalI. One clone having the correct insert is selected and referred to as pJDB207R/IF(α-3).

In an analogous manner, starting from the plasmids p31Y/IF(α-3), p31R/IF(α-2), p31R/IF(α-2)Δ72, p31R/IF(α-2)Δ82, p31Y/IF(α-2), p31Y/IF(α-2)Δ72, p31Y/IF(α-2)Δ82, p31R/IF(α-1), p31R/IF(α-1)Δ, p31Y/IF(α-1) and p31Y/IF(α-1)Δ, the following clones with the correct insert are obtained:

pJDB207Y/IF(α-3),
pJDB207R/IF(α-2),
pJDB207R/IF(α-2)Δ72,
pJDB207R/IF(α-2)Δ82,
pJDB207Y/IF(α-2),
pJDB207Y/IF(α-2)Δ72,
pJDB207Y/IF(α-2)Δ82,
pJDB207R/IF(α-1),
pJDB207R/IF(α-1)Δ,
pJDB207Y/IF(α-1), and
pJDB207Y/IF(α-1)Δ.

Example 29: Transformation of *Saccharomyces cerevisia* AH220 and induction of interferon production

Plasmids pJDB207/IF2(5₁)Δ72, pJDB207/IF2(5₁)Δ82, pJDB207/IF2(1'b)Δ, pJDB207R/IF(α-3), pJDB207Y/IF(α-3), pJDB207R/IF(α-2), pJDB207R/IF(α-2)Δ72, pJDB207R/IF(α-2)Δ82, pJDB207Y/IF(α-2), pJDB207Y/IF(α-2)Δ72, pJDB207Y/IF(α-2)Δ82, pJDB207R/IF(α-1), pJDB207R/IF(α-1)Δ, pJDB207[/IF(α-1) and pJDB207[/IF(α-1)Δ (see Examples 22 and 28, respectively) are each introduced into *Saccharomyces cerevisiae* strain AH220, (a, trp1, leu2—3, leu2—112, his3, pho5, pho3) using the transformation protocol described by Hinnen et al. (1) Transformed yeast cells are selected on yeast minimal medium plates deficient in leucine. Single transformed yeast colonies are picked and grown as described in Example 7. The different yeast colonies are referred to as

*Saccharomyces cerevisiae* AH220/pJDB207/IF2(5₁)Δ72,
*Saccharomyces cerevisiae* AH220/pJDB207/IF2(5₁)Δ82,
*Saccharomyces cerevisiae* AH220/pJDB207/IF2(1'b)Δ,
*Saccharomyces cerevisiae* AH220/pJDB207R/IF(α-3),
*Saccharomyces cerevisiae* AH220/pJDB207Y/IF(α-3),
*Saccharomyces cerevisiae* AH220/pJDB207R/IF(α-2),
*Saccharomyces cerevisiae* AH220/pJDB207R/IF(α-2)Δ72,
*Saccharomyces cerevisiae* AH220/pJDB207R/IF(α-2)Δ82,
*Saccharomyces cerevisiae* AH220/pJDB207Y/IF(α-2),
*Saccharomyces cerevisiae* AH220/pJDB207Y/IF(α-2)Δ72,
*Saccharomyces cerevisiae* AH220/pJDB207Y/IF(α-2)Δ82,
*Saccharomyces cerevisiae* AH220/pJDB207R/IF(α-1),
*Saccharomyces cerevisiae* AH220/pJDB207R/IF(α-1)Δ,
*Saccharomyces cerevisiae* AH220/pJDB207Y/IF(α-1) and
*Saccharomyces cerevisiae* AH220/pJDB207Y/IF(α-1)Δ.

Example 30: Transformation of *Saccharomyces cerevisiae* strain GRF18 and induction of interferon production

Analogous to the process described in Example 29, *Saccharomyces cerevisiae* strain GRF18 (α, his3—11, his3—15, leu2—3, leu2—112, can$^R$) is transformed with the plasmids listed in Example 29. The different yeast colonies are referred to as

*Saccharomyces cerevisiae* GRF18/pJDB207/IF2(5₁)Δ72,
*Saccharomyces cerevisiae* GRF18/pJDB207/IF2(5₁)Δ82,
*Saccharomyces cerevisiae* GRF18/pJDB207/IF2(1'b)Δ,
*Saccharomyces cerevisiae* GRF18/pJDB207R/IF(α-3),
*Saccharomyces cerevisiae* GRF18/pJDB207Y/IF(α-3),
*Saccharomyces cerevisiae* GRF18/pJDB207R/IF(α-2),
*Saccharomyces cerevisiae* GRF13/pJDB207R/IF(α-2)Δ72,
*Saccharomyces cerevisiae* GRF18/pJDB207R/IF(α-2)Δ82,
*Saccharomyces cerevisiae* GRF18/pJDB207Y/IF(α-2),
*Saccharomyces cerevisiae* GRF18/pJDB207Y/IF(α-2)Δ72,

*Saccharomyces cerevisaie* GRF18/pJDB207Y/IF(α-2)Δ82,
*Saccharomyces cerevisiae* GRF18/pJDB207R/IF(α-1),
*Saccharomyces cerevisiae* GRF18/pJDB207R/IF(α-1)Δ,
*Saccharomyces cerevisiae* GRF18/pJDB207Y/IF(α-1) and
*Saccharomyces cerevisuae* GRF18/pJDB207Y/IF(α-1)Δ.

Example 31: Preparation of yeast cell extracts and determination of the interferon titer

Cells from 50 ml of culture medium at a cell density of $1-2 \times 10^7$/ml are collected by centrifugation in a Sorvall GSA rotor for 10 min. at 3000 rpm. The cells are resuspended in 2 ml of 0.1 M $KH_2PO_4$ pH 7.4, and centrifuged at room temperature for 5 min. at 3000 rpm. The sedimented cells are resuspended in 1.2 ml ice cold lysis mix [0.1 M potassium phosphate buffer pH 7.4, 1% (v/v) Triton X-100, 0.1 mM PMSF (Merck)] and transferred to a 30 ml Corex tube. 1.6 g of glass beads (0.4 mm in diameter) are added to the cells and the suspension is shaken on a Vortex Mixer (Scientific Instruments Inc., USA) at full speed for 30 sec. and then cooled for 1 min. in an ice bath. This shaking procedure is repeated 5 to 10 times until more than 90% of the cells are broken (check under light microscope).

Cell debris and glass beads are removed from the solution by centrifugation for 10 min. at 8000 rpm at 4°C in a Sorvall HB-4 rotor. The supernatant is transferred to Eppendorf tubes, frozen in liquid nitrogen and stored at −60°C. Interferon activity is determined according to the procedure of Armstrong (32) using human CCL-23 cells and vesicular stomatitis virus (VSV) as the challenge virus. The results are summarized in Table 5.

The interferon activity in *S. cerevisiae* strains AH220 and GRF18 after transformation with a recombinant plasmid is generally identical. Table 6 shows a comparison of the interferon activities of both strains after transformation with the plasmids listed as examples.

TABLE 5:

Interferon activity in *S. cerevisiae* strain AH220 after transformation with the following recombinant plasmids:

| Plasmids | Example | Interferon activity expressed in | |
| --- | --- | --- | --- |
| | | units/ml yeast cell extract | units/l yeast cell culture |
| pJDB207/IF(8'$_1$) | 17 | $1 \cdot 10^7$ | $2 \cdot 10^8$ |
| pJDB207R/IF(α-3) | 28 | $1 \cdot 10^8$ | $2 \cdot 10^9$ |
| pJDB207Y/IF(α-3) | 28 | $1 \cdot 10^8$ | $2 \cdot 10^9$ |
| pJDB207/IF1(5$_1$) | 17 | $7 \cdot 10^5$ | $1.4 \cdot 10^7$ |
| pJDB207/IF2(5$_1$) | 17 | $5 \cdot 10^5$ | $1.0 \cdot 10^7$ |
| pJDB207/IF3(5$_1$) | 17 | $3 \cdot 10^3$ | $6.3 \cdot 10^4$ |
| pJDB207/IF2(5$_1$)Δ72 | 22 | $5 \cdot 10^5$ | $1 \cdot 10^7$ |
| pJDB207/IF2(5$_1$)Δ82 | 22 | $5 \cdot 10^5$ | $1 \cdot 10^7$ |
| pJDB207R/IF(α-2) | 28 | $1 \cdot 10^7$ | $2 \cdot 10^8$ |
| pJDB207R/IF(α-2)Δ72 | 28 | $1 \cdot 10^7$ | $2 \cdot 10^8$ |
| pJDB207R/IF(α-2)Δ82 | 28 | $1 \cdot 10^7$ | $2 \cdot 10^8$ |
| pJDB207Y/IF(α-2) | 28 | $1 \cdot 10^7$ | $2 \cdot 10^8$ |
| pJDB207Y/IF(α-2)Δ72 | 28 | $1 \cdot 10^7$ | $2 \cdot 10^8$ |
| pJDB207Y/IF(α-2)Δ82 | 28 | $1 \cdot 10^7$ | $2 \cdot 10^8$ |
| pJDB207/IF2(1'b) | 17 | $4 \cdot 10^3$ | $8 \cdot 10^4$ |
| pJDB207/IF2(1'b)Δ | 22 | $1 \cdot 10^3$ | $2 \cdot 10^4$ |
| pJDB207R/IF(α-1) | 28 | $5 \cdot 10^4$ | $1 \cdot 10^6$ |
| pJDB207R/IF(α-1)Δ | 28 | $4 \cdot 10^4$ | $8 \cdot 10^5$ |
| pJDB207Y/IF(α-1) | 28 | $5 \cdot 10^4$ | $1 \cdot 10^6$ |
| pJDB207Y/IF(α-1)Δ | 28 | $4 \cdot 10^4$ | $8 \cdot 10^5$ |

TABLE 6:

Comparison of interferon activity in *S. cerevisiae* strains AH220 and GRF18 after transformation with the following recombinant plasmids:

| Plasmids | Interferon activity (units/l yeast cell culture) | |
|---|---|---|
| | AH220 | GRF18 |
| pJDB207/IF(8'$_1$) | $2 \cdot 10^8$ | $2 \cdot 10^8$ |
| pJDB207R/IF(α-3) | $2 \cdot 10^9$ | $2 \cdot 10^9$ |
| pJDB207/IF2(5$_1$) | $1 \cdot 10^7$ | $9 \cdot 10^6$ |
| pJDB207R/IF(α-2) | $2 \cdot 10^8$ | $2 \cdot 10^8$ |
| pJDB207R/IF(α-2)Δ82 | $2 \cdot 10^8$ | $2 \cdot 10^8$ |

Example 32: Production of interferon-α-2 by a recombinant strain of the yeast *Saccharomyces cerevisiae* on a 300 l scale

*Saccharomyces cerevisiae* strain GRF18/pJDB207R/IF(α-2)Δ82 carries a plasmid which include a leucine marker allowing selective maintenance of the plasmid in the host organism a structural gene for human interferon-α-2 and the acid phosphatase *PHO5* promoter which allows expression of the IFN-α-2 gene in media with limiting amounts of inorganic phosphate.

The strain is maintained or agar slant cultures prepared with a defined medium lacking the amino acid leucine to ensure retention of the plasmid. Freshly inoculated slants are incubated for 24 hours at 30°C. The surface culture of one slant is resuspended in 3 ml pre-culture medium which is then transferred to the first shake flask pre-culture. The 500 ml flask has a single baffle and contains 100 ml pre-culture medium having hhe following composition (values in g/l): Yeast extract (Difco), 10.0; L-asparagine, 6.6; $KH_2PO_4$, 1.0; $MgSO_4 \cdot 7H_2O$, 1.0; L-histidine, 0.02 and D-glucose (monohydrate), 33.0.

The medium which has been prepared using deionised water, has a pH value of approximately 6.0. The glucose is sterilised separately. This first pre-culture is incubated for 24 hours at 30°C on an orbital shaker with 5 cm throw at a speed of 250 rev/min.

The first pre-culture flask provides the inoculum for the second pre-culture flasks. These flasks receive an inoculum level of 1% v/v. The medium and incubation conditions are identical with those for the first pre-culture. The culture broths from 36 such flasks are combined to provide a 1% v/v. inoculum for the main production fermenter.

The production fermenter has a total volume of approximately 500 l, contains 4 baffles and a single six-bladed disc turbine agitator with a diameter of 230 mm. The agitation rate is 450 rev/min, the overpressure 0.3 bar and the aeration rate is 1 vol/vol/min. The fermenter contains 300 l of a medium with the following composition (values in g/l): L-asparagine, 2.0; L-histidine, 0.02; $KH_2PO_4$, 0.03; $MgSO_4 \cdot 7H_2O$, 0.5; NaCl, 0.1; $CaCl_2 \cdot 2H_2O$, 0.1; KCl, 1.0; D-glucose (monohydrate), 20.0; vitamin solution, 5 ml/l and trace element solution, 5 ml/l. The medium is adjusted to pH 7.2 using NaOH before sterilisation. The glucose, vitamins and trace elements are sterilised separately and added to the medium. The stock solutions for vitamins and trace elements have the following compositions (in g/l); Vitamins—biotin, 0.0002; calcium-D-pantothenat, 0.04; folic acid, 0.002; nicotinic acid, 0.04; p-aminobenzoic acid, 0.02; pyridoxine hydrochloride, 0.04; riboflavin, 0.02; thiamine hydrochloride, 0.04; and inositol, 0.2 in 1 l of deionised water; trace elements—boric acid, 0.05; $CuSO_4 \cdot 5H_2O$, 0.004; KI, 0.01; $FeCl_3 \cdot 6H_2O$, 0.02; $MnSO_4 \cdot 4H_2O$, 0.04; $Na_2MoO_4 \cdot 2H_2O$, 0.02 and $ZnSO_4 \cdot 7H_2O$, 0.04 in 1 l of deionised water. The fermentation temperature is 30°C. The pH value falls to a value of about 4.0—4.2 but can be controlled if desired at an intermediate value using sodium hydroxide. After fermenting for about 18 hours the maximum yield of interferon is reached [as determined according to Armstrong (32)]. The optical density, which reaches about 2.0 units, and the acid phosphatase activity are useful indications of the progress of the fermentation. The fermentation broth may be cooled to 10°C if required prior to harvesting of the yeast cells.

Example 33: Isolation and purification of HLyIFN-α-2

a. Preparation of the polypeptide solution for the monoclonal antibody column

A total volume of 600 l of culture broth having a pH of 4.1 is cooled to 10°C. The cells are separated using an Alfa-Laval BRPX-207 de-sludger centrifuge. The clear supernatant contains no IFN-activity. Residual supernatant liquor entrained with the cells is displaced by washing with 20 l Lysis Buffer A [100 mM $KH_2PO_4$, 500 mM NaCl, 0.1% v/v Triton X-100® and 0.1 mM PMSF adjusted with KOH to pH 7.5]. The contents of the centrifuge bowl (7 l) are ejected with complete desludging and the de-sludger washed once with 5 l Lysis Buffer A. The cell mass obtained is diluted with Buffer A to 60 l and has a pH value of 7.3. The suspension is cooled to 5—10°C and passed through a DYNO®-Mill (type KD5) at a feed rate of 100 l/h. The mill is equipped with polyurethane agitator discs and 4200 ml glass beads of 0.5—0.75 mm diameter and is operated at 1625 rev/min. The ruptured cell suspension (pH~7.3) is centrifuged as described previously. The supernatant (75 l) is concentrated to 3 l by ultrafiltration. An aliquot (300 ml) of this polypeptide

EP 0 100 561 B1

solution is passed through a H1P100 Hollow filter cartridge using an Amicon DC-2 Hollow Fibre System. A further 2 l of buffer system B [30 mM Tris-HCl, 500 mM NaCl, adjusted to pH 8.5] is applied to the filter. The combined filtrate and washings (2 l) are concentrated to 100 ml by means of a H1P10 Hollow filter cartridge. The concentrate is adsorbed onto a column of DEAE-Trisacryl® M DEAE (LKB Ltd.). The column is washed and then eluted with Buffer C (200 mM NaCl, 25 mM Tris-HCl at pH 8.5). The eluate has an interferon activity of $1.4 \times 10^6$ IU/mg polypeptide when assayed according to the method of Armstrong (32). The eluate is stored at $-20°C$ prior to further purification on the monoclonal antibody column.

b) Purification of human LyIFN-α-2 on a monoclonal antibody column

The monoclonal antibody column K2 (purchased from CELLTECH U.K.) (bed volume 20 ml), is equilibrated with 20 mM Na-phosphate, 154 mM NaCl, pH 7.4 and portions of the above polypeptide solution are applied onto the column at room temperature with a flow rate of 50 ml/h. The first fractions containing the nonadsorbed polypeptides and 100 ml of PBS washings are discarded. Further non specific bound polypeptides are eluted with 110 ml of PBS containing additional 0.5 M NaCl and 0.2% Triton X 100®. The column is washed with 200 ml of 20 mM Na-phosphate, 0.3 M NaCl, pH 7.4, whereupon the specifically adsorbed polypeptides are eluted with 50 ml of Buffer D (0.1 M citric acid, 0.3 M NaCl, pH 2. This solution is adjusted to pH 6.3 with 2N NaOH and concentrated at 4°C with the aid of an immersible-CX™ molecular separator (Millipore®). The concentrate is applied onto a Sephadex G-25® fine column (2.6×34 cm, 200 ml bed volume) equilibrated with 0.025 M histidine · HCl at pH 6.3. The column is eluted with the same histidine · HCl buffer at 4°C and with a flow rate of 42 ml/h. 20 fractions are collected of each 10.5 ml. Polypeptide containing fractions are detected by their optical absorption of 280 nm. Fractions 7 and 8 contain the polypeptide with IFN activity as localised by the assay according to Armstrong (32). The active fractions containing LyIFN-α-2 are stored at $-20°C$ until further use. The IFN activity of the fractions is $1.8 \cdot 10^8$ IU/mg polypeptide (32).

By lyophilizing the above fractions from 1 ml solution 20—40 µg of polypeptide are obtained.

SDS polyacrylamide gel electrophoresis (cf. (53)) reveals a molecular weight for the obtained LyIFN-α-2 of about 18 kDaltons.

Example 34: Secretion of interferon by transformed yeast cells into the culture medium

In order to determine the effect of a N-terminal protein signal sequence on protein secretion, yeast strain S. cerevisiae GRF18/pJDB207/IF(8′₁) (containing a hybrid signal sequence, see example 17) and yeast strain S. cerevisiae GRF18/pJDB207R/IF(α-3) (without signal sequence) are grown as described in Example 28. The amount of the produced interferon present in the culture medium as well as the amount of interferon present in cell extracts (prepared as described in Example 31) is determined and the results are given in table 7.

TABLE 7:

Comparison of interferon secretion of transformed S. cerevisiae GRF18 strains into the culture medium:

| S. cerevisiae strain | Interferon activity (units/l yeast cell culture) | |
|---|---|---|
| | cell extract | culture medium |
| GRF18/pJDB207R/IF(α-3) | $1.5 \cdot 10^9$ | $<3 \cdot 10^4$ |
| GRF18/pJDB207/IF(8′₁) | $2 \cdot 10^8$ | $2 \cdot 10^7$ |

References
1. A. Hinnen et al., "Transformation of yeast", Proc. Natl. Acad. Sci. USA 75, 1929 (1978)
2. J. D. Beggs, "Transformation of yeast by a replicating hybrid plasmid", Nature 275, 104 (1978)
3. J. Hicks et al., "Properties of yeast transformation", Cold Spring Harbor, Symp. Quant. Biol. 43, 1305 (1979)
4. K. Struhl et al., "High-frequency transformation of yeast; autonomous replication of hybrid DNA molecules", Proc. Natl. Acad. Sci. USA 76, 1035 (1979)
5. R. A. Hitzeman et al., "Expression of a human gene for interferon in yeast", Nature 293, 717 (1981)
6. J. D. Beggs et al., "Abnormal expression of chromosomal rabbit β-globin gene in Saccharomyces cerevisiae", Nature 283, 835 (1980)
7. R. Axel, "The use of eukaryotic promoter sequences in the production of proteinaceous materials", PCT patent application 81/02425.
8. J. A. Carbon et al., "DNA capable of replication and stable mitotic maintenance in a host eukaryote, method of preparation thereof and eukaryotic cell containing same", European patent application 48081 (The reagents of the University of California)
9. D. T. Stinchcomb et al., "Eukaryotic autonomously replicating segment", European patent application 45573 (The board of trustees of Leland Stanford Junior University)

## EP 0 100 561 B1

10. "Plasmidvektoren, Hybridplasmide und ihre Verwendung zur Herstellung von Proteinen", German Offenlegungsschrift 2 923 297 (Institut Pasteur)

11. "Procédé de production de protéines par expression des gènes correspondants dans des microorganismes et vecteurs susceptibles d'être mis en oeuvre dans de tels procédés", French Patent application 2 458 585 (Institut Pasteur)

12. M. Aigle et al., "Nouveux plasmides hybrides et microorgnanismes les contenant", European patent application 11562 (Agence nationale de valorisation de la recherche)

13. A. Schurr et al., J. Gen. Microbiol. 65, 291 (1971) and A. Toh-e et al., Mol. Gen. Genet. 162, 139 (1978)

14. P. Mildner et al., Biochim, Biophys. Acta 429, 274 (1976)

15. A. M. Maxam et al. in "Methods in Enzymology", vol. 65, p. 499, New York 1980

16. A. Hinnen et al., "Vectors for cloning in yeast", Curr. Top. Microbiol, Immunol. 96, 101 (1982)

17. A. Hinnen et al. in "Eukaryotic Gene Regulation", vol. 14, p. 43, New York 1979

18. "Genetic Engineering" (ed. A. M. Chakrabarty), West Palm Beach 1978

19. J. Lodder, "The Yeasts", Amsterdam 1971

20. M. Grunstein et al., Proc. Natl. Acad. Sci. USA 72, 3961 (1979)

21. A. Jimenez et al., Nature 287, 869 (1980)

22. T. Staehelin et al., J. Biol. Chem. 256, 9750 (1981)

23. M. V. Olscn et al., J. Mol. Biol. 132, 387 (1979)

24. H. Meyer, FEBS Lett. 90, 341 (1978)

25. B. Hohn et al. in "Genetic Engineering", vol. 2, p. 169, New York 1980

26. B. Hohn in "Methods of Enzymology", vol. 68, p. 299, New York 1979

27. N. Mantei et al., Gene 10, 1 (1980)

28. J. D. Beggs in "Genetic Engineering", vol. 2, p. 175, new York 1981

29. M. Mandel et al., J. Mol. Biol. 53, 159 (1970)

30. J. H. Miller, "Experiments in Molecular Genetics", Cold Spring Harbor 1972

31. A. Toh-e et al., J. Bacteriol. 113, 727 (1973)

32. J. A. Armstrong, Appl. Microbiol. 21, 723 (1971)

33. J. B. Gurdon, J. Embryol. Exp. Morph 20, 401—414 (1968)

34. Barth, J. Embryol. Exp. Morph. 7, 210—222 (1959)

35. A. Colman, Cell 17, 517 (1979)

36. A. Efstratiadis et al., Cell 4, 367—378 (1975)

37. T. Maniatis et al., Cell 8, 163—182 (1976)

38. J. H. J. Hoeijmakers et al., Gene 8, 391—417 (1980)

39. A. C. Peacock et al., Biochemistry 6, 1818 (1967)

40. K. Itakura et al., J. Am. Chem. Soc. 97, 7327 (1975)

41. J. F. M. de Rooij et al., Recl. Trav. Chim. Pays-Bas 98, 537—548 (1979)

42. W. Mueller et al., J. Mol. Biol. 124, 343 (1978)

43. D. V. Goeddel et al., Nature 290, 20 (1981)

44. C. Weissmann, European patent application No. 32134 (Biogen N. V.)

45. T. Taniguchi et al., Gene 10, 11 (1980)

46. M. Streuli et al., Science 209, 1343 (1980)

47. Perlman et al., Proc. Natl. Acad. Sci. USA 79, 781 (1982)

48. A. J. Berk et al., Cell 12, 721—732 (1977)

49. J. Messing, In the 3rd Cleveland Symposium on Macromolecules: Recombinant DNA (ed. A. Walton), Elsevier, Amsterdam 1981, pp. 143—153

50. D. S. Holmes et al., Anal. Biochem. 114, 193—197 (1981)

51. N. M. Gough et al., J. Mol. Biol. 162, 43—67 (1982)

52. Pasek et al., Nature 282, 575—579 (1979).

53. U.K. Laemmli, Nature 227, 680—685 (1970).

## APPENDIX

Symbols used in figures 10-14 of the accompanying drawings have the following meanings:

□    amino acid exchange

• and ± nucleotide exchange

－－－－－    sequence not present in prior art

▼    polyadenylation sites in prior art

↓    deletion of a nucleotide

↑    insertion of a nucleotide

In the respective figures the indicated symbols are referring to the closest prior art references as mentioned in Example 10.

In the other figures of the accompanying drawings, the symbols used have the following meanigs:

⬜⬜⬜⬜    HBVs gene

═══════    pBR322 sequences

▦▦▦▦    yeast chromosomal DNA derived from PHO3, PHO5 region

▨▨▨▨    yeast 2 µ plasmid DNA

═══════    yeast chromosomal DNA derived from LEU 2 region

══╳══    deletion of a restriction site

▬▬▬▬    human lymphoblastoid interferon gene

═══════    yeast chromosomal DNA derived from TRPl region

42

## EP 0 100 561 B1

$\underline{\quad\blacktriangledown\quad}$ deletion in pBR322

$\underline{\quad\downarrow\quad}$ restriction site

$\underline{\quad\longrightarrow\quad}$ direction of transcription

$\underline{\quad\square\quad}$ linker DNA stretch

amp$^R$: ampicillin resistance gene

tet$^R$: tetracycline resistance gene

**Claims for the Contracting States: BE, CH, DE, FR, IT, LI, LU, NL, SE**

1. A DNA restriction fragment consisting essentially of the repressible yeast acid phosphatase (*PHO5*) promoter comprising the sequence

                                                         G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

and subfragments and mutants thereof which retain the promoter function.

2. A DNA fragment according to claim 1 having the sequence

43

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

and subfragments and mutants thereof which retain the promoter function.

3. A DNA fragment according to claim 1 having the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTACTATGG

CTTCATCTCTCATGAGAATAAGAACAACAACAAATAGAGCAAGCAAATTCGGG

and mutants thereof which retain the promoter function.

4. A DNA fragment according to claim 1 having the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

and mutants thereof which retain the promoter function.

5. A method for producing a DNA fragment according to anyone of claims 1 to 4, which comprises

(A) preparing the acid phosphatase *PHO5* gene by complementing acid phosphatase *PHO5* deficient yeast strains by transformation with plasmid DNA from a yeast gene library containing the wild-type copy of said gene and isolating said gene,

(B) preparing subclones of the obtained gene, and

(C) identifying the location of the promoter region of the above subclones and isolating by restriction digest DNA fragments essentially consisting of the acid phosphatase *PHO5* promoter, and for the preparation of subfragments, shortening the DNA restriction fragments obtained by digestion with a restriction endonuclease or with a suitable exonuclease.

6. A yeast hybrid vector comprising a DNA fragment consisting essentially of the repressible yeast acid phosphatase (*PHO5*) promoter according to anyone of claims 1 to 4 and a yeast or non-yeast polypeptide coding region which is controlled by said promoter.

7. A hybrid vector according to claim 6 wherein the yeast or non-yeast polypeptide coding region codes for an enzyme, a hormone, a polypeptide with immunomodulatory, anti-viral or anti-cancer properties, an antibody, a viral antigen, a vaccine or a clotting factor.

8. A hybrid vector according to claim 7 wherein the yeast or non-yeast polypeptide coding region codes for a human interferon.

9. A hybrid vector according to claim 8 wherein the yeast or non-yeast polypeptide coding region codes for a human lymphoblastoid interferon.

10. A hybrid vector according to any one of claims 6 to 9 including additional DNA sequences derived from the group consisting of a bacterial plasmid, bacteriophage λ, yeast 2 μ plasmid and/or yeast chromosomal DNA.

11. A hybrid vector according to claim 10 including yeast chromosomal DNA comprising an autonomously replicating segment and a marker gene.

12. A hybrid vector according to claim 6 comprising transcription termination signals of a yeast gene.

13. A hybrid vector according to claim 12 comprising the transcription termination signals of the *PHO5* gene.

14. A hybrid vector according to claim 6 wherein the yeast or non-yeast polypeptide coding region is preceded by a signal sequence.

15. A hybrid vector according to claim 6 wherein the yeast or non-yeast polypeptide coding region is joined to the acid phosphatase *PHO5* promoter between the major acid phosphatase *PHO5* mRNA start and the ATG of the acid phopshatase *PHO5* coding region.

16. A method for producing a hybrid vector according to anyone of claims 6 to 15 which method comprises introducing into a vector DNA a DNA fragment consisting essentially of the repressible yeast acid phosphatase (*PHO5*) promoter according to anyone of claims 1 to 4 and a yeast or non-yeast polypeptide coding region such that it is controlled by said promoter.

17. A yeast host transformed with a hybrid vector according to anyone of claims 6 to 15.

18. A transformed yeast according to claim 17 wherein the yeast is *Saccharomyces cerevisiae*.

19. A method for producing a transformed yeast according to anyone of claims 17 and 18 comprising transforming yeast with a hybrid vector according to anyone of claims 6 to 15.

20. A method for producing a yeast or non-yeast polypeptide or a naturally occurring derivative thereof, which method comprises the steps of (1) culturing transformed yeast according to anyone of claims 17 and 18 and (2) isolating and purifying the polypeptide or derivative thereof.

21. A method for producing a yeast or non-yeast polypeptide according to claim 20, characterized in

## EP 0 100 561 B1

that the transformed yeast is cultured in a nutrient medium containing assimilable sources of carbon and nitrogen and inorganic salts.

22. A method for producing a yeast or non-yeast polypeptide according to claim 20, wherein the nutrient medium contains a low concentration of inorganic phosphate.

23. A method according to claim 20, wherein the polypeptide is an enzyme, a hormone, a polypeptide with immunomodulatory, anti-viral or anti-cancer properties, an antibody, a viral antigen, a vaccine or a clotting factor.

24. A method according to claim 20, wherein the polypeptide is a human interferon.

25. A method according to claim 20, wherein the human interferon is a human lymphoblastoid interferon.

26. A method according to claim 20, wherein the human lymphoblastoid interferon is IFN-α-1.

27. A method according to claim 20, wherein the human lymphoblastoid interferon is IFN-α-2.

28. A method according to claim 20, wherein the human lymphoblastoid interferon is IFN-α-3.

29. A hybrid vector comprising a DNA fragment consisting essentially of the repressible yeast acid phosphatase (PHO5) promoter according to anyone of claims 1 to 4, and additional DNA sequences derived from the group consisting of a bacterial plasmid, bacteriophage λ, yeast 2 μ plasmid and/or yeast chromosomal DNA.

30. A hybrid vector according to claim 29 in which the additional DNA sequences comprise a replication origin of E. coli and a selective genetic marker for E. coli.

31. A hybrid vector according to claim 29 in which the additional DNA sequences comprise a yeast replication origin and a selective genetic marker for yeast.

32. A hybrid vector according to claim 30 which additionally comprises a yeast replication origin and a selective genetic marker for yeast.

## Claims for the Contracting State: AT

1. A method for producing a DNA restriction fragment consisting essentially of the repressible yeast acid phosphatase (PHO5) promoter comprising the sequence

G

ATCCGAAAGTTGTATTCAACAAGAATGCGCAAATATGTCAACGTATTTGGAAGTCATCTT

ATGTGCGCTGCTTTAATGTTTTTCTCATGTAAGCGGACGTCGTCTATAAACTTCAAACGAA

GGTAAAAGGTTCATAGCGCTTTTTTCTTTGTCTGCACAAAGAAATATATATTAAATTAGCA

CGTTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

and subfragments and mutants thereof which retain the promoter function, which method comprises

(A) preparing the acid phosphatase PHO5 gene by complementing acid phosphatase PHO5 deficient yeast strains by transformation with plasmid DNA from a yeast gene library containing the wild-type copy of said gene and isolating said gene,

(B) preparing subclones of the obtained gene, and

(C) identifying the location of the promoter region of the above subclones and isolating by restriction digest DNA fragments essentially consisting of the acid phosphatase PHO5 promoter, and for the preparation of subfragments, shortening the DNA restriction fragments obtained by digestion with a restriction endonuclease or with a suitable exonuclease.

2. A method according to claim 1 characterized in that the DNA fragment has the sequence

46

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

or a subfragment or mutant thereof which retain the promoter function.
3. A method according to claim 1 characterized in that the DNA fragment has the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGGG

or a mutant thereof which retain the promoter function.
4. A method according to claim 1 characterized in that the DNA fragment has the sequence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

or a mutant which retain the promoter function.

5. A method for producing a yeast hybrid vector comprising a DNA fragment consisting essentially of the repressible yeast acid phosphatase promoter *PHO5* obtainable according to anyone of claims 1 to 4 and a yeast or a non-yeast polypeptide coding region which is controlled by said promoter, which method comprises introducing into a vector DNA said DNA fragment and a yeast or non-yeast polypeptide coding region such that it is controlled by said promoter.

6. A method according to claim 5 wherein the yeast or non-yeast polypeptide coding region codes for an enzyme, a hormone, a polypeptide with immunomodulatory, anti-viral or anticancer properties, an antibody, a viral antigen, a vaccine or a clotting factor.

7. A method according to claim 5 wherein the yeast or non-yeast polypeptide coding region codes for a human interferon.

8. A method according to claim 5 wherein the yeast or non-yeast polypeptide coding region codes for a human lymphoblastoid interferon.

9. A method according to claim 5 characterized in that the hybrid vector includes additional DNA sequences derived from the group consisting of a bacterial plasmid, bacteriophage λ, yeast 2 μ plasmid and/or yeast chromosomal DNA.

10. A method according to claim 5 characterized in that the hybrid vector includes yeast chromosomal DNA containing an autonomously replicating segment and a marker gene.

11. A method according to claim 5 characterized in that the hybrid vector comprises transcription termination signals of a yeast gene.

12. A method according to claim 5 characterized in that the hybrid vector comprises transcription termination signals of the *PHO5* gene.

13. A method according to claim 5 characterized in that the yeast or non-yeast polypeptide coding region is preceded by a signal sequence.

14. A method according to claim 5 characterized in that the yeast or non-yeast polypeptide coding region is joined to the acid phosphatase *PHO5* promoter region between the major acid phosphatase *PHO5* mRNA start and the ATG of the acid phosphatase *PHO5* coding region.

15. A method for producing transformed yeast containing a hybrid vector obtainable according to anyone of claims 5 to 14, which method comprises transforming yeast with said hybrid vector.

16. A method according to claim 15 wherein the yeast is *Saccharomyces cerevisiae*.

17. A method for producing a yeast or non-yeast polypeptide or a naturally occurring derivative thereof, which method comprises the steps of (1) culturing transformed yeast obtainable by the method according to anyone of claims 15 and 16 and (2) isolating and purifying the polypeptide or derivative thereof.

18. A method for producing a yeast or non-yeast polypeptide according to claim 17, characterized in that the transformed yeast is cultured in a nutrient medium containing assimilable sources of carbon and nitrogen and inorganic salts.

19. A method for producing a yeast or non-yeast polypeptide according to claim 17, wherein the nutrient medium contains low concentration of inorganic phosphate.

20. A method according to claim 17, wherein the polypeptide is an enzyme, a hormone, a polypeptide with immunomodulatory, anti-viral or anti-cancer properties, an antibody, a viral antigen, a vaccine or a clotting factor.

21. A method according to claim 17, wherein the polypeptide is a human interferon.

22. A method according to claim 17, wherein the human interferon is a human lymphoblastoid interferon.

48

23. A method according to claim 17, wherein the human lymphoblastoid interferon is IFN-α-1.

24. A method according to claim 17, wherein the human lymphoblastoid interferon is IFN-α-2.

25. A method according to claim 17, wherein the human lymphoblastoid interferon is IFNα-3.

26. A method for producing a hybrid vector comprising a DNA fragment consisting essentially of the repressible yeast acid phosphatase promoter (PHO5) obtainable according to anyone of claims 1 to 4 and additional DNA sequences derived from the group consisting of a bacterial plasmid, bacteriophage λ, yeast 2 μ plasmid and/or yeast chromosomal DNA which method comprises introducing said DNA fragment into a vector DNA containing said additional DNA sequences.

27. A method according to claim 26 characterized in that the additional DNA sequences comprise a replication origin of E. coli and a selective genetic marker for E. coli.

28. A method according to claim 26 characterized in that the additional DNA sequences comprise a yeast replication origin and a selective genetic marker for yeast.

29. A method according to claim 27 characterized in that the hybrid vector additionally comprises a yeast replication origin and a selective genetic marker for yeast.

**Patentansprüche für die Vertragsstaaten: BE, CH, DE, FR, IT, LU, NL, SE, LI**

1. DNA-Restriktionsfragment, das im wesentlichen aus dem repressiblen (PHO5)-Promoter der sauren Hefephosphatase mit der folgenden Sequenz besteht:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

und Subfragmente und seine Mutanten, die die Promotorfunktion beibehalten.

2. DNA-Fragment nach Anspruch 1 mit der folgenden Sequenz:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

und Subfragmente und seine Mutanten, die die Promotorfunktion beibehalten.
3. DNA-Fragment nach Anspruch 1 mit der folgenden Sequenz:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACAGGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTACTATGG

CTTCATCTCTCATGAGAATAAGAACAACAACAAATAGAGCAAGCAAATTCGGG.

und seine Mutanten, die die Promotorfunktion beibehalten.
4. DNA-Fragment nach Anspruch 1 mit der folgenden Sequenz:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

und seine Mutanten, die die Promotorfunktion beibehalten.

5. Verfahren zur Herstellung eines DNA-Fragments nach einem der Ansprüche 1 bis 4, bei dem man

(A) das *PHO5*-Gen der sauren Phosphatase herstellt, indem man Hefestämme komplementiert, denen die saure Phosphatase *PHO5* fehlt, indem man Plasmid-DNA aus einer Hefegenbank transformiert, die die Wildtypkopie des Gens enthält, und das Gen isoliert,

(B) Subklone des erhaltenen Gens herstellt und

(C) die Lage des Promotorbereichs der genannten Subklone identifiziert und DNA-Fragmente durch Restriktionsverdau isoliert, die im wesentlichen aus dem *PHO5*-Promotor der saueren Phosphatase bestehen, und

für die Herstellung von Subfragmenten die RNA-Restriktionsfragmente, die durch Verdau mit einer Restriktionsendonuklease oder mit einer geeigneten Exonuklease erhalten worden sind, kürzt.

6. Hefehybridvektor mit einem DNA-Fragment, das im wesentlichen aus dem repressiblen *PHO5*-Promotor der saueren Hefephosphatase nach einem der Ansprüche 1 bis 4 und einem Bereich besteht, der ein Hefe- oder nicht-Hefe Polypeptid kodiert, wobei der Bereich durch den Promotor kontrolliert wird.

7. Hybridvektor nach Anspruch 6, bei dem der Bereich, der das Hefe- oder nicht-Hefepolypeptid kodiert, ein Enzym, ein Hormon, ein Polypeptid mit immunomodulatorischen oder antiviralen Eigenschaften oder Antikrebsiegenschaften, einen Antikörper, ein Virusantigen, einen Impfstoff oder einen Gerinnungsfaktor kodiert.

8. Hybridvektor nach Anspruch 7, bei dem der Bereich, der das Hefe- oder nicht-Hefepolypeptid kodiert, Humaninterferon kodiert.

9. Hybridvektor nach Anspruch 8, bei dem der Bereich, der das Hefe- oder nicht-Hefepolypeptid kodiert, Humanlymphoblastoidinterferon kodiert.

10. Hybridvektor nach einem der Ansprüche 6 bis 9 unter Einschluß zusätzlicher DNA-Sequenzen, die sich aus der durch bakterielle Plasmide, Bakteriophagen-lambda, Hefe-2-µ-Plasmide und/oder chromosomale Hefe-DNA gebildeten Gruppe ableiten.

11. Hybridvektor nach Anspruch 10 unter Einschluß chromosomaler DNA mit einem autonom replizierenden Segment und einem Markergen.

12. Hybridvektor nach Anspruch 6 mit Transkriptionsterminationssignalen eines Hefegens.

13. Hybridvektor nach Anspruch 12 mit dem Transkriptionsterminationssignal des *PHO5*-Gens.

14. Hybridvektor nach Anspruch 6, bei dem dem Bereich, der das Hefe- oder nicht-Hefepolypeptid kodiert, eine Signalsequenz vorausgeht.

15. Hybridvektor nach Anspruch 6, bei dem der Bereich, der das Hefe- oder nicht-Hefepolypeptid kodiert, mit dem *PHO5*-Promotor der sauren Phosphatase zwischen dem Haupt-mRNA-Start der sauren Phosphatase *PHO5* und dem ATG fer Kodierregion der sauren Phosphatase *PHO5* verbunden ist.

16. Verfahren zur Herstellung eines Hybridvektors nach einem der Ansprüche 6 bis 15, bei dem man in eine Vektor-DNA ein DNA-Fragment, das im wesentlichen aus dem repressiblen *PHO5*-Promotor der sauren Hefephosphatase gemäß einem der Ansprüche 1 bis 4 und einem ein Hefe- oder Nicht-Hefepolypeptid kodierenden Bereich besteht, derart einführt, daß es durch den Promotor kontrolliert wird.

17. Wirtshefe, die mit einem Hybridvektor nach einem der Ansprüche 6 bis ·15 transformiert ist.

18. Transformierte Hefe nach Anspruch 17, wobei die Hefe *Saccharomyces cerevisiae* ist.

19. Verfahren zur Herstellung einer transformierten Hefe nach einem der Ansprüche 17 bis 18, bei dem man Hefe mit einem Hybridvektor nach einem der Ansprüche 6 bis 15 transformiert.

20. Verfahren zur Herstellung eines Hefe- oder Nicht-Hefepolypeptids oder eines seiner natürlich auftretenden Derivate, wobei das Verfahren die folgenden Stufen umfaßt:

(1) Kultivieren von transformierter Hefe nach einem der Ansprüche 17 bis 18 und

(2) Isolieren und Reinigen des Polypeptids oder seines Derivats.

21. Verfahren zur Herstellung eines Hefe- oder Nicht-Hefepolypeptids nach Anspruch 20, dadurch gekennzeichnet, daß man die transformierte Hefe in einem Nährmedium kultiviert, das assimilierbare Kohlenstoff- und Stickstoffquellen und anorganische Salze enthält.

22. Verfahren zur Herstellung eines Hefe- oder Nicht-Hefepolypeptids nach Anspruch 20, bei dem das Nährmedium eine niedrige Konzentration an anorganischen Phosphat aufweist.

23. Verfahren nach Anspruch 20, bei dem es sich bei dem Polypeptid um ein Enzym, ein Hormon, ein Polypeptid mit immunomodulatorischen oder antiviralen Eigenschaften oder Antikrebseigenschaften, einen Antikörper, ein Virusantigen, einen Impfstoff oder Gerinnungsfaktor handelt.

24. Verfahren nach Anspruch 20, bei dem es sich bei dem Polypeptid um Humaninterferon handelt.

25. Verfahren nach Anspruch 20, bei dem es sich bei dem Humaninterferon um Humanlymphoblastoidinterferon handelt.

26. Verfahren nach Anspruch 20, bei dem es sich bei dem Humanlymphoblastoidinterferon um IFN-α-1 handelt.

27. Verfahren nach Anspruch 20, bei dem es sich bei dem Humanlymphoblastoidinterferon um IFN-α-2 handelt.

28. Verfahren nach Anspruch 20, bei dem es sich bei dem Humanlymphoblastoidinterferon um IFN-α-3 handelt.

29. Hybridvektor mit einem DNA-Fragment, das im wesentlichen aus dem repressiblen *PHO5*-Promotor der sauren Hefephosphatase nach einem der Ansprüche 1 bis 4 und zusätzlichen DNA-Sequenzen besteht, die sich aus der durch bakterielle Plasmide, Bakteriophagen-lambda, Hefe-2-μ-Plasmide und/oder chromosomale Hefe-DNA gebildeten Gruppe ableiten.

30. Hybridvektor nach Anspruch 29, bei dem die zusätzlichen DNA-Sequenzen einen Replikationsstart von *E. coli* und einen selektiven genetischen Marker für *E. coli* umfassen.

31. Hybridvektor nach Anspruch 29, bei dem die zusätzlichen DNA-Sequenzen einen Hefe-Replikationsstart und einen selektiven genetischen Marker für Hefe umfassen.

32. Hybridvektor nach Anspruch 30, der zusätzlich einen Hefe-Replikationsstart und einen selektiven genetischen Marker für Hefe umfaßt.

**Patentansprüche für den Vertragsstaat: AT**

1. Verfahren zur Herstellung eines DNA-Restriktionsfragments, das im wesentlichen aus dem repressiven (*PHO5*)-Promotor der sauren Hefephosphatase mit der folgenden Sequenz besteht:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

und von Subfragmenten und seinen Mutanten, die die Promotorfunktion beibehalten, bei dem man

(A) das *PHO5*-Gen der saueren Phosphatase herstellt, indem man Hefestämme komplementiert, denen die saure Phosphotase *PHO5* fehlt, indem man Plasmid-DNA aus einer Hefegenbank transformiert, die eine Wildtypkopie des Gens enthält und das Gen isoliert,

(B) Subklone des erhaltenen Gens herstellt und

(C) die Lage des Promotorbereichs der genannten Subklone identifiziert und DNA-Fragmente durch Restriktionsverdau isoliert, die im wesentlichen aus dem *PHO5*-Promotor der sauren Phosphatase bestehen und für die Herstellung von Subfragmenten die DNA-Restriktionsfragmente, die durch Verdau mit Restriktionsendonuklease oder mit einer geeigneten Exonuklease erhalten worden sind, kürzt.

2. Verfahren nach Anspruch 1, dadurch gekennzeichnet, daß das DNA-Fragment die folgende Sequenz besitzt:

```
                                                                                 G
ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG
```

oder ein Subfragment oder seine Mutante, die die Promotorfunktion beibehält.

3. Verfahren nach Anspruch 1, dadurch gekennzeichnet, daß das DNA-Fragment die folgende Sequenz besitzt:

```
                                                                                 G
ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTACTATGG

CTTCATCTCTCATGAGAATAAGAACAACAACAAATAGAGCAAGCAAATTCGGG
```

oder seine Mutante, die die Promotorfunktion beibehält.

4. Verfahren nach Anspruch 1, dadurch gekennzeichnet, daß das DNA-Fragment die folgende Sequenz besitzt:

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

oder seine -Mutante, die die Promotorfunktion beibehält.

5. Verfahren zur Herstellung eines Hefe-Hybridvektors mit einem DNA-Fragment, das im wesentlichen aus dem repressiblen (*PHO5*)-Promotor der sauren Hefephosphatase, der nach einem der Ansprüche 1 bis 4 erhältlich ist und einem ein Hefe- oder Nicht-Hefepolypeptid kodierenden Bereich besteht, der durch den Promotor kontrolliert wird, wobei man bei diesem Verfahren in eine Vektor-DNA das DNA-Fragment und einen ein Hefe- oder Nicht-Hefepolypeptid kodierenden Bereich derart einführt, daß er durch den Promotor kontrolliert wird.

6. Verfahren nach Anspruch 5, bei dem der Bereich, der das Hefe-oder Nicht-Hefepolypeptid kodiert, ein Enzym, ein Hormon, ein Polypeptid mit immunomodulatorischen oder antiviralen Eigenschaften oder Antikrebseigenschaften, einen Antikörper, ein Virusantigen, einen Impfstoff oder einen Gerinnungsfaktor kodiert.

7. Verfahren nach Anspruch 5, bei dem der Bereich, der das Hefe-oder Nicht-Hefepolypeptid kodiert, Humaninterferon kodiert.

8. Verfahren nach Anspruch 5, bei dem der Bereich, der das Hefe-oder Nicht-Hefepolypeptid kodiert, Humanlymphoblastoidinterferon kodiert.

9. Verfahren nach Anspruch 5, dadurch gekennzeichnet, daß der Hybridvektor zusätzliche DNA-Sequenzen umfaßt, die sich von der durch bakterielle Plasmide, Bakteriophagen-lambda, Hefe-2-μ-Plasmide und/oder chromosomale DNA gebildeten Gruppe ableiten.

10. Verfahren nach Anspruch 5, dadurch gekennzeichnet, daß der Hybridvektor chromosomale Hefe-DNA umfaßt, die ein autonom replizierendes Segment und ein Markergen enthält.

11. Verfahren nach Anspruch 5, dadurch gekennzeichnet, daß der Hybridvektor Transkriptionsterminationssignale eines Hefegens umfaßt.

12. Verfahren nach Anspruch 5, dadurch gekennzeichnet, daß der Hybridvektor Transkriptions-terminationssignale des *PHO5*-Gens umfaßt.

13. Verfahren nach Anspruch 5, dadurch gekennzeichnet, daß dem Bereich, der das Hefe- oder Nicht-Hefepolypeptid kodiert, eine Signalsequenz vorausgeht.

14. Verfahren nach Anspruch 5, dadurch gekennzeichnet, daß der Bereich, der das Hefe- oder Nicht-Hefepolypeptid kodiert, mit dem Bereich des *PHO5*-Promotors der sauren Phosphatase zwischen dem Hauptstart der *PHO5*-mRNA der sauren Phosphatase und dem ATG des Bereichs verbunden ist, der die saure Phosphatase *PHO5* kodiert.

15. Verfahren zur Herstellung transformierter Hefe mit einem Gehalt an einem Hybridvektor, der nach einem der Ansprüche 5 bis 14 erhältlich ist, wobei das Verfahren das Transformieren von Hefe mit dem Hybridvektor umfaßt.

16. Verfahren nach Anspruch 15, bei dem die Hefe *Saccharomyces cerevisiae* ist.

17. Verfahren zur Herstellung eines Hefe- oder Nicht-Hefepolypeptids oder eines seiner natürlich auftretenden Derivate, wobei das Verfahren die folgenden Stufen umfaßt:

(1) Kultivieren von transformierter Hefe, die nach dem Verfahren nach einem der Ansprüche 15 bis 16 erhältlich ist und

(2) Isolieren und Reinigen des Polypeptids oder seines Derivats.

18. Verfahren zur Herstellung eines Hefe- oder Nicht-Hefepolypeptids nach Anspruch 17, dadurch gekennzeichnet, daß man die transformierte Hefe in einem Nährmedium kultiviert, das assimilierbarer Kohlenstoff- und Stickstoffquellen und anorganische Salze umfaßt.

19. Verfahren zur Herstellung eines Hefe- oder Nicht-Hefepolypeptids nach Anspruch 17, bei dem das Nährmedium eine niedrige Konzentration an anorganischem Phosphat aufweist.

20. Verfahren nach Anspruch 17, bei dem es sich bei dem Polypeptid um ein Enzym, ein Hormon, ein

# EP 0 100 561 B1

Polypeptid mit immunomodulatorischen oder antiviralen Eigenschaften oder Antikrebseigenschaften, einen Antikörper, ein Virusantigen, einen Impfstoff oder einen Gerinnungsfaktor handelt.

21 Verfahren nach Anspruch 17, bei dem es sich bei dem Polypeptid um Humaninterferon handelt.

22. Verfahren nach Anspruch 17, bei dem es sich bei dem Humaninterferon um Humanlymphoblastoidinterferon handelt.

23. Verfahren nach Anspruch 17, bei dem es sich bei dem Humanlymphoblastoidinterferon um IFN-α-1 handelt.

24. Verfahren nach Anspruch 17, bei dem es sich bei dem Humanlymphoblastoidinterferon um IFN-α-2 handelt.

25. Verfahren nach Anspruch 17, bei dem es sich bei dem Humanlymphoblastoidinterferon um IFN-α-3 handelt.

26. Verfahren zur Herstellung eines Hybridvektors, der ein DNA-Fragment umfaßt, das im wesentlichen aus dem repressiven (PHO5)-Promotor der saurer Hefephosphatase, der nach einem der Ansprüche 1 bis 4 erhältlich ist, und zusätzlichen DNA-Sequenzen besteht, die sich aus der durch bakterielle Plasmide, Bakteriophagen-lambda, Hefe-2-μ-Plasmide und/oder chromosomale Hefe-DNA gebildeten Gruppe ableiten, wobei man bei dem Verfahren das DNA-Fragment in eine Vektor-DNA einführt, die die zusätzlichen DNA-Sequenzen enthält.

27. Verfahren nach Anspruch 26, dadurch gekennzeichnet, daß die zusätzlichen DNA-Sequenzen einen Replikationsstart von *E. coli* und einen selektiven genetischen Marker für *E. coli* umfassen.

28. Verfahren nach Anspruch 26, dadurch gekennzeichnet, daß die zusätzlichen DNA-Sequenzen einen Hefe-Replikationsstart und einen selektiven genetischen Marker für Hefe umfassen.

29. Verfahren nach Anspruch 27, dadurch gekennzeichnet, daß der Hybridvektor zusätzlich einen Hefe-Replikationsstart und einen selektiven genetischen Marker für Hefe umfaßt.

**Revendications pour les Etats Contractants: BE, CH, DE, FR, IT, LU, NL, SE, LI**

1. Fragment de restriction d'ADN consistant essentiellement en promoteur répressible de phosphatase acide de levure (PHO5) comprenant la séquence

```
                                                                         G
ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG
```

et ses sous-fragments et mutants qui conservent la fonction de promoteur.

2. Fragment d'ADN selon la revendication 1 ayant la séquence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

et ses sous-fragments et mutants qui conservent la fonction de promoteur.
3. Fragment d'ADN selon la revendication 1 ayant la séquence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTACTATGG

CTTCATCTCTCATGAGAATAAGAACAACAACAAATAGAGCAAGCAAATTCGGG

et ses mutants qui conservent la fonction de promoteur.
4. Fragment d'ADN selon la revendiction 1 ayant la séquence

ATCCGAAAGTTGTATTCAACAAGAATGCGCAAATATGTCAACGTATTTGCAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

et ses mutants qui conservent la fonction de promoteur.

5. Méthode de production d'un fragment d'AND selon l'une quelconque des revendications 1 à 4, qui comprend

(A) la préparation du gène *PHO5* de phosphatase acide en complémentant des souches de levure déficitaires en *PHO5* de phosphatase acide par transformation par un ADN plasmidique provenant d'une bangue de gènes de levure contenant la copie de type sauvage dudit gène et en isolant ledit gène,

(B) la préparation de sous-clones du gène obtenu, et

(C) L'identification de la localisation de la région du promoteur des sous-clones ci-dessus et l'isolement par digestion de restriction des fragments d'ADN consistant essentiellement en promoteur *PHO5* de phosphatase acide,

et de préparation de sous-fragments, raccourcissant les fragments de restriction d'ADN obtenus par digestion avec une endonucléase de restriction ou avec une exonucléase appropriée.

6. Vecteur hybride de levure comprenant un fragment d'ADN consistant essentiellement en promoteur répressible de phosphatase acide de levure (*PHO5*) selon l'une quelconque des revendications 1 à 4 et une région codant pour un polypeptide de levure ou non-levure qui est contrôlée par ledit promoteur.

7. Vecteur hybride selon la revendication 6 dans lequel la région codant pour un polypeptide de levure ou non-levure code pour une enzyme, une hormone, un polypeptide avec des propriétés immuno-modulatrices, anti-virales ou anticancéreuses, un anticorps, un antigène viral, un vaccin ou un facteur de coagulation.

8. Vecteur hybride selon la revendication 7, dans lequel la région codant pour un polypeptide de levure ou non-levure code pour un interféron humain.

9. Vecteur hybride selon la revendication 8, dans lequel la région codant pour un polypeptide de levure ou non-levure code pour un interféron de lymphoblastoïdes humains.

10. Un vecteur hybride selon l'une quelconque des revendications 6 à 9 comprenant des séquences d'ADN supplémentaires dérivées du groupe consistant en un ADN de plasmide bactérien, de bactériophage lambda, d'un plasmide de levure 2μ et/ou d'un ADN chromosomique de levure.

11. Vecteur hybride selon la revendication 10 incluant un ADN chromosomique de levure comprenant un segment se répliquant de façon autonome et un gène marquer.

12. Vecteur hybride selon la revendication 6 comprenant des signaux d'arrêt de transcription d'un gène de levure.

13. Vecteur hybride selon la revendication 12 comprenant les signaux d'arrêt de transcription du gène *PHO5*.

14. Vecteur hybride selon la revendication 6 dans lequel la région codant pour un polypeptide de levure ou non-levure est précédée d'une séquence signal.

15. Vecteur hybride selon la revendication 6 dans lequel la région codant pour un polypeptide de levure ou non-levure est liée au promoteur de *PHO5* de phosphatase acide entre le principal début d'ARNm de *PHO5* de phosphatase acide et l'ATG de la région codant pour *PHO5* de phosphatase acide.

16. Méthode de production d'un vecteur hybride selon l'une quelconque des revendications 6 à 15, méthode qui comprend l'introduction dans un ADN de vecteur d'un fragment d'ADN consistant essentiellement en promoteur répressible de phosphatase acide le levure (*PHO5*) selon l'une quelconque des revendications 1 à 4 et en une région codant pour un polypeptide de levure ou non-levure de telle sorte qu'elle soit contrôlée par ledit promoteur.

17. Hôte levure transformé par un vecteur hybride selon l'une quelconque des revendications 6 à 15.

18. Levure transformée selon la revendication 17, la levure étant *Saccharomyces cerevisiae*.

19. Méthode de production d'une levure transformée selon l'une des revendications 17 et 18 comprenant la transformation de la levure par un vecteur hybride selon l'une quelconque des revendications 6 à 15.

20. Méthode de production d'un polypeptide de levure ou non-levure ou d'un dérivé de ce dernier existant naturellement, méthode qui comprend les étapes de (1) culture de la levure isolée selon l'une des revendications 17 et 18, et (2) isolement et purification du polypeptide ou de son dérivé.

21. Méthode de production d'un polypeptide de levure ou non-levure selon la revendication 20, caractérisé par le fait que la levure transformée est cultivée dans un milieu nutritif contenant des sources assimilables de carbone et d'azote et des sels minéraux.

22. Méthode de production d'un polypeptide de levure ou non-levure selon la revendication 20, dans laquelle le milieu nutritif contient une faible concentration de phosphate minéral.

23. Méthode selon la revendication 20, dans laquelle le polypeptide est une enzyme, une hormone, un polypeptide avec des propriétiés immuno-régulatrices, antivirales ou anticancéreuses, un anticorps, un antigène viral, un vaccin ou un facteur de coagulation.

24. Méthode selon la revendication 20, dans laquelle le polypeptide est un interféron humain.

25. Méthode selon la revendication 20, dans laquelle l'interféron humain est un interféron de lymphoblastoïdes humains.

26. Méthode selon la revendication 20, dans laquelle l'interféron de lymphoblastoïdes humains est IFN-alpha-1.

27. Méthode selon la revendication 20, dans laquelle l'interféron de lymphoblastoïdes humains est IFN-alpha-2.

28. Méthode selon la revendication 20, dans laquelle l'interféron de lymphoblastoïdes humains est IFN-alpha-3.

29. Vecteur hybride comprenant un fragment d'ADN consistant essentiellement en promoteur répressible de phosphatase acide le levure (*PHO5*) selon l'une quelconque des revendications 1 à 4, et des séquences d'ADN supplémentaires dérivées du groupe consistant et un ADN de plasmide bactérien, de bactériophage lambda, de plasmide de levure 2μ et/ou d'un ADN chromosomique de levure.

30. Vecteur Hybride selon la revendication 29 dans lequel les séquences d'ADN supplémentaires comprennent une origine de réplication de *E. coli* et un marquer génétique sélectif pour *E. coli*.

31. Vecteur hybride selon la revendication 29 dans lequel les séquences d'ADN supplémentaires comprennent une origine de réplication de levure et un marquer génétique sélectif pour la levure.

32. Vecteur hybride selon la revendication 30 qui comprend en outre une origine de réplication de levure et un marqueur génétique sélectif pour la levure.

**Revendications pour L'etet Contractant: AT**

1. Méthode de production d'un fragment de restriction d'ADN consistant essentiellement en promoteur répressible de phosphatase acide de levure (*PHO5*) comprenant la séquence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

et ses sous-fragments et mutants qui conservent la fonction de promoteur, méthode qui comprend (A) la préparation du gène *PHO5* de phosphatase acide en complémentant des souches de levure

déficitaires en *PHO5* de phosphatase acide par transformation par un ADN plasmidique provenant d'une banque de gènes de levure contenant la copie de type sauvage dudit gène et en isolant ledit gène,

(B) la préparation de sous-clones du gène obtenu, et

(C) L'identification de la localisation de la région du promoteur des sousclones ci-dessus et l'isolement par digestion de restriction des fragments d'ADN consistant essentiellement en promoteur *PHO5* de phosphatase acide,

et de préparation de sous-fragments, raccourcissant les fragments de restriction d'ADN obtenus par digestion avec une endonucléase de restriction ou avec une exonucléase appropriée.

2. Méthode selon la revendication 1, caractérisé en ce que le fragment d'ADN a la séquence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTACCA

ATGTTTAAATCTGTTGTTTATTCAATTTTAGCCGCTTCTTTGGCCAATGCAGGTACCATT

CCCTTAGGCAAACTAGCCGATG

ou un sous-fragment ou un mutant de celle-ci qui conserve la fonction de promoteur.

3. Méthode selon la revendication 1, caractérisé en ce que le fragment d'ADN a la séquence

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGGG

ou un mutant de celle-ci, qui conserve la fonction de promoteur.

4. Méthode selon la revendication 1, caractérisée en ce que le fragment d'ADN a la séquence

```
ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG
```

ou un mutant de celle-ci, qui conserve la fonction de promoteur.

5. Méthode de production d'un vecteur hybride de levure comprenant un fragment d'ADN consistant essentiellement en promoteur répressible de phosphatase acide de levure (PHO5) pouvant être obtenu selon l'une quelconque des revendications 1 à 4 et une région codant pour un polypeptide de levure ou non-levure qui est contrôlée par ledit promoteur, méthode qui comprend l'introduction dans un ADN de vecteur dudit fragment d'ADN et une région codant pour un polypeptide de levure ou non-levure de telle sorte qu'elle soit contrôlée par ledit promoteur.

6. Méthode selon la revendication 5, dans laquelle le région codant pour un polypeptide de levure ou non-levure code pour une enzyme, une hormone, un polypeptide avec des propriétés immunomodulatrices, antivirales ou anticancéreuses, un anticorps, un antigène viral, un vaccin ou un facteur de coagulation.

7. Méthode selon la revendication 5, dans laquelle la région codant pour un polypeptide de levure ou non-levure code pour un interféron humain.

8. Méthode selon la revendication 5, dans laquelle la région codant pour un polypeptide de levure ou non-levure code pour un interféron de lymphoblastoïdes humains.

9. Méthode selon la revendication 5, caractérisé en ce que le vecteur hybride comprend des séquences d'ADN supplémentaires dérivées du groupe consistant en un ADN de plasmide bactérien, de bactériophage lambda, de plasmide de levure 2μ et/ou d'un ADN chromosomique de levure.

10. Méthode selon la revendication 5, caractérisée en ce que le vecteur hybride comprend un ADN chromosomique de levure contenant un segment se répliquant de façon autonome et un gène marqueur.

11. Méthode selon la revendication 5, caractériée en ce que le vecteur hybride comprend des signaux d'arrêt de transcription d'un gène de levure.

12. Méthode selon la revendication 5, caractérisée en ce que le vecteur hybride comprend des signaux d'arrêt de transcription du gène PHO5.

13. Méthode selon la revendication 5, caractérisée en ce.que la région codant pour un polypeptide de levure ou non-levure est précédée d'une séquence signal.

14. Méthode selon la revendication 5, caractérisée en ce que la région codant pour le polypeptide de levure ou non-levure est reliée à la région du promoteur de phosphatase acide PHO5 entre le principal début d'ARNm de PHO5 de phosphatase acide et l'ATG de la région codant pour PHO5 de phosphatase acide.

15. Méthode de production de levure transformée contenant un vecteur hybride pouvant être obtenu par l'une quelconque des revendications 5 à 14, méthode qui comprend la transformation de la levure par ledit vecteur hybride.

16. Méthode selon la revendication 15, dans laquelle la levure est Saccharomyces cerevisiae.

17. Méthode de production d'un polypeptide de levure ou non-levure ou d'un dérivé de ce dernier existant naturellement, méthode qui comprend les étapes de (1) culture de la levure transformée pouvant être obtenue par la méthode selon l'une des revendications 15 et 16, et (2) isolement et purification de polypeptide ou de son dérivé.

18. Méthode de production d'un polypeptide de levure ou non-levure selon la revendication 17, caractérisée en ce que la levure transformée est cultivée dans un milieu nutritif contenant des sources assimilables de carbone et d'azote et des sels minéraux.

19. Méthode de production d'un polypeptide de lévure ou non-levure selon la revendication 17, dans laquelle le milieu nutritif contient une faible concentration de phosphate minéral.

20. Méthode selon la revendication 17, dans laquelle le polypeptide est une enzyme, une hormone, un polypeptide avec des propriétés immuno-modulatrices, antivirales ou anticancéreuses, un anticorps, un antigène viral, un vaccin ou un facteur de coagulation.

21. Méthode selon la revendication 17, dans laquelle le polypeptide est un interféron humain.

22. Méthode selon la revendication 17, dans laquelle l'interféron humain est un inteféron de lymphoblastoïdes humains.

23. Méthode selon la revendication 17, dans laquelle l'interféron de lymphoblastoïdes humains est IFN-alpha-1.

24. Méthode selon la revendication 17, dans laquelle l'interféron de lymphoblastoïdes humains est IFN-alpha-2.

25. Méthode selon la revendication 17, dans laquelle l'interféron de lymphoblastoïdes humains est IFN-alpha-3.

26. Méthode de production d'un vecteur hybride comprenant un fragment d'ADN consistant essentiellement en promoteur répressible de phosphatase acide de levure (*PHO5*) pouvant être obtenu selon l'une quelconque des revendications 1 à 4 et des séquence d'ADN supplémentaires dérivées du groupe consistant en un ADN de plasmide bactérien, un bactériophage lambda, un plasmide de levure 2μ et/ou un ADN chromosomique de levure, méthode qui comprend l'introduction dudit fragment d'ADN dans un ADN de vecteur contenant lesdites séquence d'ADN supplémentaires.

27. Méthode selon la revendication 26, caractérisée en ce que les séquences d'ADN supplémentaires comprennent une origine de réplication de *E. coli* et un marqueur génétique sélectif pour *E. coli*.

28. Méthode selon la revendication 26, caractérisée en ce que les séquences d'ADN supplémentaires comprennent une origine de réplication de levure et un marqueur génétique sélectif pour la levure.

29. Méthode selon la revendication 27, caractérisée en ce que le vecteur hybride comprend en outre une origine de réplication de levure et un marqueur génétique sélectif pour la levure.

# Fig.1

STARTING PLASMIDS FOR DNA SEQUENCING AND EXPRESSION PLASMID CONSTRUCTION

## _Fig. 2_

LOCALIZATION OF THE REPRESSIBLE (PHO5) AND THE CONSTITUTIVE (PHO3) ACID PHOSPHATASE GENE

phosphatase activity       cloned subfragments

repressed   derepressed
(+P$_i$)      (-P$_i$)

EP 0 100 561 B1

# Fig. 3

NUCLEOTIDE SEQUENCE OF THE BamHI-SalI RESTRICTION FRAGMENT
OF PHO5 INCLUDING THE PHO5 PROMOTER REGION

                                                            G

-540
    ATCCGAAAGTTGTATTCAACAAGAATGCGCAAATATGTCAACGTATTTGGAAGTCATCTT

-480
    ATGTGCGCTGCTTTAATGTTTTCTCATGTAAGCGGACGTCGTCTATAAACTTCAAACGAA

-420
    GGTAAAAGGTTCATAGCGCTTTTTCTTTGTCTGCACAAAGAAATATATATTAAAATTAGCA

-360
    CGTTTTCGCATAGAACGCAACTGCACAATGCCAAAAAAAGTAAAAGTGATTAAAAGAGTT

-300
    AATTGAATAGGCAATCTCTAAATGAATCGATACAACCTTGGCACTCACACGTGGGACTAG

-240
    CACAGACTAAATTTATGATTCTGGTCCCTGTTTTCGAAGAGATCGCACATGCCAAATTAT

-180
    CAAATTGGTCACCTTACTTGGCAAGGCATATACCCATTTGGGATAAGGGTAAACATCTTT

-120
    GAATTGTCGAAATGAAACGTATATAAGCGCTGATGTTTTGCTAAGTCGAGGTTAGTATGG

-60
    CTTCATCTCTCATGAGAATAAGAACAACAACAAATAGAGCAAGCAAATTCGAGATTACCA

+1
    ATG TTT AAA TCT GTT GTT TAT TCA ATT TTA GCC GCT TCT TTG GCC +45
    MET PHE LYS SER VAL VAL TYR SER ILE LEU ALA ALA SER LEU ALA

    AAT GCA GGT ACC ATT CCC TTA GGC AAA CTA GCC GAT G+82
    ASN ALA GLY THR ILE PRO LEU GLY LYS LEU ALA ASP

3

## _Fig. 4_

CONSTRUCTION SCHEME FOR THE HYBRID PLASMIDS p30IFN2(8'₁)
AND p30IFN2'(8'₁)

## 𝑭𝒊𝒈. 𝟓

LIGATION OF THE <u>PHO5</u> PROMOTER DNA WITH THE cDNA INSERT OF CG-PBR322/HLYcIFN-8₁

a. DNA SEGMENT CONTAINING THE <u>PHO5</u> PROMOTER AND ADJACENT PROTEIN CODING REGION

BamHI

↓........ <u>PHO5</u> PROMOTER ————— ATG TTT AAA TCT GTT GTT     +1

                                        +43
TAT TCA ATT TTA GCC GCT TCT TTG GCC AAT GCA GGT ACC .....

Bal I (Hae III)

b. DNA SEGMENT CONTAINING INTERFERON PROMOTER AND ADJACENT PROTEIN CODING REGION
OF CG-PBR322/HLYcIFN-8₁

                                          +1    Hae III

......... INTERFERON PROMOTER ——————— ATG GCC TCG CCC TTT GCT

         +28
TTA CTG ATG GCC CTG GTG GTG CTC AGC TGC AAG TCA AGC ........

            Hae III                           EcoRI

c. DNA SEGMENT OF THE FUSION PRODUCT OF a. AND b. IN P30IFN1(8₁) AND
N-TERMINAL SEQUENCE OF THE RESULTING INTERFERON POLYPEPTIDE

BamHI

↓............. <u>PHO5</u> PROMOTER ———— ATG TTT AAA TCT GTT GTT    +1
                                              Met Phe Lys Ser Val Val

                          Hae III                         EcoRI

TAT TCA ATT TTA GCC GCT TCT TTG GCC CTG GTG GTG CTC ........
Tyr Ser Ile Leu Ala Ala Ser Leu Ala Leu Val Val Leu ........

————————— <u>PHO5</u> SEQUENCES

〰〰〰〰〰〰〰〰 INTERFERON SEQUENCES

## Fig.6

CONSTRUCTION OF PLASMID  pJDB207/IFN2'(8'$_1$)

NAMALWA ds cDNA SYNTHESIS AND CONSTRUCTION OF RECOMBINANT DNA MOLECULES

Fig. 7

EP 0 100 561 B1

## Fig. 8

SYNTHESIS OF DNA-PRIMER (13-mer) COMPLEMENTARY TO $\alpha_1$-AND $\beta$-IFN mRNA

Line

1  M—OPO—M—OPO—OPOCE  M—OPO—M—OPO—OPOCE  M—OPO—OPOCE  D—OPO—OPOCE  M—OPO—OPOCE  M—OBz

2      H————P          H————P        M————P      H————P    M————P    H—Bz

3  M————————P      M————————P    M————————P    M————————Bz

4  M————————P      H————————P    M————————P    H————————Bz

5  M————————————————P    M————————————————Bz

6  M————————————————P    H————————————————Bz

7  M—O-P-O—O-P-O—O-P-O—O-P-O—O-P-O—O-P-O—O-P-O—O-P-O—O-P-O—O-P-O—O-P-O—O-P-O—OBz

8  5'd(CCTTCTGGAACTG)3'

### Abbreviations:

M = Monomethoxytrityl  CE = 2-Cyanoethyl  Ibu = Isobutyl  P = Phosphotriester  H = 5'-OH

R = 2-Chlorphenyl  Bz = Benzoyl  D = Dimethoxytrityl  P = Phosphodiester

## Fig. 9

### SYNTHESIS OF HuIFN-β AND HuIFN-α SPECIFIC PROBE AND USE FOR THE INDENTIFICATION OF HUMAN LYMPHOBLASTOID IFN-CONTAINING CLONES.

## Fig. 10

NUCLEOTIDE SEQUENCE OF THE cDNA INSERT OF THE RECOMBINANT PLASMID DNA CG-pBR 322/HLycIFN-1'b

```
      1
      MET ALA LEU SER PHE SER LEU LEU MET ALA VAL LEU VAL LEU SER TYR LYS SER ILE CYS SER LEU GLY
ACATCCCA ATG GCC CTG TCC TTT TCT TTA CTG ATG GCC GTG CTG GTG CTC AGC TAC AAA TCC ATC TGT TCT CTG GGC
                                                                                                     69

CYS  ASP LEU PRO GLN THR HIS SER LEU GLY ASN ARG ARG ALA LEU ILE LEU LEU ALA GLN MET GLY ARG ILE SER
TGT  GAT CTG CCT CAG ACC CAC AGC CTG GGT AAT AGG AGG GCC TTG ATA CTC CTG GCA CAA ATG GGA AGA ATC CCC
                                                                                                    144

PRO PHE SER CYS LEU LYS ASP ARG HIS ASP PHE GLY PHE PRO GLN GLU GLU PHE ASP GLY ASN GLN PHE GLN LYS
CCT TTC TCC TGC CTG AAG GAC AGA CAT GAC TTT GGA TTT CCC CAG GAG GAG TTT GAT GGC AAC CAG TTC CAG AAG
                                                                                                    219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAT CTC TTC AGC ACA AAG GAC TCA TCT GCT
                                                                                                    294

THR TRP GLU GLN SER LEU LEU GLU LYS PHE SER THR GLU LEU ASN GLN GLN LEU ASN ASP LEU GLU ALA CYS VAL
ACT TGG GAA CAG AGC CTC CTA GAA AAA TTT TCC ACT GAA CTT AAC CAG CAG CTG AAT GAC CTG GAA GCC TGC GTG
                                                                                                    369

ILE GLN GLU VAL GLY VAL GLU GLU THR PRO LEU MET ASN VAL ASP SER ILE LEU ALA VAL LYS LYS TYR PHE GLN
ATA CAG GAG GTT GGG GTG GAA GAG ACT CCC CTG ATG AAT GTG GAC TCC ATC CTG GCT GTG AAG AAA TAC TTC CAA
                                                                                                    444

ARG ILE THR LEU TYR LEU THR GLU LYS LYS TYR SER PRO CYS ALA TRP GLU VAL VAL ARG ALA GLU ILE MET ARG
AGA ATC ACT CTT TAT CTG ACA GAG AAG AAA TAC AGC CCT TGT GCC TGG GAG GTT GTC AGA GCA GAA ATC ATG AGA
                                                                                                    519

SER PHE SER LEU SER LYS ILE PHE GLN GLU ARG LEU ARG ARG LYS GLU
TCC TTC TCT TTA TCA AAA ATT TTT CAA GAA AGA TTA AGG AGG AAG GAA TGA AACCTGTTTCAACATGGAAATGATCTGTATT
                                                                                                    601

GACTAATACACCAGTCCACACTTCTATGACTTCTGCCATTTCAAAGACTCATTTCTCCTATAACCACCGCATGAGTTGAATCAAAATTTTCAGATCTTT
                                                                                                    700

TCAGGAGTGTAAGGAAACATCATGTTTACCTGTGCAGGCACTAGTCCTTTACAGATGACCATGCTGATAGATCTAATTATCTATCTATTGAAATATTTA
                                                                                                    799

TTTATTTATTAGATTTAAATTATTTTTGTCCATGTAATATTATGTGTACTTTTACATTGTGTTATATCAAAATATGTTATTTATATTTAGTCAATATAT
                                                                                                    898

TATTTTCTTTTTATTAATTTTTACTATTAAAACTTCTTATATTATTTGTTTATTG
```

## Fig. 11

NUCLEOTIDE SEQUENCE OF THE cDNA INSERT OF THE RECOMBINANT PLASMID DNA CG-pBR 322/HLycIFN-$\beta_1$

```
                                   SER SER ASN PHE GLN CYS GLN LYS LEU LEU TRP GLN LEU ASN
                                   AGC AGC AAT TTT CAG TGT CAG AAG CTC CTG TGG CAA TTG AAT 138

GLY ARG LEU GLU TYR CYS LEU LYS ASP ARG MET ASN PHE ASP ILE PRO GLU GLU ILE LYS GLN LEU GLN GLN PHE
GGG AGG CTT GAA TAC TGC CTC AAG GAC AGG ATG AAC TTT GAC ATC CCT GAG GAG ATT AAG CAG CTG CAG CAG TTC 213

GLN LYS GLU ASP ALA ALA LEU THR ILE TYR GLU MET LEU GLN ASN ILE PHE ALA ILE PHE ARG GLN ASP SER SER
CAG AAG GAG GAC GCC GCA TTG ACC ATC TAT GAG ATG CTC CAG AAC ATC TTT GCT ATT TTC AGA CAA GAT TCA TCT 288

SER THR GLY TRP ASN GLU THR ILE VAL GLU ASN LEU LEU ALA ASN VAL TYR HIS GLN ILE ASN HIS LEU LYS THR
AGC ACT GGC TGG AAT GAG ACT ATT GTT GAG AAC CTC CTG GCT AAT GTC TAT CAT CAG ATA AAC CAT CTG AAG ACA 363

VAL LEU GLU GLU LYS LEU GLU LYS GLU ASP PHE THR ARG GLY LYS LEU MET SER SER LEU HIS LEU LYS ARG TYR
GTC CTG GAA GAA AAA CTG GAG AAA GAA GAT TTC ACC AGG GGA AAA CTC ATG AGC AGT CTG CAC CTG AAA AGA TAT 438

TYR GLY ARG ILE LEU HIS TYR LEU LYS ALA LYS GLU TYR SER HIS CYS ALA TRP THR ILE VAL ARG VAL GLU ILE
TAT GGG AGG ATT CTG CAT TAC CTG AAG GCC AAG GAG TAC AGT CAC TGT GCC TGG ACC ATA GTC AGA GTG GAA ATC 513

LEU ARG ASN PHE TYR PHE ILE ASN ARG LEU THR GLY TYR LEU ARG ASN
CTA AGG AAC TTT TAC TTC ATT AAC AGA CTT ACA GGT TAC CTC CGA AAC TGAAGATCTCCTAGCCTGTGCCTCTGGGACTGGAC 596

AATTGCTTCAAGCATTCTTCAACCAGCAGATGCTGTTTAAGTGACTGATGGCTAATGTACTGCATATGAAAGGACACTAGAAGATTTTGAAATTTTTAT 695

TAAATTATGAGTTATTTTTATTTATTTAAATTTTATTTTGGAAAATAAATTATTTTTGGTGCAAAAGTC
```

# Fig. 12

NUCLEOTIDE SEQUENCE OF THE cDNA INSERT OF THE RECOMBINANT PLASMID DNA CG-pBR 322/HLycIFN-4$_1$

```
                                                                        SER SER ALA
                                                                        TCA TCT GCT 294

ALA TRP ASP GLU THR LEU LEU ASP LYS PHE TYR THR GLU LEU TYR GLN GLN LEU ASN ASP LEU GLU ALA CYS VAL
GCT TGG GAT GAG ACC CTC CTA GAC AAA TTC TAC ACT GAA CTC TAC CAG CAG CTG AAT GAC CTG GAA GCC TGT GTG 369

ILE GLN GLY VAL GLY VAL THR GLU THR PRO LEU MET LYS GLU ASP SER ILE LEU ALA VAL ARG LYS TYR PHE GLN
ATA CAG GGG GTG GGG GTG ACA GAG ACT CCC CTG ATG AAG GAG GAC TCC ATT CTG GCT GTG AGG AAA TAC TTC CAA 444

ARG ILE THR LEU TYR LEU LYS GLU LYS LYS TYR SER PRO CYS ALA TRP GLU VAL VAL ARG ALA GLU ILE MET ARG
AGA ATC ACT CTC TAT CTG AAA GAG AAG AAA TAC AGC CCT TGT GCC TGG GAG GTT GTC AGA GCA GAA ATC ATG AGA 519

SER PHE SER LEU SER THR ASN LEU GLN GLU SER LEU ARG SER LYS GLU
TCT TTT TCT TTG TCA ACA AAC TTG CAA GAA AGT TTA AGA AGT AAG GAA TGA AAACTGGTTCAACATGGAAATGATTTTCATT 601

GATTCGTATGCCAGCTCACCTTTTTATGATCTGCCATTTCAAAGACTCATGTTTCTGCTATGACCATGACACGATTTAAATCTTTTCAAATGTTTTTAG 700

GAGTATTAATCAACATTGTATTCAGCTCTTAAGGCACTAGTCCCTTACAGAGGACCATGCTGACTGATCCATTATCTATTTAAATATTTTTAAAATATT 799

ATTTATTTAACTATTTATAAAACAACTTATTTTTGTTCATATTATGTCATGTGCACCTTTGCACAGTGGTTAATGTAATAAAATGTGTTCTTTGTATTT 898

GGTATATTTATTTTGTGTTGTTCATTGAACTTTTGCTATGGAACTTTTGTACTTGTTTATTCTTTAAAATGAAATTCCAAGCCTAATTGTGCAACCTGA 997

TTACAGAATAACTGGTACACTTCATTTATCCATCAATATTATATTCAAGATATAAGTAAAAATAAACTTTCTGTAAACCAGTTG
```

# Fig. 13

NUCLEOTIDE SEQUENCE OF THE C-DNA INSERT OF THE RECOMBINANT PLASMID DNA CG-PBR 322/HLYCIFN-8'₁

```
                                                        CCCAAGGTTCAGAGTCACCCATCTCAGCAAGCCCAGAAGCATCTGCAAT

         1
         MET ALA SER PRO PHE ALA LEU LEU MET|ALA|LEU VAL VAL LEU SER CYS LYS SER SER CYS SER LEU GLY
ATCTATG ATG GCC TCG CCC TTT GCT TTA CTG ATG|GCC|CTG GTG GTG CTC AGC TGC AAG TCA AGC TGC TCT CTG GGC₆₉
         Hae III                                Hae III

CYS ASP LEU PRO GLU THR HIS SER LEU ASP ASN ARG ARG THR LEU MET LEU LEU ALA GLN MET SER ARG ILE SER
TGT GAT CTC CCT GAG ACC CAC AGC CTG GAT AAC AGG AGG ACC TTG ATG CTC CTG GCA CAA ATG AGC AGA ATC TCT₁₄₄

PRO SER SER CYS LEU MET ASP ARG HIS ASP PHE GLY PHE PRO GLN GLU GLU PHE ASP GLY ASN GLN PHE GLN LYS
CCT TCC TCC TGT CTG ATG GAC AGA CAT GAC TTT GGA TTT CCC CAG GAG GAG TTT GAT GGC AAC CAG TTC CAG AAG₂₁₉

ALA PRO ALA ILE SER VAL LEU HIS GLU LEU ILE GLN GLN ILE PHE ASN LEU PHE THR THR LYS ASP SER SER ALA
GCT CCA GCC ATC TCT GTC CTC CAT GAG CTG ATC CAG CAG ATC TTC AAC CTC TTT ACC ACA AAA GAT TCA TCT GCT₂₉₄

ALA TRP ASP GLU ASP LEU LEU ASP LYS PHE CYS THR GLU LEU TYR GLN GLN LEU ASN ASP LEU GLU ALA CYS VAL
GCT TGG GAT GAG GAC CTC CTA GAC AAA TTC TGC ACC GAA CTC TAC CAG CAG CTG AAT GAC TTG GAA GCC TGT GTG₃₆₉

MET GLN GLU GLU ARG VAL GLY GLU THR PRO LEU MET ASN|ALA|ASP SER ILE LEU ALA VAL LYS LYS TYR PHE ARG
ATG CAG GAG GAG AGG GTG GGA GAA ACT CCC CTG ATG AAT|GCG|GAC TCC ATC TTG GCT GTG AAG AAA TAC TTC CGA₄₄₄

ARG ILE THR LEU TYR LEU THR GLU LYS LYS TYR SER PRO CYS ALA TRP GLU VAL VAL ARG ALA GLU ILE MET ARG
AGA ATC ACT CTC TAT CTG ACA GAG AAG AAA TAC AGC CCT TGT GCC TGG GAG GTT GTC AGA GCA GAA ATC ATG AGA₅₁₉

SER LEU SER LEU SER THR ASN LEU GLN GLU ARG LEU ARG ARG LYS GLU
TCC CTC TCT TTA TCA ACA AAC TTG CAA GAA AGA TTA AGG AGG AAG GAA TAA CACCTGGTCCAACATGAAACAATTCTTATTG₆₀₁

ACTCATATACCAGGTCACGCTTTCATGAATTCTGCCATTTCAAAGACTCTCACTTCTGCTATAACTATGACCATGCTGATAAACTGATTTATCTATTTA₇₀₀
                        EcoR I

AATATTTATTTAGCTATTCATAAGATTTAAAATTATTTTTGTTCATATAACATCATGTGCATCTTTACACTGTGGTTAGTGTAATAAAACATGTTCCTTA₇₉₉

TATTTACTCAAATTCATTATTTTA
```

EP 0 100 561 B1

**Fig. 14.**

ATCTGAACCAGCTCAGCAGCATCCACAAC

1
MET ALA LEU THR PHE TYR LEU |LEU| VAL ALA LEU VAL VAL LEU SER TYR LYS SER PHE SER SER LEU GLY
ATCTACA ATG GCC TTG ACT TTT TAT TTA |CTG| GTG GCC CTA GTG GTG CTC AGC TAC AAG TCA TTC AGC TCT CTG GGC $_{69}$

CYS ASP LEU PRO GLN THR HIS SER LEU GLY ASN ARG ARG ALA LEU ILE LEU LEU ALA GLN MET ARG ARG ILE SER
TGT GAT CTG CCT CAG ACT CAC AGC CTG GGT AAC AGG AGG GCC TTG ATA CTC CTG GCA CAA ATG CGA AGA ATC TCT $_{144}$

PRO PHE SER CYS LEU LYS ASP ARG HIS ASP PHE GLU PHE PRO GLN GLU GLU PHE ASP ASP LYS GLN PHE GLN LYS
CCT TTC TCC TGC CTG AAG GAC AGA CAT GAC TTT GAA TTC CCC CAG GAG GAG TTT GAT GAT AAA CAG TTC CAG AAG $_{219}$

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAC CTC TTC AGC ACA AAG GAC TCA TCT GCT $_{294}$

ALA LEU ASP GLU THR LEU LEU ASP GLU PHE TYR ILE GLU LEU ASP GLN GLN LEU ASN ASP LEU GLU |SER|CYS|VAL|
GCT TTG GAT GAG ACC CTT CTA GAT GAA TTC TAC ATC GAA CTT GAC CAG CAG CTG AAT GAC CTG GAG|TCC|TGT|GTG| $_{369}$

|MET| GLN GLU VAL GLY VAL ILE GLU SER PRO LEU MET TYR GLU ASP SER ILE LEU ALA VAL ARG LYS TYR PHE GLN
|ATG| CAG GAA GTG GGG GTG ATA GAG TCT CCC CTG ATG TAC GAG GAC TCC ATC CTG GCT GTG AGG AAA TAC TTC CAA $_{444}$

ARG ILE THR LEU TYR LEU THR GLU LYS LYS TYR SER SER CYS ALA TRP GLU VAL VAL ARG ALA GLU ILE MET ARG
AGA ATC ACT CTA TAT CTG ACA GAG AAG AAA TAC AGC TCT TGT GCC TGG GAG GTT GTC AGA GCA GAA ATC ATG AGA $_{519}$

SER PHE SER LEU SER ILE ASN LEU GLN LYS ARG LEU LYS SER LYS GLU
TCC TTC TCT TTA TCA ATC AAC TTG CAA AAA AGA TTG AAG AGT AAG GAA TGA GACCTGGTACAACACGGAAATGATTCTTATA $_{601}$

GACTAATACAGCAGCTCACACTTCGACAAGTTGTGCTCTTTCAAAGACCCTTGTTTCTGCCAAAACCATGCTATGTTTTGAATCAAATGTGTCAAGTGT $_{700}$

TTTCAGGAGTGTTAAGCAACATCCTGTTCAGCTGTATGGGCACTAGTCCCTTACAGATGACCATGCTGATGGATCTATTCATCTATTTATTTAAATCTT $_{799}$

TATTTAGTTAACTATCTATAGGGCTTAAATTAGTTTTGTTCATATTATATTATGTGAACTTTTACATTGTGAATTGTGTAACAAAAACATGTTCTTTAT $_{898}$

ATTTATTATTTTGCCTTGTTTATTAAATTTTTACTATAG

## CONSTRUCTION OF CG-pBR (AP) /Ly IFN-α-1 RECOMBINANT DNA

ApPr:β-lactamase expression control region

*Fig. 15*

## Fig. 16

CG-pBR(AP)/LyIFN-α-1

PROMOTOR β-LACTAMASE      MET

...GAGACAATAACCCTGATAAATGCTTCAATAATATTGAAAAAGGAAGAGTAATG

CYS ASP LEU PRO GLN THR HIS SER LEU GLY ASN ARG ARG ALA LEU ILE LEU LEU ALA GLN MET GLY ARG ILE SER
TGT GAT CTG CCT CAG ACC CAC AGC CTG GGT AAT AGG AGG GCC TTG ATA CTC CTG GCA CAA ATG GGA AGA ATC CCC 144

PRO PHE SER CYS LEU LYS ASP ARG HIS ASP PHE GLY PHE PRO GLN GLU GLU PHE ASP GLY ASN GLN PHE GLN LYS
CCT TTC TCC TGC CTG AAG GAC AGA CAT GAC TTT GGA TTT CCC CAG GAG GAG TTT GAT GGC AAC CAG TTC CAG AAG 219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAT CTC TTC AGC ACA AAG GAC TCA TCT GCT 294

THR TRP GLU GLN SER LEU LEU GLU LYS PHE SER THR GLU LEU ASN GLN GLN LEU ASN ASP LEU GLU ALA CYS VAL
ACT TGG GAA CAG AGC CTC CTA GAA AAA TTT TCC ACT GAA CTT AAC CAG CAG CTG AAT GAC CTG GAA GCC TGC GTG 369

ILE GLN GLU VAL GLY VAL GLU GLU THR PRO LEU MET ASN VAL ASP SER ILE LEU ALA VAL LYS LYS TYR PHE GLN
ATA CAG GAG GTT GGG GTG GAA GAG ACT CCC CTG ATG AAT GTG GAC TCC ATC CTG GCT GTG AAG AAA TAC TTC CAA 444

ARG ILE THR LEU TYR LEU THR GLU LYS LYS TYR SER PRO CYS ALA TRP GLU VAL VAL ARG ALA GLU ILE MET ARG
AGA ATC ACT CTT TAT CTG ACA GAG AAG AAA TAC AGC CCT TGT GCC TGG GAG GTT GTC AGA GCA GAA ATC ATG AGA 519

SER PHE SER LEU SER LYS ILE PHE GLN GLU ARG LEU ARG ARG LYS GLU
TCC TTC TCT TTA TCA AAA ATT TTT CAA GAA AGA TTA AGG AGG AAG GAA TGA AACCTGTTTCAACATGGAAATGATCTGTATT 601

GACTAATACACCAGTCCACACTTCTATGACTTCTGCCATTTCAAAGACTCATTTCTCCTATAACCACCGCATGAGTTGAATCAAAATTTTCAGATCTTT 700

TCAGGAGTGTAAGGAAACATCATGTTTACCTGTGCAGGCACTAGTCCTTTACAGATGACCATGCTGATAGATCTAATTATCTATCTATTGAAATATTTA 799

TTTATTTATTAGATTTAAATTATTTTTGTCCATGTAATATTATGTGTACTTTTACATTGTGTTATATCAAAATATGTTATTTATATTTAGTCAATATAT 898

TATTTTCTTTTTATTAATTTTTACTATTAAAACTTCTTATATTATTTGTTTATTG...

## *Fig.17*

CONSTRUCTION OF THE RECOMBINANT DNA PLASMID CG-pBR(AP)/Ly IFN-α-3

## Fig. 18

CG-pBR(AP)/LyIFN-α-3

PROMOTOR β-LACTAMASE     MET

...GAGACAATAACCCTGATAAATGCTTCAATAATATTGAAAAAGGAAGAGTAATG

CYS ASP LEU PRO GLU THR HIS SER LEU ASP ASN ARG ARG THR LEU MET LEU LEU ALA GLN MET SER ARG ILE SER
TGT GAT CTC CCT GAG ACC CAC AGC CTG GAT AAC AGG AGG ACC TTG ATG CTC CTG GCA CAA ATG AGC AGA ATC TCT 144

PRO SER SER CYS LEU MET ASP ARG HIS ASP PHE GLY PHE PRO GLN GLU GLU PHE ASP GLY ASN GLN PHE GLN LYS
CCT TCC TCC TGT CTG ATG GAC AGA CAT GAC TTT GGA TTT CCC CAG GAG GAG TTT GAT GGC AAC CAG TTC CAG AAG 219

ALA PRO ALA ILE SER VAL LEU HIS GLU LEU ILE GLN GLN ILE PHE ASN LEU PHE THR THR LYS ASP SER SER ALA
GCT CCA GCC ATC TCT GTC CTC CAT GAG CTG ATC CAG CAG ATC TTC AAC CTC TTT ACC ACA AAA GAT TCA TCT GCT 294

ALA TRP ASP GLU ASP LEU LEU ASP LYS PHE CYS THR GLU LEU TYR GLN GLN LEU ASN ASP LEU GLU ALA CYS VAL
GCT TGG GAT GAG GAC CTC CTA GAC AAA TTC TGC ACC GAA CTC TAC CAG CAG CTG AAT GAC TTG GAA GCC TGT GTG 369

MET GLN GLU GLU ARG VAL GLY GLU THR PRO LEU MET ASN ALA ASP SER ILE LEU ALA VAL LYS LYS TYR PHE ARG
ATG CAG GAG GAG AGG GTG GGA GAA ACT CCC CTG ATG AAT GCG GAC TCC ATC TTG GCT GTG AAG AAA TAC TTC CGA 444

ARG ILE THR LEU TYR LEU THR GLU LYS LYS TYR SER PRO CYS ALA TRP GLU VAL VAL ARG ALA GLU ILE MET ARG
AGA ATC ACT CTC TAT CTG ACA GAG AAG AAA TAC AGC CCT TGT GCC TGG GAG GTT GTC AGA GCA GAA ATC ATG AGA 519

SER LEU SER LEU SER THR ASN LEU GLN GLU ARG LEU ARG ARG LYS GLU
TCC CTC TCT TTA TCA ACA AAC TTG CAA GAA AGA TTA AGG AGG AAG GAA TAA CACCTGGTCCAACATGAAACAATTCTTATTG 601

ACTCATATACCAGGTCACGCTTTCATGAATTCTGCCATTTCAAAGACTCTCACTTCTGCTATAACTATGACCATGCTGATAAACTGATTTATCTATTTA 700

AATATTTATTTAGCTATTCATAAGATTTAAATTATTTTTGTTCATATAACATCATGTGCATCTTTACACTGTGGTTAGTGTAATAAAACATGTTCCTTA 799

TATTTACTCAAATTCATTATTTT...

18

**Fig. 14**

CG-pBR(AP)/LyIFN-α-2

PROMOTOR β-LACTAMASE    MET

...GAGACAATAACCCTGATAAATGCTTCAATAATATTGAAAAAGGAAGAGTAATG

```
CYS ASP LEU PRO GLN THR HIS SER LEU GLY ASN ARG ARG ALA LEU ILE LEU LEU ALA GLN MET ARG ARG ILE SER
TGT GAT CTG CCT CAG ACT CAC AGC CTG GGT AAC AGG AGG GCC TTG ATA CTC CTG GCA CAA ATG CGA AGA ATC TCT   144

PRO PHE SER CYS LEU LYS ASP ARG HIS ASP PHE GLU GLY PRO GLN PHE ASP ASP LYS GLN PHE GLN LYS
CCT TTC TCC TGC CTG AAG GAC AGA CAT GAC TTT GAA TTC CCC CAG GAG TTT GAT GAT AAA CAG TTC CAG AAG   219

ALA GLN ALA ILE SER VAL LEU HIS GLU MET ILE GLN GLN THR PHE ASN LEU PHE SER THR LYS ASP SER ALA
GCT CAA GCC ATC TCT GTC CTC CAT GAG ATG ATC CAG CAG ACC TTC AAC CTC TTC AGC ACA AAG GAC TCA TCT GCT   294

ALA LEU ASP GLU THR LEU LEU ASP GLU PHE TYR ILE GLU LEU ASP GLN GLN LEU ASN ASP LEU GLU SER CYS VAL
GCT TTG GAT GAG ACC CTT CTA GAT GAA TTC TAC ATC GAA CTT GAC CAG CAG AAT GAC CTG GAG TCC TGT GTG   369

MET GLN GLU VAL GLY VAL ILE GLU VAL MET TYR GLU SER PRO LEU MET LYS TYR PHE GLN
ATG CAG GAA GTG GGG GTG ATA GAG GTC ATG TAC CTG ATG TCT CCC CTG ATG AAA TAC TTC CAA   444

ARG ILE THR LEU TYR LEU THR GLU LYS LYS TYR SER SER CYS ALA TRP GLU VAL VAL ARG ALA GLU ILE MET ARG
AGA ATC ACT CTA TAT CTG ACA GAG AAG AAA TAC AGC TCT TGT GCC TGG GAG GTT GTC AGA GCA GAA ATC ATG AGA   519

SER PHE SER LEU SER ILE ASN LEU GLN LYS ARG LEU LYS SER LYS GLU
TCC TTC TCT TTA TCA ATC AAC TTG CAA AAA AGA TTG AAG AGT AAG GAA TGA GACCTGGTACAACACGGAAATGATTCTTATA   601

GACTAATACAGCAGCTCACACTTCGACACTTGTGCTCTTCAAAGACCCTGTTCTGCCAAAACCATGCTATGTTTTGAATCAAATGTGTCAAGTGT   700

TTTCAGGAGTGTTAAGCAACATCCTGTTCAGCTGTATGGGCACTAGTCCCTTACAGATGACCATGCTGCTGATGGATCTATTCATCTATTTAAATCTT   799

TATTTAGTTAACTATCTATAGGGCTTAAATTAGTTTTGTTCATATTATTATTATTATGTGAACTTTTACATTGTGTGTAACAAAAACATGTTCTTTAT   898

ATTTATTATTTTGCCTTGTTTATTAAATTTTTACTATAG...
```

## Fig:20 CONSTRUCTION OF EXPRESSION PLASMID p31

20

EP 0 100 561 B1

# Fig. 21

NUCLEOTIDE SEQUENCE OF THE Sau3A-PstI PHO5 TRANSCRIPTION
TERMINATION FRAGMENT

GATCCTGGTACGTTCCTCAAGGTGCTCGTGTCTACACCGAAAAATTCCAATGTTCTAACG

ACACCTACGTCAGATACGTCATTAACGATGCTGTTGTTCCAATTGAAACCTGTTCCACTG

GTCCAGGGTTCTCTTGTGAAATCAATGACTTCTACGACTATGCTGAAAAGAGAGTAGCCG

GTACTGACTTCCTAAAGGTCTGTAACGTCAGCAGCGTCAGTAACTCTACTGAATTGACCT

TCTACTGGGACTGGAACACTACTCATTACAACGCCAGTCTATTGAGACAATAGTTTTGTA

TAACTAAATAATATTGGAAACTAAATACGAATACCCAAATTTTTTATCTAAATTTTGCCG

AAAGATTAAAATCTGCA

21

INSERTION OF IFN-5₁ AND IFN-1'b CODING SEQUENCES INTO PLASMID p31

Fig.22

## *Fig. 23*

CONSTRUCTION OF EXPRESSION PLASMID p31/IF (8'₁)

p31/IF(8'₁)

**Fig. 24.** CONSTRUCTION OF A CORRECT PHO5-HBVs JUNCTION

EP 0 100 561 B1

## Fig. 25

SEQUENCE ARRANGEMENT AFTER <u>PHO5</u> PROMOTER AND HBVs PROTEIN
CODING REGION FUSION (PLASMID PBR322/<u>PHO5</u>/HBVs).

BamHI ┌──► <u>PHO5</u> mRNA ┌──► ──► <u>PHO5</u>
...TAAGAACAACAACAAATAGAGCAAGCAAATTCGAGATTACCAATGTTTAAATCTGTTGTTTA

KpnI
TTCAATTTTAGCCGCTTCTTTGGCCAATGCAGGTACCATTCCCTTAGGCAAACTAGCCGAT|GTCGAG|

HhaI
↓ ──►HBVs
GATTGGGGACCCTGCGCTGAACATGGAGAAC.......

└────────┘ FUSION POINT (HYBRID RESTRICTION SITE SalI /XhoI)

──────► <u>PHO5</u> START <u>PHO5</u> PROTEIN CODING REGION

──────► HBVs START HBVs PROTEIN CODING REGION

┌──────► MAJOR mRNA INITIATION SITE OF <u>PHO5</u>

┌──► ◄──┐
┌ ┐ DELETION ENDPOINTS OF PBR322/<u>PHO5</u>/HBVs△14

# _Fig. 26_

**CONSTRUCTION OF PLASMIDS pJDB207/PH05/HBVs △14 AND pJDB207/PH05/HBVs △14t**

pJDB 207

pBR322/PH05/HBVs △14

pJDB 207/IF (8'₁)

1. BamHl digest
2. isolation of 1.8kb fragment

pJDB207/PH05/HBVs △14

1. BamHl digest
2. isolation of 1.8kb fragment ligation

1. BamHl digest
2. isolation of 6.9kb fragment

pJDB207/PH05/HBVs △14t

*Fig. 27* CONSTRUCTION OF PLASMIDS pJDB207/IF2 (1'b)△
AND pJDB207/IF2 (5₁)△ 72

# 𝑭𝒊𝒈.2𝐵

NUCLEOTIDE SEQUENCE OF CLONES pJDB207/IF2(5₁) Δ 72 AND pJDB207/IF2(5₁)Δ82 AROUND THE NEW JUNCTION

(Xho LINKER) BETWEEN THE 3'NONTRANSLATED REGION OF IFN-5₁ AND THE PHO5 TRANSCRIPTION TERMINATION REGION

a) pJDB207/IF2(5₁)Δ72:

ILEASNLEUGLNLYSARGLEULYSSERLYSGLU
......ATCAACTTGCAAAAAAGATTGAAGAGTAAGGAATGAGACCTGGTACAACACGGAAATGATTCTTATAGACTACCTCGAGGGTCAGCAGCGTCAGTAACTCTACTGAATTGACCTTCTACTGG
————————————— IFN-5₁ SEQUENCE ————————————————→ Xho
                                                                                          LINKER

GACTGGAACACTACTCATTACAACGCCAGTCTATTGAGACAATAGTTTTGTATAACTAAATAATATTGGAAACTAAATACGAATACCCAAATTTTTTATCTAAATTTTGCCGAAAGATTAAAATCTGCA.....
                                                                                                                              mRNA ———┘                    Pst I
————————————————— PHO5 TRANSCRIPTION TERMINATION FRAGMENT —————————————————————→

b) pJDB207/IF2(5₁)Δ82:

ILEASNLEUGLNLYSARGLEULYSSERLYSGLU
......ATCAACTTGCAAAAAAGATTGAAGAGTAAGGAATGAGACCTGGTACCCTCGAGGGGAACACTACTCATTACAACGCCAGTCTATTGAGACAATAGTTTT GTATAACTAAATAATATTGGA
——————————IFN-5₁ SEQUENCE ——————————→ Xho      ←——————————— PHO5 TRANSCRIPTION TERMINATION FRAGMENT ———
                                                LINKER

AACTAAATACGAATACCCAAATTTTTTATCTAAATTTTGCCGAAAGATTAAAATCTGCA.....
                              mRNA ———┘                Pst I
————————————————————————————————→

## CONSTRUCTION OF HYBRID PLASMID CG-pBR322/HLycIFN(α-3)-252 CONTAINING A PORTABLE HUMAN LYMPHOBLASTOID IFN-α-3 cDNA INSERT.

Fig.29

## ‗Fig.30

STRUCTURE OF HYBRID PLASMIDS CG-pBR322/HLycIFN ( α-2 )-261 AND
CG-pBR322/HLyc IFN (α-1)-258

CG-pBR322/HLyc IFN (α-2)-261

CG-pBR322/HLyc IFN (α-1)-258

**Fig. 31**   DELETION OF THE PHO5 SIGNAL SEQUENCE IN EXPRESSION PLASMID p 31

## Fig.32

COLLECTION OF Bal 31 DELETION IN THE PHO5 REGION WITH EcoRI LINKERS AT THE ARROWS

-141                                                                                                    -42
GGGATAAGGGTAAACATCTTTGAATTGTCGAAATGAAACGTATATAAGCGCTGATGTTTTGCTAAGTCGAGGTTAGTATGGCTTCATCTCTCATGAGAAT
          ↑                                                          ↑        ↑       ↑      ↑     ↑↑
          h                                                          i        K       m      F     O M

-41 ┌→ PHO5 mRNA                                    →  PHO5                                              + 59
AAGAACAACAACAAATAGAGCAAGCAAATTCGAGATTACCAATGTTTAAATCTGTTGTTTATTCAATTTTAGCCGCTTCTTTGGCCAATGCAGGTACCAT
      ↑        ↑↑↑  ↑ ↑↑  ↑ ↑      ↑        ↑              ↑  ↑↑         ↑           BalI
      l        kSH  C NL  V P      R        Y              d  eG         E

──────→ PHO5                STARTPOINT OF PHO5 PROTEIN CODING REGION

┌──→ PHO5 mRNA              MAJOR PHO5 mRNA INITIATION SITE

↑                           ENDPOINT OF Bal 31 DELETION (FROM Bal I
                            RESTRICTION SITE) AND POSITION OF EcoRI
                            LINKER ADDITION

EP 0 100 561 B1

# Fig. 33

NUCLEOTIDE SEQUENCE OF THE BamHI-EcoRI RESTRICTION FRAGMENT
CONTAINING THE PHO5/R PROMOTER REGION

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGGG

# 𝕱𝖎𝖌.34.

NUCLEOTIDE SEQUENCE OF THE BamHI-EcoRI RESTRICTION FRAGMENT
CONTAINING THE PHO5/Y PROMOTER REGION

```
                                                                    G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG
```

## Fig. 35

INSERTION OF LYMPHOBLASTOID IFN-α-3 DNA INTO PLASMID p31/R

## _Fig. 36_

INSERTION OF LYMPHOBLASTOID IFN-α-2-DNA INTO PLASMID p 31/R

CG-pBR322/HLycIFN(α-2)- 261

1. Xbal digest
2. partial EcoRI digest
3. isolation of 252 bp fragment

pJDB 207/IF2 (5₁)

1. Hind III + Xbal digests
2. isolation of 0.9 kb fragment

EcoRI   Xbal

Xbal EcoRI BamHI HindIII

p31/R 4.3 kb

1. Hind III + EcoRI digests
2. isolation of 3.9 kb fragment

ligation

p31R/IF(α-2) 5.2 kb

## *Fig. 37*

### INSERTION OF LYMPHOBLASTOID IFN-α-1 DNA INTO PLASMID p 31/R

CG-pBR322/HLycIFN(α-1)-258

1. PvuII + EcoRI digests
2. isolation of 286 bp fragment

pJDB207/IF2 (1'b)

1. PvuII + HindIII digests
2. isolation of 0.9kb fragment

1. HindIII + EcoRI digests
2. isolation of 3.9kb fragment

ligation

## Fig.38

### CONSTRUCTION OF EXPRESSION PLASMID pJDB207R/IF(α-3)

pJDB207 6.9kb

EcoRI
EcoRI
amp$^R$
Sal I
BamHI
EcoRI
HindIII

Hind III
Pst I
amp$^R$
p31R/IF(α-3) 4.9kb
BamHI
EcoRI
Sal I
BamHI
EcoRI

1. Hind III + Sal I digests
2. isolation of 6.2kb fragment

1. Hind III + Sal I digests
2. isolation of 1.86kb fragment

HindIII    EcoRI    EcoRI    Sal I

Sal I  BamHI  EcoRI    EcoRI    Pst I  Hind III
BamHI

ligation

EcoRI
EcoRI
amp$^R$
8.0kb
HindIII
Pst I
BamHI
EcoRI
Sal I
BamHI  EcoRI

pJDB207R/IF(α-3)

38